(19) Europäisches Patentamt
European Patent Office
Office européen des brevets

(11) **EP 3 253 492 B1**

(12) **EUROPEAN PATENT SPECIFICATION**

(45) Date of publication and mention
of the grant of the patent:
**03.04.2024 Bulletin 2024/14**

(21) Application number: **16708007.6**

(22) Date of filing: **05.02.2016**

(51) International Patent Classification (IPC):
**B01L 7/00** *(2006.01)* **G01N 21/64** *(2006.01)*
**G01N 35/02** *(2006.01)* **G01N 35/00** *(2006.01)*

(52) Cooperative Patent Classification (CPC):
**B01L 7/52; G01N 21/274; G01N 21/6452;**
**G01N 35/026;** B01L 2200/145; B01L 2300/1822;
G01N 2021/6439; G01N 2035/00316;
G01N 2035/00366

(86) International application number:
**PCT/US2016/016856**

(87) International publication number:
**WO 2016/127107 (11.08.2016 Gazette 2016/32)**

(54) **SYSTEMS FOR BIOLOGICAL ANALYSIS**

SYSTEME ZUR BIOLOGISCHEN ANALYSE

SYSTÈMES D'ANALYSE BIOLOGIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **06.02.2015 US 201562113212 P**

(43) Date of publication of application:
**13.12.2017 Bulletin 2017/50**

(60) Divisional application:
**24157142.1**

(73) Proprietor: **Life Technologies Corporation
Carlsbad, CA 92008 (US)**

(72) Inventors:
• **CHU, Yong**
  **Carlsbad, California 92008 (US)**
• **MARKS, Jeffrey**
  **Carlsbad, California 92008 (US)**
• **FREUDENTHAL, Jacob**
  **Carlsbad, California 92008 (US)**
• **CHEN, Mingsong**
  **Carlsbad, California 92008 (US)**
• **TOH, Tiong Han**
  **Carlsbad, California 92008 (US)**
• **AGUANNO, Mauro**
  **Carlsbad, California 92008 (US)**
• **LAU, Lik Seng**
  **Carlsbad, California 92008 (US)**
• **LOH, Lian Seng**
  **Carlsbad, California 92008 (US)**
• **TEO, Kok Siong**
  **Carlsbad, California 92008 (US)**
• **CHU, Zeng Wei**
  **Carlsbad, California 92008 (US)**
• **MATHERS, Xin**
  **Carlsbad, California 92008 (US)**
• **UY, Michael**
  **Carlsbad, California 92008 (US)**
• **YEO, Huei Steven**
  **Carlsbad, California 92008 (US)**
• **BOO, Kuan Moon**
  **Carlsbad, California 92008 (US)**
• **LEE, Way Xuang**
  **Carlsbad, California 92008 (US)**
• **KOO, Chin Yong**
  **Carlsbad, California 92008 (US)**
• **TEO, Wei Fuh**
  **Carlsbad, California 92008 (US)**
• **LAU, Soo Yong**
  **Carlsbad, California 92008 (US)**
• **SHIN, Hon Siu**
  **Singapore 529233 (SG)**
• **TAN, Zeqi**
  **Carlsbad, California 92008 (US)**

- **WESSEL, Thomas**
  **Carlsbad, California 92008 (US)**
- **WOO, David**
  **Carlsbad, California 92008 (US)**

(74) Representative: **Ziermann, Oliver**
  **Life Technologies GmbH**
  **Frankfurter Straße 129b**
  **64293 Darmstadt (DE)**

(56) References cited:
  **EP-A1- 2 615 462       WO-A1-2014/020977**
  **GB-A- 2 472 454        US-A1- 2012 080 610**

**Description**

**FIELD OF THE DISCLOSURE**

The present invention relates generally to systems, devices, and methods for observing, testing, and/or analyzing one or more biological samples, and more specifically to systems, devices, and methods for observing, testing, and/or analyzing an array of biological samples.

**BACKGROUND**

Generally, there is a need to increasingly automate biological analysis systems to increase efficiency. For example, advances in automated biological sample processing instruments allow for quicker and more efficient analysis of samples.

There is also an increasing need to provide biological analysis systems with designs that cater to user needs, such as ease of install, ease of use, minimal necessary lab space.

A prior art analysis system is disclosed e.g. in EP 2 615 462 A1.

**SUMMARY OF THE DISCLOSURE**

In an embodiment of the present invention, a biological analysis system is provided. The system comprises a sample block assembly comprising a sample block configured to accommodate a sample holder, the sample holder configured to receive a plurality of samples. The system further comprises a control system configured to cycle the plurality of samples through a series of temperatures, and a tray configured to reversibly slide the sample block assembly from a closed to an open position to allow user access to the plurality of sample holders.

In another embodiment, a biological analysis system is provided. The system comprises a block assembly comprising a sample block having a plurality of block wells, the sample block configured to accommodate a sample holder, the sample holder configured to receive a plurality of samples. The system further comprises a control system configured to cycle the plurality of samples through a series of temperatures and an optical system configured to deliver excitation light to the plurality of samples and detect a fluorescence level emitted from each of the plurality of samples. The system further comprises a heated cover comprising a lower plate, a heater, and an upper plate having a plurality of upper plate apertures. The lower plate can have a mating surface for mating with an upper surface of the sample holder, the mating surface having a plurality of lower plate apertures each aligned with an associated one of the plurality of block wells to allow excitation light to pass to the block wells.

In yet another embodiment, not claimed but useful for understanding the invention, a biological analysis system is provided. The system comprises a plurality of system modules, the modules comprising a detector module, an emission module, an excitation module, and a base module. The plurality of system modules can be configured to be reversibly connected to form a first biological analysis device type.

In a further embodiment not claimed but useful for understanding the invention, a biological analysis system is provided. The system comprises an instrument and a calibration system for calibrating the instrument. The instrument can comprise a block assembly comprising a sample block configured to accommodate a sample holder having a plurality of reaction sites, and an optical system capable of imaging florescence emission from a plurality of reaction sites. The calibration system can comprise a region-of-interest (ROI) calibrator configured to determine reaction site positions in an image. The calibration system can also comprise a pure dye calibrator configured to determine the contribution of a fluorescent dye used in each reaction site by comparing a raw spectrum of the fluorescent dye to a pure spectrum calibration data of the fluorescent dye. The calibration system can further comprise an instrument normalization calibrator configured to determine a filter normalization factor. The calibration system can even further comprise an RNase P validator configured to validate the instrument is capable of distinguishing between two different quantities of sample. The calibration system can also comprise a display engine configured to display calibration results.

Additional aspects, features, and advantages of the present invention are set forth in the following description and claims, particularly when considered in conjunction with the accompanying drawings in which like parts bear like reference numbers.

**BRIEF DESCRIPTION OF THE FIGURES**

FIG. 1 is a block diagram that illustrates an exemplary instrument system, upon which embodiments of the present teachings may be implemented.

FIG. 2 is a block diagram that illustrates a computer system, upon which embodiments of the present teachings may be implemented.

FIG. 3 illustrates an exemplary distributed network system according to various embodiments described herein.

FIG. 4 illustrates a thermal cycler system with a housing according to various embodiments described herein.

FIG. 5 illustrates thermal cycler system of FIG. 4 with a movable tray in an open position according to various embodiments described herein.

FIG. 6 illustrates a modular instrument system according to various embodiments described herein.

FIG. 7 is a schematic representation of a system according to an embodiment of the present invention.

FIG. 8 is a normalized spectrum plot of various light sources, including a light source according to an embodiment of the current invention.

FIG. 9 is plot of spectral integration over various wavelength ranges for the light source spectrums shown in FIG. 8.

FIG. 10 is a solid model representation of an optical and sample processing system according to an embodiment of the present invention.

FIG. 11 is a magnified, solid model representation of the optical system shown in FIG. 7.

FIG. 12 is a section view of a portion of the optical system shown in FIG. 10.

FIG. 13 is a top perspective view of an imaging unit according to an embodiment of the present invention.

FIG. 14 is a sectional view of the imaging unit shown in FIG. 13.

FIGS. 15 and 16 are bottom perspective views of the imaging unit shown in FIG. 13.

FIGS. 17-19 are magnified views of portions of the imaging unit shown in FIG. 13.

FIG. 20 is a section view of the system shown in FIG. 11.

FIG. 21 illustrates a calibration workflow for a biological instrument according to various embodiments described herein.

FIG. 22 illustrates a sequence of steps used in the calibration of qPCR instruments.

FIG. 23 illustrates the regions-of-interest for a 96 well sample container.

FIG. 24 is an image of a calibration plate with FAM dye occupying each well of a 96-well calibration plate.

FIGS. 25 and 26 depict an example workflow according to an embodiment of the present disclosure.

FIG. 27A illustrates calibration plates with checkerboard configurations according to an embodiment of the present disclosure.

FIG. 27B is an image of a 4 dye checkerboard 96-well calibration plate with FAM, VIC, ROX and SYBR dyes in the same configuration as illustrated by plate 3100 in FIG. 11A.

FIG. 28A illustrates dye mixtures used in various embodiments of the present teachings.

FIG. 28B illustrates pure dyes and main channel filter combinations for various embodiment of the present teachings.

FIG. 29 illustrates % deviation of dye mixtures before normalization according to various embodiments of the present teachings.

FIG. 30 illustrates % deviation of dye mixtures after normalization according to various embodiments of the present teachings.

FIG. 31 illustrates a closer view of % deviation of dye mixtures after normalization according to various embodiments of the present teachings.

FIG. 32 is a flow chart depicting a normalization process according to various embodiments of the present teachings.

FIG. 33 illustrates an exemplary method for validating an instrument according to various embodiments described herein.

FIG. 34 illustrates another exemplary method for validation an instrument according to various embodiments described herein.

FIG. 35 illustrates determining a plurality of fluorescence thresholds from amplification data according to various embodiments described herein.

FIG. 36 illustrates a system for validation of an instrument according to various embodiments described herein.

FIG. 37 illustrates a system for calibration of an instrument according to various embodiments described herein.

FIG. 38 illustrates a slidable assembly according to various embodiments described herein.

FIG. 39 illustrates the slidable assembly of FIG. 38 with sample block assembly removed, according to various embodiments described herein.

FIG. 40 is a side view of the embodiment of FIG. 38 according to various embodiments described herein.

FIGS. 41A, 41B and 41C provide different views of optical sensors of the slidable assembly according to various embodiments described herein.

FIGS. 42A and 42B provide different views of optical sensors of the slidable assembly according to various embodiments described herein.

FIG. 43 illustrates an embodiment of a sample block assembly according to various embodiments described herein.

FIG. 44A illustrates a heated cover according to various embodiments described herein.

FIGS. 44B and 44C provide top views of a pressure plate according to various embodiments described herein.

FIG. 45 illustrates a pulley system of a heated cover according to various embodiments described herein.

## DETAILED DESCRIPTION

The following description provides embodiments of the present invention, which are generally directed to systems, devices, and methods for preparing, observing, testing, and/or analyzing an array of biological samples. Such description is not intended to limit the scope of the present invention, but merely to provide a description of embodiments.

SYSTEM OVERVIEW

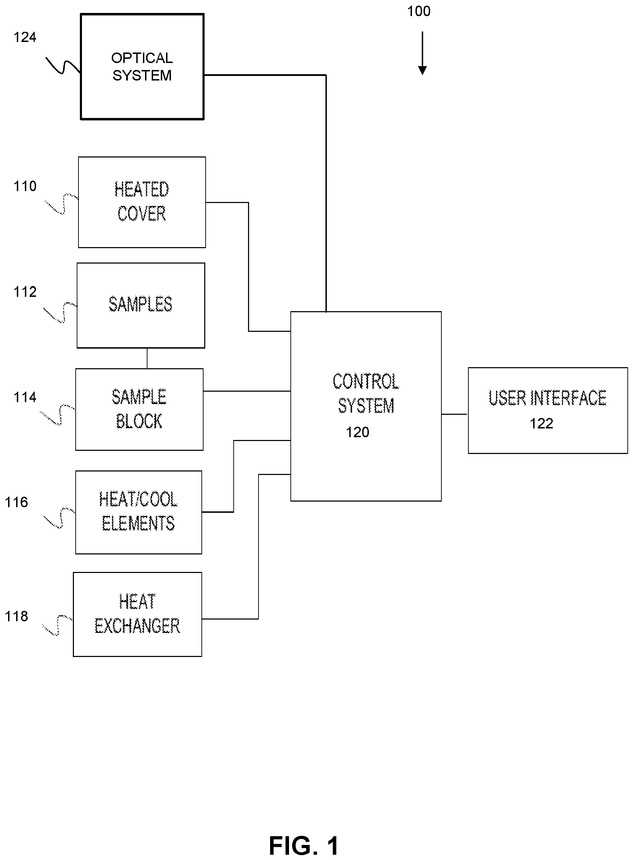

To prepare, observe, test, and/or analyze an array of biological samples, one example of an instrument that may be utilized according to various embodiments is a thermal cycler device, such as an end-point polymerase chain reaction (PCR) instrument or a quantitative, or real-time, PCR instrument. FIG. 1 is a block diagram that illustrates a thermal cycler system 100, upon which embodiments of the present teachings may be implemented. Thermal cycler system 100 includes a heated cover 110, discussed in greater detail below, which is placed over a sample block 114, having a plurality of reaction regions or sample block wells, configured to be loaded with a plurality of samples 112 on a sample holder (not shown), also discussed in greater detail below.

In various embodiments, the sample holder may have a plurality of sample regions, or wells, configured for receiving a plurality of samples, wherein the wells may be sealed within the sample holder via a lid, cap, sealing film or any other sealing mechanism between the wells and heated cover 110. Some examples of a sample holder may include, but are not limited to, any size multi-well plate, card or array including, but not limited to, a 24-well microtiter plate, 48-well microtiter plate, a 96-well microtiter plate, a 384-well microtiter plate, a microcard, a through-hole array, or a substantially planar holder, such as a glass or plastic slide. The wells in various embodiments of a sample holder may include depressions, indentations, ridges, and combinations thereof, patterned in regular or irregular arrays formed on the surface of the sample holder substrate. Sample or reaction volumes can also be located within wells or indentations formed in a substrate, spots of solution distributed on the surface a substrate, or other types of reaction chambers or formats, such as samples or solutions located within test sites or volumes of a microfluidic system, or within or on small beads or spheres.

In another embodiment, an initial sample or solution may be divided into hundreds, thousands, tens of thousands, hundreds of thousands, or even millions of reaction sites, each having a volume of, for example, a few nanoliters, about one nanoliter, or less than one nanoliter (e.g., 10's or 100's of picoliters or less).

Thermal cycler system 100 may also include a sample block 114, elements for heating and cooling 116, a heat exchanger 118, a control system 120, and a user interface 122, wherein components 114, 116 and 118 can be included within a thermal block assembly. More detail of the thermal block assembly will be discussed below.

In an embodiment, the elements for heating and cooling 116 can be thermoelectric devices such as, for example, Peltier devices. The number of thermoelectric devices used within a thermal block assembly can depend on a number of factors including, but not limited to, cost, the number of independent zones desired, and the size of the sample holder. For example, a sample block for holding a 48-well microtiter plate may be sized to accommodate a single thermoelectric device, whereas sample blocks configured for plates having more wells may accommodate more than one thermoelectric device such as, for example, four thermoelectric devices. Moreover, if control over multiple zones on a sample block is desired, the number of thermoelectric devices can vary from a single thermoelectric device to, for example, a thermoelectric device per sample region (e.g., well, through-hole, reaction site, etc.) on the sample block. For example, for the sample block can be divided into, for example, 6 sub-blocks of 16-well format together forming a 96-well array that can accommodate a 96-well microtiter plate. If may be desired to provide independent zonal control to each of the sub-blocks, thereby allowing for 6 thermoelectric devices, each of which correspond to an associated sub-block.

In a realisation mode not according to the invention thermal cycler system 100 can have a two-sided thermal assembly, where elements for heating and cooling 116 and heat exchanger 118 can be provided above (upper side) and below (lower side) sample block 114. In such a realisation mode not according to the invention the upper side of the two-sided thermal assembly provided above sample block 114, can replace heater cover 110. Such a configuration could provide more uniform heating from above and below the samples. For a real-time thermal cycler, the upper side can have portions of clear construction to allow for the passing of an excitation light source and emitted fluorescence. Such portions can be made of any clear material including, for example, plastic and glass.

Thermal cycler system 100 can also have an optical system 124. In FIG. 1, optical system 124 may have an illumination source (not shown) that emits electromagnetic energy, an optical sensor, detector, or imager (not shown), for receiving electromagnetic energy from samples 112 in a sample holder, and optics used to guide the electromagnetic energy from each DNA sample to the imager. The optical system is discussed in more detail below.

Control system 120 may be used to control the functions of optical system 124, heated cover 110, and the thermal block assembly, which can comprise sample block 114, heating and cooling elements 116, and heat exchanger 118. Control system 120 may be accessible to an end user through user interface 122 of thermal cycler system 100 in FIG. 1. Control system 120 may be used to control calibrations of thermal cycler system 100, as will be discussed in further detail below.

COMPUTER-IMPLEMENTED SYSTEM

Methods of in accordance with embodiments described herein, may be implemented in a computer system.

Those skilled in the art will recognize that the operations of the various embodiments may be implemented using hardware, software, firmware, or combinations thereof, as appropriate. For example, some processes can be carried out using processors or other digital circuitry under the control of software, firmware, or hard-wired logic. (The term "logic" herein refers to fixed hardware, programmable logic and/or an appropriate combination thereof, as would be recognized by one skilled in the art to carry out the recited functions.) Software and firmware can be stored on non-transitory computer-readable media. Some other processes can be implemented using analog circuitry, as is well known to one of ordinary skill in the art. Additionally, memory or other storage, as well as communication components, may be employed in embodiments of the invention.

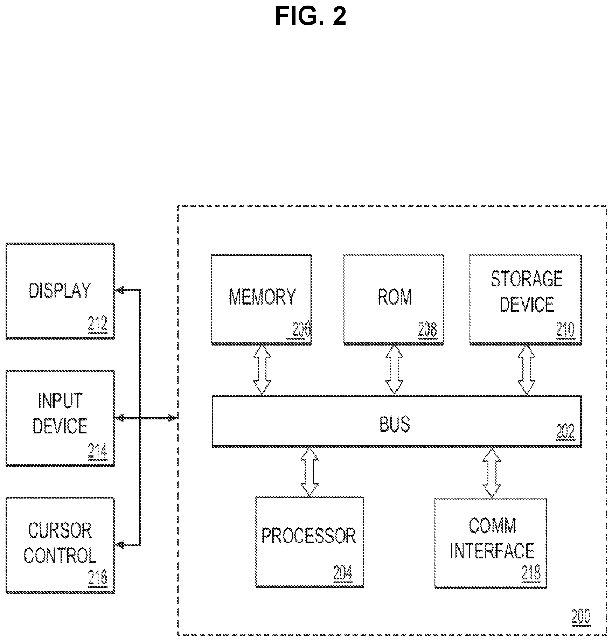

FIG. 2 is a block diagram that illustrates a computer system 200 that may be employed to carry out processing functionality, according to various embodiments. Instruments to perform experiments may be connected to the exemplary computing system 200. According to various embodiments, the instruments that may be utilized include, for example, thermal cycler system 100 of FIG. 1. Computing system 200 can include one or more processors, such as a processor 204. Processor 204 can be implemented using a general or special purpose processing engine such as, for example, a microprocessor, controller or other control logic. Processor 204 can be connected to a bus 202 or other communication medium.

Referring to FIG. 2, a computer system 200 may provide control to the function of thermal cycler system 100 in FIG. 1, as well as the user interface function. Additionally, computer system 200 of FIG. 2 may provide data processing, display and report preparation functions. All such instrument control functions may be dedicated locally to the PCR instrument. As such, computer system 200 can serve as control system 120 illustrated in FIG. 1. Computer system 200 of FIG. 2 may also provide remote control of part or all of the control, analysis, and reporting functions, as will be discussed in more detail subsequently.

Computing system 200 of FIG. 2 may also be embodied in any of a number of forms, such as a rack-mounted computer, mainframe, supercomputer, server, client, a desktop computer, a laptop computer, a tablet computer, hand-held computing device (e.g., PDA, cell phone, smart phone, palmtop, etc.), cluster grid, netbook, embedded systems, or any other type of special or general purpose computing device as may be desirable or appropriate for a given application or environment. Additionally, a computing system 200 can include a conventional network system including a client/server environment and one or more database servers, or integration with LIS/LIMS infrastructure. A number of conventional network systems, including a local area network (LAN) or a wide area network (WAN), and including wireless and/or wired components, are known in the art. Additionally, client/server environments, database servers, and networks are well documented in the art. According to various embodiments described herein, computing system 200 may be configured to connect to one or more servers in a distributed network. Computing system 200 may receive information or updates from the distributed network. Computing system 200 may also transmit information to be stored within the distributed network that may be accessed by other clients connected to the distributed network.

Computing system 200 of FIG. 2 also includes a memory 206, which can be a random access memory (RAM) or other dynamic memory, coupled to bus 202 for storing instructions to be executed by processor 204. Memory 206 also may be used for storing temporary variables or other intermediate information during execution of instructions to be executed by processor 204.

Computing system 200 further includes a read only memory (ROM) 208 or other static storage device coupled to bus 202 for storing static information and instructions for processor 204.

Computing system 200 may also include a storage device 210, such as a magnetic disk, optical disk, or solid state drive (SSD) is provided and coupled to bus 202 for storing information and instructions. Storage device 210 may include a media drive and a removable storage interface. A media drive may include a drive or other mechanism to support fixed or removable storage media, such as a hard disk drive, a floppy disk drive, a magnetic tape drive, an optical disk drive, a CD or DVD drive (R or RW), flash drive, or other removable or fixed media drive. As these examples illustrate, the storage media may include a computer-readable storage medium having particular computer software, instructions, or data stored therein.

In alternative embodiments, storage device 210 may include other similar instrumentalities for allowing computer programs or other instructions or data to be loaded into computing system 200. Such instrumentalities may include, for example, a removable storage unit and an interface, such as a program cartridge and cartridge interface, a removable memory (for example, a flash memory or other removable memory module) and memory slot, and other removable storage units and interfaces that allow software and data to be transferred from the storage device 210 to computing system 200.

Computing system 200 of FIG. 2 can also include a communications interface 218. Communications interface 218 can be used to allow software and data to be transferred between computing system 200 and external devices. Examples of communications interface 218 can include a modem, a network interface (such as an Ethernet or other NIC card), a communications port (such as for example, a USB port, a RS-232C serial port), a PCMCIA slot and card, Bluetooth, etc. Software and data transferred via communications interface 218 are in the form of signals which can be electronic, electromagnetic, optical or other signals capable of being received by communications interface 218. These signals may be transmitted and received by communications interface 218 via a channel such as a wireless medium, wire or cable, fiber optics, or other communications medium. Some examples of a channel include a phone line, a cellular phone link, an RF link, a network interface, a local or wide area network, and other communications channels.

Computing system 200 may be coupled via bus 202 to a display 212, such as a cathode ray tube (CRT) or liquid crystal display (LCD), for displaying information to a computer user. An input device 214, including alphanumeric and other keys, is coupled to bus 202 for communicating information and command selections to processor 204, for example. An input device may also be a display, such as an LCD display, configured with touchscreen input capabilities. Another type of user input device is cursor control 216, such as a mouse, a trackball or cursor direction keys for communicating direction information and command selections to processor 204 and for controlling cursor movement on display 212. This input device typically has two degrees of freedom in two axes, a first axis (e.g., x) and a second axis (e.g., y), that allows the device to specify positions in a plane. A computing system 200 provides data processing and provides a level of confidence for such data. Consistent with certain implementations of embodiments of the present teachings, data processing and confidence values are provided by computing system 200 in response to processor 204 executing one or more sequences of one or more instructions contained in memory 206. Such instructions may be read into memory 206 from another computer-readable medium, such as storage device 210. Execution of the sequences of instructions contained in memory 206 causes processor 204 to perform the process states described herein. Alternatively hard-wired circuitry may be used in place of or in combination with software instructions to implement embodiments of the present teachings. Thus implementations of embodiments of the present teachings are not limited to any specific combination of hardware circuitry and software.

The term "computer-readable medium" and "computer program product" as used herein generally refers to any media that is involved in providing one or more sequences or one or more instructions to processor 204 for execution. Such instructions, generally referred to as "computer program code" (which may be grouped in the form of computer programs or other groupings), when executed, enable the computing system 200 to perform features or functions of embodiments of the present invention. These and other forms of non-transitory computer-readable media may take many forms, including but not limited to, non-volatile media, volatile media, and transmission media. Non-volatile media includes, for example, solid state, optical or magnetic disks, such as storage device 210. Volatile media includes dynamic memory, such as memory 206. Transmission media includes coaxial cables, copper wire, and fiber optics, including the wires that comprise bus 202.

Common forms of computer-readable media include, for example, a floppy disk, a flexible disk, hard disk, magnetic tape, or any other magnetic medium, a CD-ROM, any other optical medium, punch cards, paper tape, any other physical medium with patterns of holes, a RAM, PROM, and EPROM, a FLASH-EPROM, any other memory chip or cartridge, a carrier wave as described hereinafter, or any other medium from which a computer can read.

Various forms of computer readable media may be involved in carrying one or more sequences of one or more instructions to processor 204 for execution. For example, the instructions may initially be carried on magnetic disk of a remote computer. The remote computer can load the instructions into its dynamic memory and send the instructions over a telephone line using a modem. A modem local to computing system 200 can receive the data on the telephone line and use an infra-red transmitter to convert the data to an infra-red signal. An infra-red detector coupled to bus 202 can receive the data carried in the infra-red signal and place the data on bus 202. Bus 202 carries the data to memory 206, from which processor 204 retrieves and executes the instructions. The instructions received by memory 206 may optionally be stored on storage device 210 either before or after execution by processor 204.

It will be appreciated that, for clarity purposes, the above description has described embodiments of the invention with reference to different functional units and processors. However, it will be apparent that any suitable distribution of functionality between different functional units, processors or domains may be used without detracting from the invention. For example, functionality illustrated to be performed by separate processors or controllers may be performed by the same processor or controller. Hence, references to specific functional units are only to be seen as references to suitable means for providing the described functionality, rather than indicative of a strict logical or physical structure or organization.

DISTRIBUTED SYSTEM

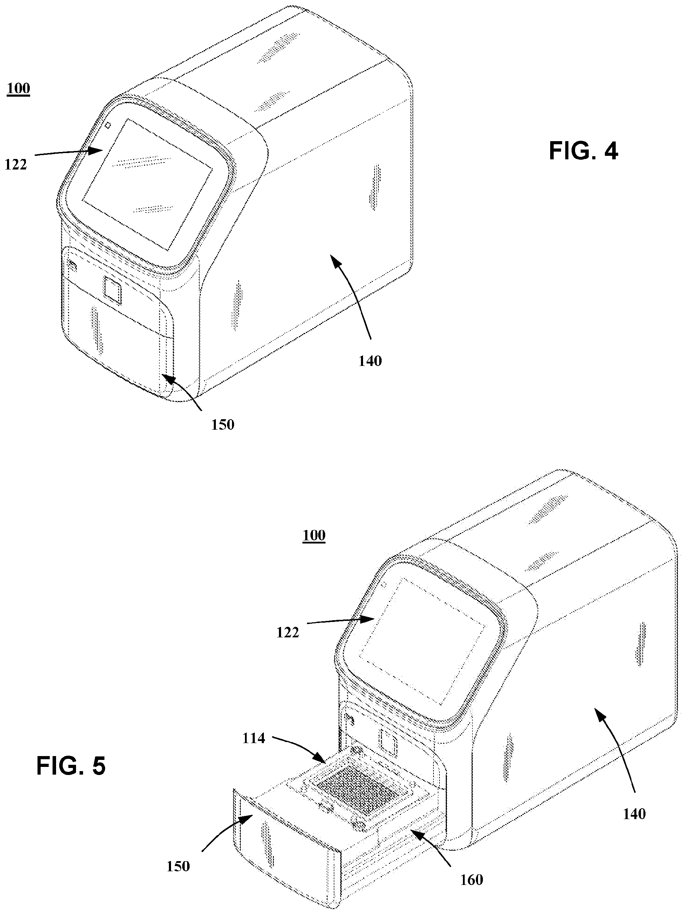

Some of the elements of a typical Internet network configuration 2500 are shown in FIG. 5, wherein a number of client machines 2502 possibly in a remote local office, are shown connected to a gateway/hub/tunnel-server/etc 2510 which is itself connected to the internet 2508 via some internet service provider (ISP) connection 2510. Also shown are other possible clients 2512 similarly connected to the internet 2508 via an ISP connection 2514, with these units communicating to possibly a central lab or an office, for example, via an ISP connection 2516 to a gateway/tunnel-server 2518 which is connected 2520 to various enterprise application servers 2522 which could be connected through another hub/router 2526 to various local clients 2530. Any of these servers 2522 could function as a development server for the analysis of potential content management and delivery design solutions as described in the present invention, as more fully described below.

MODULAR SYSTEM

FIG. 4 illustrates an embodiment of thermal cycler system 100 with a housing 140 enclosing many of the elements of system 100 discussed previously. In this embodiment, user interface 122 is provided on a front side of system 100, with a tray face 150 provided below interface 122. Housing 140 can be made of one single piece or multiple pieces based on preference.

FIG. 5 illustrates the embodiment of FIG. 4 with a movable tray 160 in an open, exposed position. Movable tray 160 can comprise sample block 114 as illustrated. In the open position, sample block 114 is available for loading with samples. Besides sample block 114, movable tray 160 may comprise other components that are ejected when movable tray 160 is in an open position. For example, movable tray 160 can comprise heating and cooling elements associated with sample block 114. Further, movable tray 160 can include an associated heat exchanger or heat sink. Movable tray 160 can be moved manually or mechanically. For example, a handle or grip can be provided if tray 160 is manually movable. If mechanically movable, a motor system can be provided within system 100, as discussed in detail below.

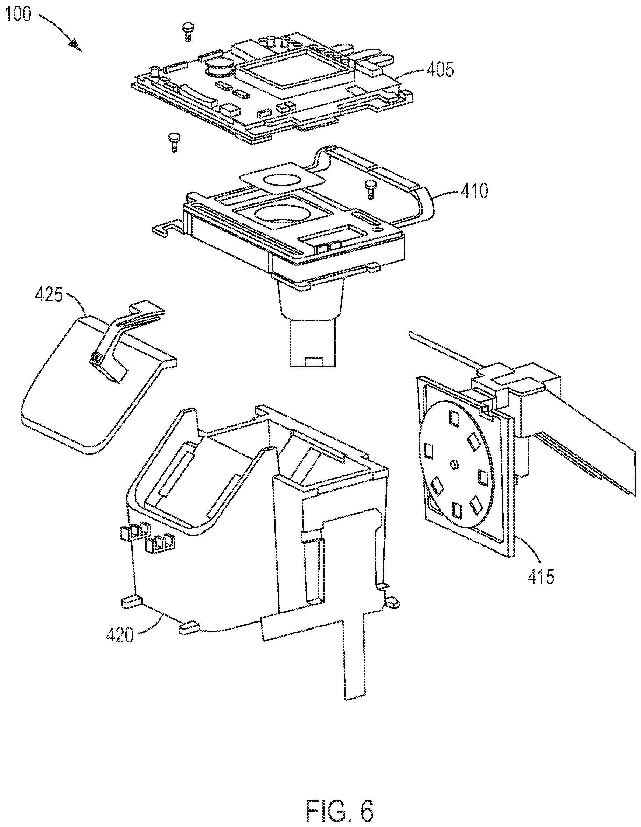

FIG. 6 illustrates an embodiment of thermal cycler system 100, where system 100 is constructed from modular components. By using a modular construction, modules can be constructed so as to be suitable for multiple instrument systems. For example, an optics module can be configured to be used on different types of instruments. Moreover, modular construction allows for ease of construction by providing already constructed instrument portions rather than requiring full instrument construction from scratch. Also, modular construction allows for ease of serviceability. By having to only connect a few modules to form a completed instrument, one would only have to reverse that process to gain access to specific modules for servicing.

In FIG. 6, system 100 includes detector module 405 (Sensor board/detector board, detector and PSB associated), emission module 410 (emission filter wheel, camera), excitation module 415 (Excitation source and excitation filter wheel), base module 420 (beamsplitter, folding mirror), and face plate 425.

Detector module 405 can include, for example, the emission sensor, emission detector, sensor printed circuit board and detector printed circuit board associated with optics system 124. Emission module 410 can include, for example, the camera and emission filter wheel associated with optics system 124. Excitation module 415 can include, for example, the excitation source, source cooling components, and excitation filter wheel associated with optics system 124. Base module 420 can include, for example, the beamsplitter and folding mirror associated with optics system 124, as well as, for example, the sample block, block heating/cooling elements, heat exchanger/sink, control system, and heater cover. Finally, face plate 425 can serve to, for example, cover the mirror components of base module 420, assist in connecting base module 420 to emission module 410, and/or provide a flat facing to accept user interface 122. The above components will be discussed in greater detail below. Moreover, the components included with specific modules discussed above are for exemplary purposes only and can be interchanged as needed. Furthermore, the number of modules can be increased or decreased as needed. For example, detector module 405 and emission module 410 can be combined into a single module. On the other hand, base module 420 can be split into multiple smaller modules.

One or more of modules 405, 410, 415 and 420 can also be used as modules for different instrument types. This flexibility allows for more efficient manufacturing as construction of multiple types of instruments can occur with common modules. For example, the modules discussed above can be connected to form a qPCR instrument with a 96-well format. One or more of the modules can also be used to form, for example, a qPCR instrument with a 384-well format, a through-hole format, a flat block format, and so on. One or more of the modules can also be used to form, for example, an endpoint PCR instrument. One or more of the modules can also be used to form, for example, qPCR instruments with different optical systems including, for example, 4-color or 6-color optical systems. One or more of the modules can also be used to form, for example, a capillary electrophoresis instrument. One or more of the modules can also be used to form, for example, a digital PCR instrument. One or more of the modules can also be used to form, for example, an optical reader.

OPTICAL SYSTEM

As summarized above and illustrated in FIG. 1, thermal cycler system 100 can include optical system 124.

As used herein the terms "radiation" or "electromagnetic radiation" means radiant energy released by certain electromagnetic processes that may include one or more of visible light (e.g., radiant energy characterized by one or more wavelengths between 400 nanometers and 700 nanometers or between 380 nanometers and 800 nanometers) or invisible electromagnetic radiations (e.g., infrared, near infrared, ultraviolet (UV), X-ray, or gamma ray radiation).

As used herein an excitation source means a source of electromagnetic radiation that may be directed toward at least one sample containing one or more chemical compounds such that the electromagnetic radiation interacts with the at least one sample to produce emission electromagnetic radiation indicative of a condition of the at least one sample. The excitation source may comprise light source. As used herein, the term "light source" refers to a source of electromagnetic radiation comprising an electromagnetic spectrum having a peak or maximum output (e.g., power, energy, or intensity) that is within the visible wavelength band of the electromagnetic spectrum (e.g., electromagnetic radiation within a wavelength in the range of 400 nanometers to 700 nanometers or in the range of 380 nanometers and 800 nanometers). Additionally or alternatively, the excitation source may comprise electromagnetic radiation within at least a portion of the infrared (near infrared, mid infrared, and/or far infrared) or ultraviolet (near ultraviolet and/or extreme ultraviolet) portions of the electromagnetic spectrum. Additionally or alternatively, the excitation source may comprise electromagnetic radiation in other wavelength bands of the electromagnetic spectrum, for example, in the X-ray and/or radio wave portions of the electromagnetic spectrum. The excitation source may comprise a single source of light, for example, an incandescent lamp, a gas discharge lamp (e.g., Halogen lamp, Xenon lamp, Argon lamp, Krypton lamp, etc.), a light emitting diode (LED), an organic LED (OLED), a laser, or the like. The excitation source may comprise a plurality of individual light sources (e.g., a plurality of LEDs or lasers). The excitation source may also include one or more excitation filters, such as a high-pass filter, a low-pass filter, or a band-pass filter. For example, the excitation filter may include a colored filter and/or a dichroic filter. The excitation source comprises a single beam or a plurality of beams that are spatially and/or temporally separated.

As used herein, an "emission" means an electromagnetic radiation produced as the result an interaction of radiation from an excitation source with one or more samples containing, or thought to contain, one or more chemical and/or biological molecules or compounds of interest. The emission may be due to a reflection, refraction, polarization, absorption, and/or other optical effect by the a sample on radiation from the excitation source. For example, the emission may comprise a luminescence or fluorescence induced by absorption of the excitation electromagnetic radiation by one or more samples. As used herein "emission light" refers to an emission comprising an electromagnetic spectrum having a peak or maximum output (e.g., power, energy, or intensity) that is within the visible band of the electromagnetic spectrum (e.g., electromagnetic radiation within a wavelength in the range of 420 nanometers to 700 nanometers).

As used herein, a lens means an optical element configured to direct or focus incident electromagnetic radiation so as to converge or diverge such radiation, for example, to provide a real or virtual image, either at a finite distance or at an optical infinity. The lens may comprise a single optical element having an optical power provided by refraction, reflection, and/or diffraction of the incident electromagnetic radiation. Alternatively, the lens may comprise a compound system including a plurality of optical element, for example, including, but not limited to, an acromatic lens, doublet lens, triplet lens, or camera lens. The lens may be at least partially housed in or at least partially enclosed by a lens case or a lens mount.

As used herein, the term "optical power" means the ability of a lens or optic to converge or diverge light to provide a focus (real or virtual) when disposed within air. As used herein the term "focal length" means the reciprocal of the optical power. As used herein, the term "diffractive power" or "diffractive optical power" means the power of a lens or optic, or portion thereof, attributable to diffraction of incident light into one or more diffraction orders. Except where noted otherwise, the optical power of a lens, optic, or optical element is from a reference plane associated with the lens or optic (e.g., a principal plane of an optic).

As used herein, the term "biological sample" means a sample or solution containing any type of biological chemical or component and/or any target molecule of interest to a user, manufacturer, or distributor of the various embodiments of the present invention described or implied herein, as well as any sample or solution containing related chemicals or compounds used for the purpose of conducting a biological assay, experiment, or test. These biological chemicals, components, or target molecules may include, but are not limited to, DNA sequences (including cell-free DNA), RNA sequences, genes, oligonucleotides, molecules, proteins, biomarkers, cells (e.g., circulating tumor cells), or any other suitable target biomolecule. A biological sample may comprise one or more of at least one target nucleic acid sequence, at least one primer, at least one buffer, at least one nucleotide, at least one enzyme, at least one detergent, at least one blocking agent, or at least one dye, marker, and/or probe suitable for detecting a target or reference nucleic acid sequence. In various embodiments, such biological components may be used in conjunction with one or more PCR methods and systems in applications such as fetal diagnostics, multiplex dPCR, viral detection, and quantification standards, genotyping, sequencing assays, experiments, or protocols, sequencing validation, mutation detection, detection of genetically modified organisms, rare allele detection, and/or copy number variation.

According to embodiments of the present invention, one or more samples or solutions containing at least one biological targets of interest may be contained in, distributed between, or divided between a plurality of a small sample volumes or reaction regions (e.g., volumes or regions of less than or equal to 10 nanoliters, less than or equal to 1 nanoliter, or less than or equal to 100 picoliters). The reaction regions disclosed herein are generally illustrated as being contained in wells located in a substrate material; however, other forms of reaction regions according to embodiments of the present invention may include reaction regions located within through-holes or indentations formed in a substrate, spots of solution distributed on the surface a substrate, samples or solutions located within test sites or volumes of a capillary or microfluidic system, or within or on a plurality of microbeads or microspheres.

While devices, instruments, systems, and methods according to embodiments of the present invention are generally directed to dPCR and qPCR, embodiments of the present invention may be applicable to any PCR processes, experiment, assays, or protocols where a large number of reaction regions are processed, observed, and/or measured. In a dPCR assay or experiment according to embodiments of the present invention, a dilute solution containing at least one target polynucleotide or nucleotide sequence is subdivided into a plurality of reaction regions , such that at least some of these reaction regions contain either one molecule of the target nucleotide sequence or none of the target nucleotide sequence. When the reaction regions are subsequently thermally cycled in a PCR protocol, procedure, assay, process, or experiment, the reaction regions containing the one or more molecules of the target nucleotide sequence are greatly amplified and produce a positive, detectable detection signal, while those containing none of the target(s) nucleotide sequence are not amplified and do not produce a detection signal, or a produce a signal that is below a predetermined threshold or noise level. Using Poisson statistics, the number of target nucleotide sequences in an original solution distributed between the reaction regions may be correlated to the number of reaction regions producing a positive detection signal. In some embodiments, the detected signal may be used to determine a number, or number range, of target molecules contained in the original solution. For example, a detection system may be configured to distinguish between reaction regions containing one target molecule and reaction regions containing two or at least two target molecules. Additionally or alternatively, the detection system may be configured to distinguish between reaction regions containing a number of target molecules that is at or below a predetermined amount and reaction regions containing more than the predetermined amount. In certain embodiments, processes, assays, or protocols for both qPCR and dPCR are conducted using a single the same devices, instruments, or systems, and methods.

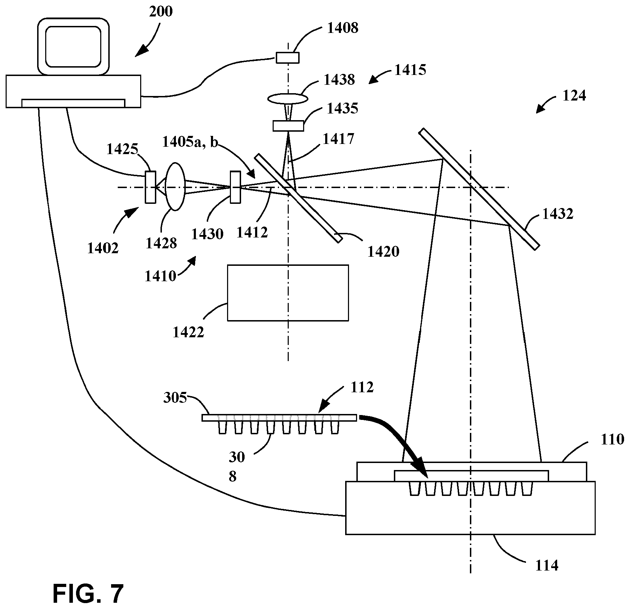

Referring to FIG. 7, system 100 may comprise one or more of a computer system, electronic processor, or controller 200, a sample block 114 configured to receive and/or processes a biological or biochemical sample, and/or an optical system 124. Without limiting the scope of the present invention, system 100 may comprise a sequencing instrument, a polymerase chain reaction (PCR) instrument (e.g., a real-time PCR (qPCR) instrument and/or digital PCR (dPCR) instrument), capillary electrophoresis instrument, an instrument for providing genotyping information, or the like.

Computer system 200 is configured to control, monitor, and/or receive data from optical system 124 and/or sample block 114. Computer system 200 may be physically integrated into optical system 124 and/or sample block 114. Additionally or alternatively, computer system 200 may be separate from optical system 124 and sample block 114, for example, an external desktop computer, laptop computer, notepad computer, tablet computer, or the like. Communication between computer system 200 and optical system 124 and/or sample block 114 may be accomplished directly via a physical connection, such as a USB cable or the like, and/or indirectly via a wireless or network connection (e.g., via Wi-Fi connection, a local area network, internet connection, cloud connection, or the like). Computer system 200 may include electronic memory storage containing instructions, routines, algorithms, test and/or configuration parameter, test and/or experimental data, or the like. Computer system 200 may be configured, for example, to operate various components of optical system 124 or to obtain and/or process data provided by sample block 114. For example, computer system 200 may be used to obtain and/or process optical data provided by one or more photodetectors of optical system 124.

In certain embodiments, computer system 200 may integrated into optical system 124 and/or sample block 114. Computer system 200 may communicate with external computer and/or transmit data to an external computer for further processing, for example, using a hardwire connection, a local area network, an internet connection, cloud computing system, or the like. The external computer may be physical computer, such as a desktop computer, laptop computer, notepad computer, tablet computer, or the like, that is located in or near system 100. Additionally or alternatively, either or both the external computer and computer system 200 may comprise a virtual device or system, such as a cloud computing or storage system. Data may be transferred between the two via a wireless connection, a cloud storage or computing system, or the like. Additionally or alternatively, data from computer system 200 (e.g., from optical system 124 and/or sample block 114) may be transferred to an external memory storage device, for example, an external hard drive, a USB memory module, a cloud storage system, or the like.

In certain embodiments, sample block 114 is configured to receive the sample holder 305. Sample holder 305 may comprise a plurality or array of spatially separated reaction regions, sites, or locations 308 for containing a corresponding plurality or array of biological or biochemical samples 114. Reaction regions 308 may comprise any plurality of volumes or locations isolating, or configured to isolate, the plurality of biological or biochemical samples 114. For example, reaction regions 308 may comprise a plurality of through-hole or well in a substrate or assembly (e.g., sample wells in a standard microtiter plate), a plurality of sample beads, microbeads, or microspheres in a channel or chamber, a plurality of distinct locations in a flow cell, a plurality of sample spots on a substrate surface, or a plurality of wells or openings configured to receive a sample holder (e.g., the cavities in a sample block assembly configured to receive a microtiter plate).

Sample block 114 may include sample holder 305. At least some of the reaction regions 308 may include the one or more biological samples 114. Biological or biochemical samples 114 may include one or more of at least one target nucleic acid sequence, at least one primer, at least one buffer, at least one nucleotide, at least one enzyme, at least one detergent, at least one blocking agent, or at least one dye, marker, and/or probe suitable for detecting a target or reference nucleic acid sequence. Sample holder 305 may be configured to perform at least one of a PCR assay, a sequencing assay, or a capillary electrophoresis assay, a blot assay. In certain embodiments, sample holder 305 may comprise one or more of a microtiter plate, substrate comprising a plurality of wells or through-holes, a substrate comprising a one or more channels, or a chamber comprising plurality of beads or spheres containing the one or more biological samples. Reaction regions 308 may comprise one or more of a plurality of wells, a plurality of through-holes in substrate, a plurality of distinct locations on a substrate or within a channel, a plurality of microbeads or microspheres within a reaction volume, or the like. Sample holder 305 may comprise a microtiter plate, for example, wherein reaction regions 308 may comprise at least 96 well, at least 384, or at least 1536 wells.

In certain embodiments, sample holder 305 may comprise a substrate including a first surface, an opposing second surface, and a plurality of through-holes disposed between the surfaces, the plurality of through-holes configured to contain the one or more biological samples, for example as discussed in Patent Application Publication Numbers US 2014-0242596 and WO 2013/138706. In such embodiments, the substrate may comprise at least 3096 through-holes or at least 20,000 through-holes. In certain embodiments, sample holder 305 may comprise an array of capillaries configured to pass one or more target molecules or sequence of molecules.

According to the invention, system 100 includes the heated cover 110, which may be disposed above sample holder 305 and/or sample block 114. Heated cover 110 may be used, for example, to prevent condensation above the samples contained in sample holder 305, which can help to maintain optical access to biological samples 114.

In certain embodiments, optical system 124 comprises an excitation source, illumination source, radiation source, or light source 1402 that produces at least a first excitation beam 1405a characterized by a first wavelength and a second excitation beam 1405b characterized by a second wavelength that is different from the first wavelength. Optical system 124 also comprises an optical sensor or optical detector 1408 configured to receive emissions or radiation from one or more biological samples in response to excitation source 1410 and/or to one or more of excitation beams 1405a, 1405b. Optical system 124 additionally comprises an excitation optical system 1410 disposed along an excitation optical path 1412 between excitation source 1402 and one or more biological samples to be illuminated. Optical system 124 further comprises an emission optical system 1415 disposed along an emission optical path 1417 between the illuminated sample(s) and optical sensor 1408. In certain embodiments, optical system 124 may comprise a beamsplitter 1420. Optical system 124 may optionally include a beam dump or radiation baffle 1422 configured reduce or prevent reflection of radiation into emission optical path 1417 from excitation source 1402 that impinges on beamsplitter 1420.

In the illustrated embodiment shown in FIG. 7, as well as other embodiments of the invention disclosed herein, excitation source 1402 comprises a radiation source 1425. Radiation source 1425 may comprise one or more of at least one an incandescent lamp, at least one gas discharge lamp, at least one light emitting diode, at least one organic light emitting diode, and/or at least one laser. For example, radiation source 1425 may comprise at least one Halogen lamp, Xenon lamp, Argon lamp, Krypton lamp, diode laser, Argon laser, Xenon laser, excimer laser, solid-state laser, Helium-Neon laser, dye laser, or combinations thereof. Radiation source 1425 may comprise a light source characterized by a maximum or central wavelength in the visible band of the electromagnetic spectrum. Additionally or alternatively, radiation source 1425 may comprise an ultraviolet, infrared, or near-infrared source with a corresponding maximum or central wavelength within on one of those wavelength bands of the electromagnetic spectrum. Radiation source 1425 may be a broadband source, for example, having a spectral bandwidth of at least 100 nanometers, at least 200 nanometers, or at least 300 nanometers, where the bandwidth is defined as a range over which the intensity, energy, or power output is greater than a predetermined amount (e.g., where the predetermined amount is at or about 1%, 5%, or 10% of a maximum or central wavelength of the radiation source). Excitation source 1402 may additionally comprise a source lens 1428 configured to condition emissions from radiation source 1425, for example, to increase the amount of excitation radiation received at sample holder 305 and/or into biological samples 114. Source lens 1428 may comprise a simple lens or may be a compound lens including two or more elements.

In certain embodiments, excitation source 1402 further comprises two or more excitation filters 1430 moveable into and out of excitation optical path 1412, for instance, used in combination with a broadband excitation source 1402. In such embodiments, different excitation filters 1430 may be used to select different wavelength ranges or excitation channels suitable for inducing fluorescence from a respective dye or marker within biological samples 114. One or more of excitation filters 1430 may have a wavelength bandwidth that is at least ±10 nanometers or at least ±15 nanometers. Excitation filters 1430 may comprise a plurality of filters that together provide a plurality of band passes suitable for fluorescing one or more of a SYBR® dye or probe, a FAMTM dye or probe, a VIC® dye or probe, a ROXTM dye or probe, or a TAMRATM dye or probe. Excitation filters 1430 may be arrange in a rotatable filter wheel (not shown) or other suitable device or apparatus providing different excitation channels using excitation source 1402. In certain embodiments, excitation filters 1430 comprise at least 5 filter or at least 6 filter.

In certain embodiments, excitation source 1402 may comprise a plurality of individual excitation sources that may be combined using one more beamsplitters or beam combiners such that radiation from each individual excitation source is transmitted along a common optical path, for example, along excitation optical path 1412 shown in FIG. 7. Alternatively, at least some of the individual excitation sources may be arranged to provided excitation beams that propagate along different, non-overlapping optical paths, for example, to illuminate different reaction regions of the plurality of reaction regions 308. Each of the individual excitation sources may be addressed, activated, or selected to illuminate reaction regions 308, for example, either individually or in groups or all simultaneously. In certain embodiments, the individual excitation sources may be arrange in a one-dimensional or two-dimensional array, where one or more of the individual excitation sources is characterized by a maximum or central wavelength that is different than that of at least one of the other individual excitation sources in the array.

In certain embodiments, first excitation beam 1405a comprises a first wavelength range over which an intensity, power, or energy of first excitation beam 1405a is above a first predetermined value and second excitation beam 1405b comprises a second wavelength range over which an intensity, power, or energy of second excitation beam 1405b is above a second predetermined value. The characteristic wavelength of the excitation beams 1405a, 1405b may be a central wavelength of the corresponding wavelength range or a wavelength of maximum electromagnetic intensity, power, or energy over the corresponding wavelength range. The central wavelengths of at least one of the excitation beams 1405 may be an average wavelength over the corresponding wavelength range. For each excitation beam 1405 (e.g., excitation beams 1405a, 1405b), the predetermined value may be less than 20% of the corresponding maximum intensity, power, or energy; less than 10% of the corresponding maximum intensity, power, or energy; less than 5% of the corresponding maximum intensity, power, or energy; or less than 1% of the corresponding maximum intensity, power, or energy. The predetermined values may be the same for all excitation beams 1405 (e.g., for both excitation beams 1405a, 1405b) or the predetermined values may be different from one another. In certain embodiments, the wavelength ranges of the first and second excitation beams 1405a, 1405b do not overlap, while in other embodiments at least one of the wavelength ranges at least partially overlaps that of the other. In certain embodiments, the first and second central wavelengths are separated by at least 20 nanometers. In certain embodiments, at least one of the first and second wavelength ranges has a value of at least 20 nanometer or at least 30 nanometers.

Excitation optical system 1410 is configured to direct excitation beams 1405a, 1405b to the one or more biological samples. Where applicable, references herein to excitation beams 1405a, 1405b may be applied to embodiment comprising more than two excitation beams 1405. For example, excitation source 1402 may be configured to direct at least five or six excitation beams 1405. Excitation beams 1405a, 1405b may be produced or provided simultaneously, may be temporally separated, and/or may be spatially separated (e.g., wherein excitation beams 1405a is directed to one reaction region 308 and excitation beams 1405b is directed to a different reaction region 308). The excitation beams 1405 may be produced sequentially, for example, by sequentially turning on and off different-colored individual radiation source 1425 that are characterized by different wavelengths or by sequentially placing different color filters in front of a single radiation source 1425. Alternatively, excitation beams 1405a, 1405b may be produced simultaneously, for example, by using a multi-wavelength band filter, beamsplitter, or mirror, or by coupling together different individual radiation source 1425, such as two different-colored light emitting diodes (LEDs). In some embodiments, excitation source 1402 produces more than two excitation beams 1405, wherein excitation optical system 1410 directs each of the excitation beams to one or more biological samples 114.

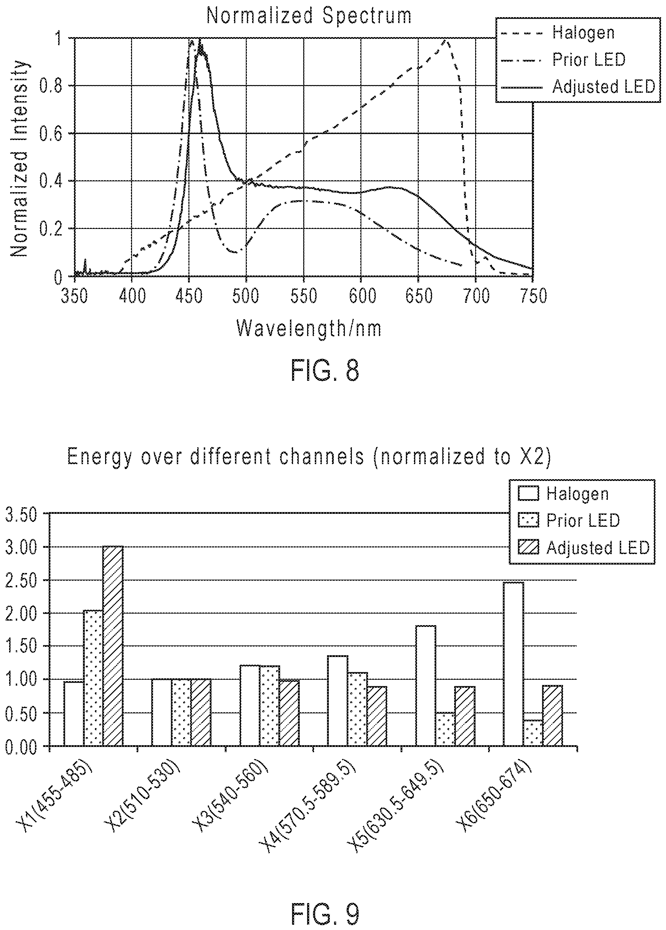

Referring to FIGS. 8-9, the spectral distribution of radiation source 1425 may be selected in a non-obvious manner to enable at least five excitation beams 1405 of different colors or excitation channels to be used with one common beamsplitter 1420, while simultaneously maintaining acceptable or predetermined data throughput for all excitation channels, for example, during each cycle of the qPCR assay. As used herein, the term "excitation channel" means each of several, distinct electromagnetic wavelength bands providing by an excitation source (e.g., excitation source 1402) that are configured to illuminate one or more biological samples. As used herein, the term "emission channel" means each of several, distinct emission wavelength bands over which electromagnetic radiation is allowed to pass onto an optical sensor or detector (e.g., optical sensor 1408).

FIG. 8 shows the relative energy over the wavelength spectrum for three different radiation sources. The dashed line plot is the spectrum of a Halogen lamp (herein referred to as "Source 1") characterized by relatively low energy levels in the blue wavelength range of the visible spectrum and increasing energy until a peak at about 670 nanometers. The dash-dot spectrum plot is that of a commercially available LED light source (herein referred to as "Source 2"), which has peak energy at around 450 nanometers and a lower peak from about 530 nanometers to about 580 nanometers, then steadily decreasing energy into the red wavelength range of the visible spectrum. The solid line plot is the spectrum of another LED light source (herein referred to as "Source 3") according to an embodiment of the present invention (e.g., an exemplary spectrum for excitation source 402). FIG. 9 shows integrated energy over various spectral ranges for each of the three sources shown in FIG. 8, where the spectrums are those of typical excitation filter used in the field of qPCR. The wavelength ranges and excitation filter designations are shown below in Table 1.

Table 1. Spectral bandwidth of excitation filters used in FIG. 9.

| Excitation Filter Channel | Wavelength Range (nanometers) |
|---|---|
| X1 | 455-485 |
| X2 | 510-530 |
| X3 | 540-560 |
| X4 | 570.5-589.5 |
| X5 | 630.5-649.5 |
| X6 | 650-674 |

In the field of qPCR, one important performance parameter is the total time to obtain emission data for samples containing multiple target dyes. For example, in some cases it is desirable to obtain emission data over 5 or 6 dyes or filter channels (e.g., X1-X5 / M1-M5 or X1-X6 / M1-M6, where "M" stands for emission channel number for a corresponding X (excitation) channel number). The inventors have found that when Source 2 is used in a system having a single, broadband beamsplitter for six EX/EM filter channels (e.g., excitation channels X1-X6 and corresponding emission channels M1-M6), the amount of time to obtain data for channel 5 and/or channel 6 could be unacceptably long for certain applications. To remedy this situation, it is possible to use one or more narrow band, dichroic beamsplitters for excitation channels 1 and/or 2 to increase the amount of excitation light receive by the sample(s), and the amount of emission light received by the sensor (so that the overall optical efficiency is increased by using dichroic beam splitter, in this case). However, this precludes a single beamsplitter arrangement, as shown in FIG. 7, and the corresponding advantages of a single beamsplitter configuration (e.g., reduced size, cost, complexity). A better solution has been discovered in which a light source such as Source 3 is used in combination with a single beamsplitter (e.g., a broadband beamsplitter such as a 50/50 beamsplitter). It has been found that the relative energy in excitation channels X1, X5, and/or X6 may be used to identify an excitation source 402 suitable for use with a single beamsplitter embodiment. Using Source 2 and Source 3 as examples, the following data shown in Table 2 below may be derived for the data shown in FIGS. 8 and 9.

**Table 2.** Normalized LED intensity of each filter channel with normalization over channel 2.

| Ratio | Source 2 | Source 3 |
|-------|----------|----------|
| X1/X2 | 2.02 | 3.00 |
| X2/X2 | 1.00 | 1.00 |
| X3/X2 | 1.20 | 0.98 |
| X4/X2 | 1.09 | 0.89 |
| X5/X2 | 0.49 | 0.90 |
| X6/X2 | 0.38 | 0.90 |

Based on such date, the inventors have found that, in certain embodiments, improved performance (e.g., in terms of shorter Channel 1 integration time) may be obtain when X1/X2 is greater than 2.5 (e.g., greater than or equal to 3). Additionally or alternatively, in other embodiments, improved performance (e.g., in terms of shorter Channel 1 integration time) may be obtain when X5/X2 is greater than 0.7 (e.g., greater than or equal to 0.9) and/or when X6/X2 is greater than 0.7 (e.g., greater than or equal to 0.9).

Referring again to FIG. 7, excitation beams 1405 are directed along excitation optical path 1412 during operation toward sample processing sample block 114, for example, toward reaction regions 308 when sample holder 305 is present. When present, source lens 1428 is configure to condition excitation beams 1405, for example, to capture and direct a large portion of the emitted radiation from excitation source 1402. In certain embodiments, one or more mirrors 1432 (e.g., fold mirrors) may be incorporated along excitation optical path 1412, for example, to make optical system 124 more compact and/or to provide predetermined package dimensions. FIG. 7 illustrated one mirror 1432; however, addition mirrors may be used, for example to meet packaging design constraints. As discussed in greater detail below herein, additional lenses may be disposed near sample holder 305, for example, in order to further condition the excitation beams 1405 and/or corresponding emissions from biological samples contained in one or more reaction regions.

Emission optical system 1415 is configured to direct emissions from the one or more biological samples to optical sensor 1408. At least some of the emissions may comprise a fluorescent emission from at least some of the biological samples in response to at least one of the excitation beams 1405. Additionally or alternatively, at least some of the emissions comprise radiation from at least one of the excitation beams 1405 that is reflected, refracted, diffracted, scattered, or polarized by at least some of the biological samples. In certain embodiments, emission optical system 1415 comprise one or more emission filters 1435 configured, for example, to block excitation radiation reflected or scattered into emission optical path 1417. In certain embodiments, there is a corresponding emission filter 1435 for each excitation filter 1430.

In certain embodiments, emission optical system 1415 comprises a sensor lens 1438 configured to direct emissions from at least some of the biological samples onto optical sensor 1408. Optical sensor 1408 may comprise a single sensor element, for example, a photodiode detector or a photomultiplier tube, or the like. Additionally or alternatively, optical sensor 1408 may comprise an array sensor including an array of sensors or pixels. Array sensor 1408 may comprise one or more of a complementary metal-oxide-semiconductor sensor (CMOS), a charge-coupled device (CCD) sensor, a plurality of photodiodes detectors, a plurality of photomultiplier tubes, or the like. Sensor lens 1438 may be configured to from an image from the emissions from one or more of the plurality of biological samples 114. In certain embodiments, optical sensor 1408 comprises two or more array sensors 1408, for example, where two or more images are formed from the emissions from one or more of the plurality of biological samples 114. In such embodiments, emissions from one or more of the plurality of biological samples 114 may be split to provide two signals of the one or more of the plurality of biological samples 114. In certain embodiments, the optical sensor comprises at least two array sensors.

Beamsplitter 1420 is disposed along both excitation and emission optical paths 1412, 1417 and is configured to receive both first and second excitation beams 1405a, 1405b during operation. In the illustrated embodiment shown in FIG. 7, beamsplitter 1420 is configured to transmit the excitation beams 1405 and to reflect emissions from the biological samples 114. Alternatively, beamsplitter 1420 may be configured to reflect the excitation beams and to transmit emissions from the biological samples 114. In certain embodiments, beamsplitter 1420 comprises a broadband beamsplitter having the same, or approximately the same, reflectance for all or most of the excitation beams 1405 provided by excitation source 1402 and directed to the reaction regions 308 (e.g., excitation beams 1405a, 1405b in the illustrated embodiment). For example, beamsplitter 1420 may be a broadband beamsplitter characterized by a reflectance that is constant, or about constant, over a wavelength band of at least 100 nanometers, over a wavelength band of at least 200 nanometers, or over the visible wavelength band of the electromagnetic spectrum, over the visible and near IR wavelength bands of the electromagnetic spectrum, or over a wavelength band from 450 nanometers to 680 nanometers. In certain embodiments, beamsplitter 1420 is a neutral density filter, for example, a filter having a reflectance of, or about, 20%, 50%, or 80% over visible wavelength band of the electromagnetic spectrum. In certain embodiments, beamsplitter 1420 is a dichroic beamsplitter that is transmissive or reflective over one or more selected wavelength ranges, for example, a multi-wavelength band beamsplitter that is transmissive and/or reflective over more than one band of wavelengths centers at or near a peak wavelength of excitation beams 1405.

In certain embodiments, beamsplitter 1420 is a single beamsplitter configure to receive some or all of the plurality of excitation beams 1405 (e.g., excitation beams 1405a, 1405b), either alone or in combination with a single beam dump 1422. Each excitation beam may be referred to as an excitation channel, which may be used alone or in combination to excite different fluorescent dyes or probe molecule in one or more of the biological samples 114. By contrast many prior art systems and instruments, for example, in the field of qPCR, provide a plurality of excitation beams by using a separate beamsplitter and/or beam dump for each excitation channel and/or each emission channel of the system or instrument. In such prior art systems and instruments, chromatically selective dichroic filters are typically used in at least some of the excitation channels to increase the amount of radiation received at the samples. Disadvantages of systems and instruments using different beamsplitters and/or beam dumps for each channel include an increase in size, cost, complexity, and response time (e.g., dues to increased mass that must be moved or rotated when changing between excitation and/or emission channels). The inventors have discovered that it is possible to replace these plural beamsplitters and/or beam dumps with the single beamsplitter 1420 and/or single beam dump 1422, while still providing an acceptable or predetermined system or instrument performance, for example, by proper selection of spectral distribution of excitation source 1402 and/or by configuring the systems or instruments to reduce the amount of stray or unwanted radiation received by optical sensor 408 (as discuss further herein). Thus, embodiments of the present invention may be used to provide systems and instruments that have reduced size, cost, complexity, and response time as compared to prior art systems and instruments.

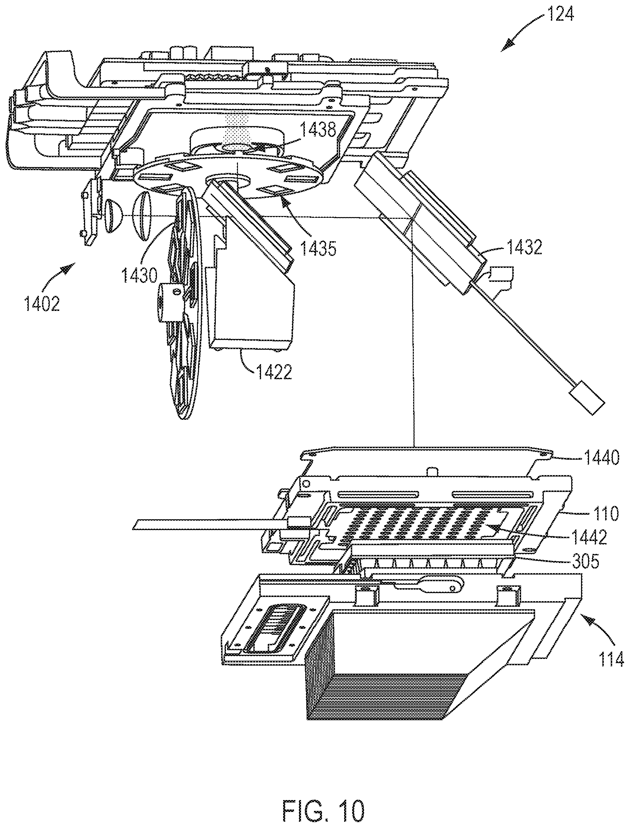

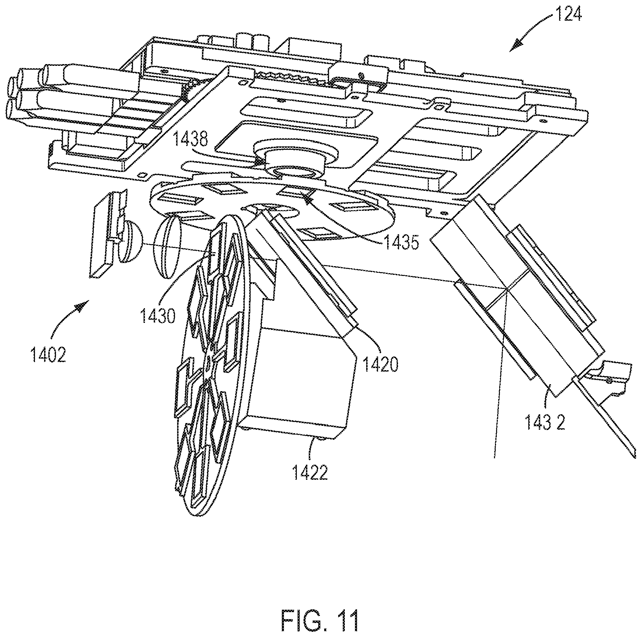

Referring to FIGS. 10-11, in certain embodiments, optical system 124 may further comprise a lens 1440 and/or a lens array 1442, which may comprise a plurality of lenses corresponding to each of the reaction regions 308 of sample holder 305. Lens 1440 may comprises a field lens, which may be configured to provide a telecentric optical system for a least one of sample holder 305, reaction regions 308, lens array 1442, or optical sensor 1408. As shown in illustrated embodiment in FIG. 10, lens 1440 may comprise a Fresnel lens.

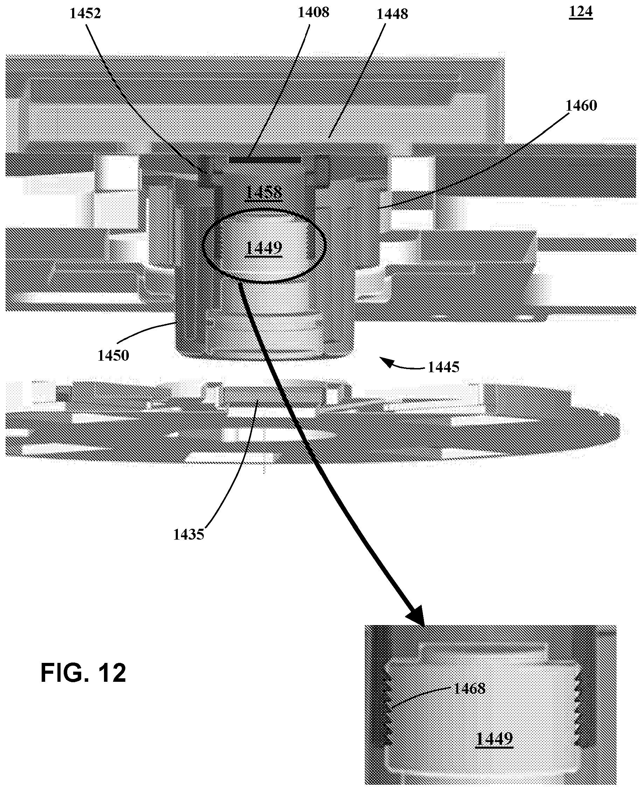

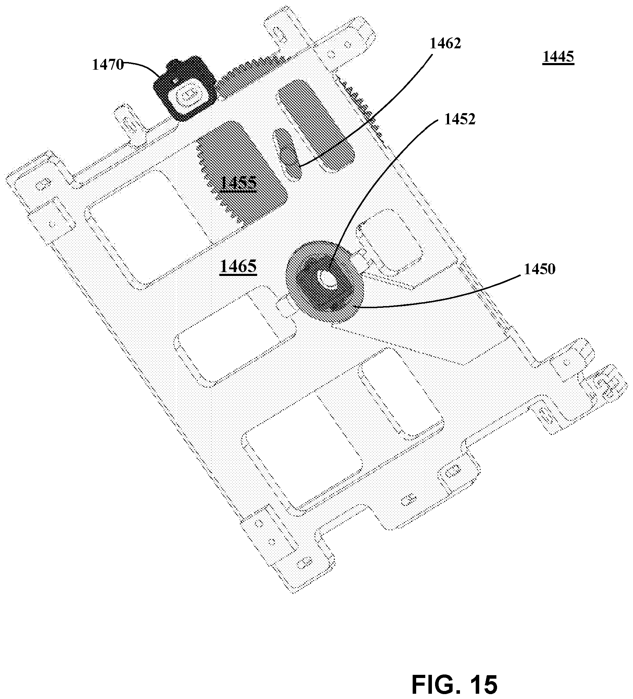

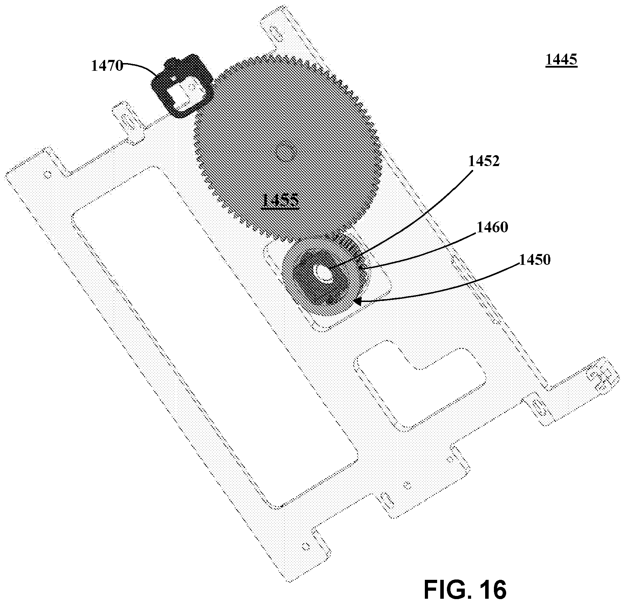

With additional reference to FIGS. 12-16, in certain embodiments, optical system 124 includes an imaging unit 1445 comprising an optical sensor circuit board 1448, sensor lens 1438(which may be a compound lens, as illustrated in FIG. 12), an inner lens mount 1449, an outer lens mount 1450, a threaded housing 1452, and a focusing gear 1455. Optical sensor circuit board 1448, threaded housing 1452, and sensor lens 1438 together may form a cavity 1458 that encloses or contains optical sensor 1408 and may be configured to block any external light from impinging optical sensor 1408 that does not enter through sensor lens 1438. Outer lens mount 1450 comprises an outer surface containing gear teeth 1460 that may be moveably or slidably engaged with the teeth of focusing gear 1455 via a resilient element (not shown), such as a spring. In certain embodiments, focusing gear 1455 moves or slide along a slot 1462 of a plate 1465, as illustrated in FIG. 16. Inner lens mount 1449 comprises a threaded portion 1468 that engages or mates with a threaded portion of threaded housing 1452.

Inner lens mount 1449 may be fixedly mounted to outer lens mount 1450, while threaded housing 1452 is fixedly mounted relative to optical sensor circuit board 1448. Inner lens mount 1449 is moveably or rotatably mounted to threaded housing 1452. Thus, focusing gear 1455 and outer lens mount 1450 may be engaged such that a rotation of focusing gear 1455 also rotates outer lens mount 1450. This, in turn, causes inner lens mount 1449 and sensor lens 1438 to move along an optical axis of sensor lens 1438 via the threads in inner lens mount 1449 and threaded housing 1452. In this manner, the focus of sensor lens 1438 may be adjusted without directly engaging sensor lens 1438 or its associated mounts 1449, 1450, which are buried within a very compact optical system 124. Engagement with focusing gear 1455 may be either by hand or automated, for example using a motor (not shown), such as a stepper motor or DC motor.

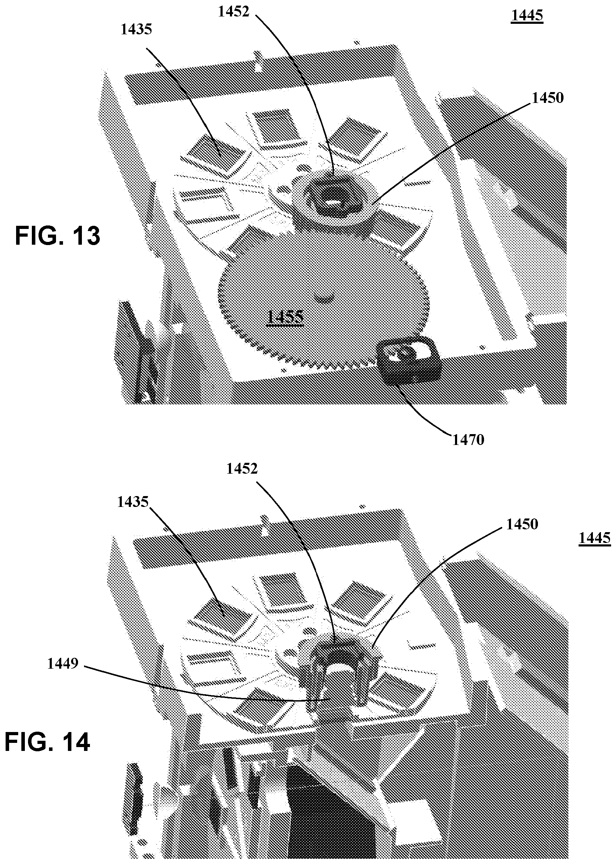

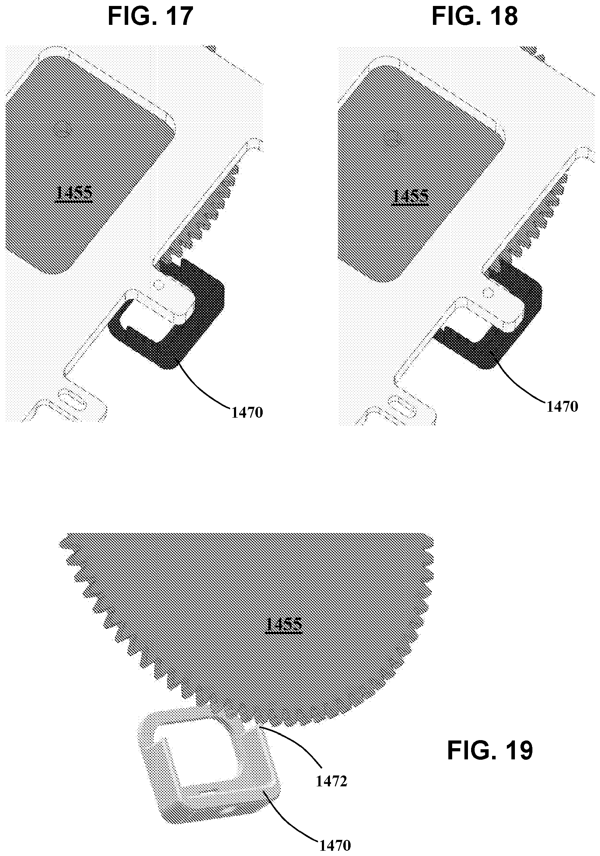

Referring to FIGS. 13 and 15-19, in certain embodiments, imaging unit 1445 further comprises a locking device or mechanism 1470. Locking device 1470 comprises an edge or tooth 1472 that may be slidably engage between two teeth of focusing gear 1455 (see FIGS. 17-19). As illustrated in FIGS. 17 and 18, locking device 1470 may have a first position (FIG. 17) in which focusing gear 1455 is free to rotate and adjust the focus of sensor lens 1438 and a second position (FIG. 18) is which focusing gear 1455 is locked in position and impeded or prevented from rotating. In this manner, the focus of sensor lens 1438 may be locked while advantageously avoiding direct with threads 1468 of inner lens mount 1449, which could damage the threads and prevent subsequent refocusing of sensor lens 1438 after being locked into position. Operation of locking device 1470 may be either manually or in an automated manner. In certain embodiments, locking mechanism 1470 further comprises a resilient element (not shown), wherein rotation of focusing gear 1455 may be accomplished by overcoming a threshold force produced by the resilient element.

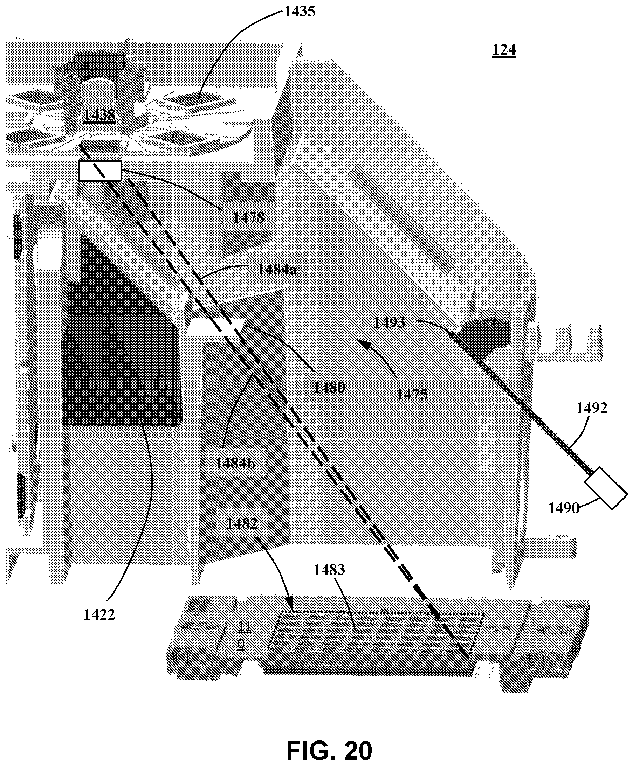

Referring to FIG. 20, optical system 124 may also include an optics housing 1477. In certain embodiments, optical system 124 includes a radiation shield 1475 comprising a sensor aperture 1478 disposed along emission optical path 1417 and at least one blocking structure 1480 disposed to cooperate with the sensor aperture 1478 such that the only radiation from excitation beams 1405, and reflected off an illuminated area 1482, to pass through sensor aperture 1478 is radiation that has also reflected off at least one other surface of, or within, the optics housing 1477. In other words, radiation shield 1475 is configured such that radiation from excitation beams 1405 reflected illuminated area 1482 are blocked from directly passing through aperture 1478 and, therefore, from passing into sensor lens 1438 and onto optical detector 1408. In certain embodiments, illuminated area 1482 comprises the area defined by all the apertures 1483 of heated cover 110 corresponding to the plurality of reaction regions 308.

In the illustrated embodiment of FIG. 20, blocking structure 1480 comprises a shelf 1480. Dashed lines or rays 1484a and 1484b may be used to illustrate the effectiveness of blocking structure 1480 in preventing light directly reflected from illuminated area 1482 from passing through sensor aperture 1478 and onto senor lens 1438 and/or optical sensor 1408. Ray 1484a originates from an edge of illuminated area 1482 and just passes shelf 1480, but does not pass through sensor aperture 1478. Ray 1484b is another ray originating from the same edge of illuminated area 1482 that is blocked by shelf 1480. As can be seen, this ray would have entered through sensor aperture 1478 were it not for the presences of shelf 1480.

With continued reference to FIG. 20, in certain embodiments, optical system 124 may further comprise an energy or power detection unit comprising a power or energy sensors 1490 optically coupled to one end of a light pipe 1492. An opposite end 1493 of light pipe 1492 is configured to be illuminated by excitation beams 1405. Light pipe end 1493 may be illuminated either directly by radiation contained in excitation beams 1405 or indirectly, for example, by radiation scattered by a diffuse surface. In certain embodiments, sensor 1490 is located outside of the excitation optical path 1412 from excitation source 1402. Additionally or alternatively, sensor 1490 is located outside optics housing 1477 and/or is located at a remote location outside instrument housing 105. In the illustrated embodiment shown in FIG. 20, light pipe end 1493 is disposed near or adjacent mirror 1432 and may be oriented so that the face of the light pipe is perpendicular, or nearly perpendicular, to the surface of mirror 1432 that reflects excitation beams 1405. The inventors have discovered that the low amount of energy or power intercepted by light pipe 1492 when oriented in this way is sufficient for the purpose of monitoring the energy or power of excitation beams 1405. Advantageously, by locating sensor 1490 outside the optical path of excitation beams a more compact optical system 124 may be provided.

In certain embodiments, light pipe 1492 comprises a single fiber or a fiber bundle. Additionally or alternatively, light 1492 may comprise a rod made of a transparent or transmissive material such as glass, Plexiglas, polymer based material such as acrylic, or the like.

Further aspects of optical system 124 can also be described as follows:

In alternative embodiment 1, an instrument for biological analysis is provided, comprising: a base configured to receive a sample holder comprising a plurality of spatially separated reaction regions for processing one or more biological samples, the base comprising a thermal cycler configured to perform a polymerase chain reaction assay on the separated biological samples; an excitation source configured to produce a first excitation beam characterized by a first wavelength and a second excitation beam characterized by a second wavelength that is different from the first wavelength; an optical sensor configured to receive emissions from the biological samples in response to the excitation source; an excitation optical system disposed along an excitation optical path between the excitation source and the sample holder, the excitation optical system comprising a sample lens disposed to direct the excitation beams toward the sample holder; an emission optical system disposed along an emission optical path between the sample holder and the optical sensor, the emission optical system configured to direct the emissions from the biological samples to the optical sensor; a beamsplitter disposed along both the excitation optical path and along the emission optical path, the beamsplitter disposed to receive the first excitation beam and to receive the second excitation beam, the sample lens disposed along the excitation optical path between the beamsplitter and the base; a beam dump configured to receive excitation beam radiation from the beamsplitter and to reflect back less than less than 10% of the excitation beam radiation back toward the beamsplitter; an imaging unit comprising: a bottom surface and an opposing top surface including an optical sensor circuit board; a sensor lens at least partially enclosed by a lens case, the bottom surface comprising a surface of the sensor lens; and a focusing mechanism comprising a gear that engages the lens case, the focusing mechanism being accessible outside the enclosure for adjusting a focus of the sensor lens; an illuminated surface disposed along the excitation optical path between the beamsplitter and the base, the illuminated surface configured to produce during use reflected radiation comprising radiation from the excitation source that is reflected by the illuminated surface; a radiation shield, comprising: a sensor aperture dispose along the emission optical path between the beamsplitter and the sensor lens; and a blocking structure disposed to cooperate with the sensor aperture during use such that none of the reflected radiation is received by the optical sensor that does not also reflect off another surface of the instrument; an energy or power detection unit comprising: an energy or power sensor located outside the optical paths; and a light pipe disposed adjacent the beamsplitter and configured to transport radiation from the beamsplitter to the power sensor; a position source configured to emit radiation and a corresponding position sensor configured to receive radiation from the position source, the position source and the position sensor configured to produce a position signal indicative of a position of an optical element disposed along at least one of the optical paths; a radiation shield configured to block at least some radiation from the position source; an optical enclosure enclosing the optical paths, the enclosure comprising a split wire grommet configured to pass wires or cable between a location outside the enclosure to a location inside the enclosure while blocking light outside the enclosure from entering the enclosure; a lens hole cover configured to allow three dimensional adjustment of the sensor lens while blocking light outside the enclosure from entering the enclosure; wherein the optical sensor is a complementary metal-oxide-semiconductor sensor.

In alternative embodiment 2, the instrument of claim 1 is provided, wherein the blocking structure is disposed to cooperate with the sensor aperture during use such that none of the reflected radiation impinges on the sensor lens that does not also reflect off another surface of the instrument.

In alternative embodiment 3, an instrument for biological analysis is provided, comprising: a base configured to receive a sample holder comprising a plurality of spatially separated reaction regions for processing one or more biological samples; an excitation source configured to produce a first excitation beam characterized by a first wavelength and a second excitation beam characterized by a second wavelength that is different from the first wavelength; an optical sensor configured to receive emissions from the biological samples in response to the excitation source; an excitation optical system disposed along an excitation optical path between the excitation source and the sample holder; an emission optical system disposed along an emission optical path between the sample holder and the optical sensor, the emission optical system configured to direct the emissions from the biological samples to the optical sensor; an imaging unit comprising: a bottom surface and an opposing top surface including an optical sensor circuit board; a sensor lens at least partially enclosed by a lens case, the bottom surface comprising a surface of the sensor lens; and a focusing mechanism comprising a gear that engages the lens case, the focusing mechanism being accessible outside the enclosure for adjusting a focus of the sensor lens.

In alternative embodiment 4, an instrument for biological analysis is provided, comprising: a base configured to receive a sample holder comprising a plurality of spatially separated reaction regions for processing one or more biological samples; a thermal controller configured to control a temperature of at least one of base, the sample holder, or the separated biological samples; an excitation source configured to produce a first excitation beam characterized by a first wavelength and a second excitation beam characterized by a second wavelength that is different from the first wavelength; an optical sensor configured to receive emissions from the biological samples in response to the excitation source; an excitation optical system disposed along an excitation optical path between the excitation source and the sample holder; an emission optical system disposed along an emission optical path between the sample holder and the optical sensor, the emission optical system configured to direct the emissions from the biological samples to the optical sensor; a sensor lens configured to direct emissions from at least some of the biological sample onto the optical sensor; an illuminated surface disposed along the excitation optical path between the beamsplitter and the base, the illuminated surface configured to produce during use reflected radiation comprising radiation from the excitation source that is reflected by the illuminated surface; a radiation shield, comprising: a sensor aperture dispose along the emission optical path between the beamsplitter and the sensor lens; and a blocking structure disposed to cooperate with the sensor aperture during use such that none of the reflected radiation is received by the optical sensor that does not also reflect off another surface of the instrument.

In alternative embodiment 5, an instrument for biological analysis is provided, comprising: a base configured to receive a sample holder comprising a plurality of spatially separated reaction regions for processing one or more biological samples; an excitation source configured to produce a first excitation beam characterized by a first wavelength and a second excitation beam characterized by a second wavelength that is different from the first wavelength; an optical sensor configured to receive emissions from the biological samples in response to the excitation source; an excitation optical system disposed along an excitation optical path between the excitation source and the sample holder; an emission optical system disposed along an emission optical path between the sample holder and the optical sensor, the emission optical system configured to direct the emissions from the biological samples to the optical sensor; an energy or power detection unit comprising: an energy or power sensor located outside the optical paths; and a light pipe disposed adjacent the beamsplitter and configured to transport radiation from the beamsplitter to the power sensor.

In alternative embodiment 6, an instrument for biological analysis is provided, comprising: a base configured to receive a sample holder comprising a plurality of spatially separated reaction regions for processing one or more biological samples; an excitation source configured to produce a first excitation beam characterized by a first wavelength and a second excitation beam characterized by a second wavelength that is different from the first wavelength; an optical sensor configured to receive emissions from the biological samples in response to the excitation source; an excitation optical system disposed along an excitation optical path between the excitation source and the sample holder; an emission optical system disposed along an emission optical path between the sample holder and the optical sensor, the emission optical system configured to direct the emissions from the biological samples to the optical sensor; a position source configured to emit radiation and a corresponding position sensor configured to receive radiation from the position source, the position source and the position sensor configured to produce a position signal indicative of a position of an optical element disposed along at least one of the optical paths; a radiation shield configured to block at least some radiation from the position source.

In alternative embodiment 7, an instrument for biological analysis is provided, comprising: a base configured to receive a sample holder comprising a plurality of spatially separated reaction regions for processing one or more biological samples; an excitation source configured to produce a first excitation beam characterized by a first wavelength and a second excitation beam characterized by a second wavelength that is different from the first wavelength; an optical sensor configured to receive emissions from the biological samples in response to the excitation source; an excitation optical system disposed along an excitation optical path between the excitation source and the sample holder; an emission optical system disposed along an emission optical path between the sample holder and the optical sensor, the emission optical system configured to direct the emissions from the biological samples to the optical sensor; a beamsplitter disposed along both the excitation optical path and along the emission optical path, the beamsplitter disposed to receive the first excitation beam and to receive the second excitation beam. an optical enclosure enclosing the optical paths, the enclosure comprising a split wire grommet configured to pass wires or cable between a location outside the enclosure to a location inside the enclosure while blocking light outside the enclosure from entering the enclosure; a lens hole cover configured to allow three dimensional adjustment of the sensor lens while blocking light outside the enclosure from entering the enclosure.

In alternative embodiment 8, an instrument for biological analysis is provided, comprising: a base configured to receive a sample holder comprising a plurality of spatially separated reaction regions for processing one or more biological samples; an excitation source configured to produce a first excitation beam characterized by a first wavelength and a second excitation beam characterized by a second wavelength that is different from the first wavelength; an optical sensor configured to receive emissions from the biological samples in response to the excitation source; an excitation optical system disposed along an excitation optical path between the excitation source and the sample holder; an emission optical system disposed along an emission optical path between the sample holder and the optical sensor, the emission optical system configured to direct the emissions from the biological samples to the optical sensor; a beamsplitter disposed along both the excitation optical path and along the emission optical path, the beamsplitter disposed to receive the first excitation beam and to receive the second excitation beam, wherein the optical sensor is a complementary metal-oxide-semiconductor sensor.

In alternative embodiment 9, the instrument of any of embodiments 1, [00125], [00127], [00127], [00128], [00129], or [00130] is provided, further comprising one or more emission filters disposed along the emission optical path.

In alternative embodiment 10, the instrument of any of embodiments 1, [00125], [00127], [00127], [00128], [00129], or [00130] is provided, wherein at least some of the emissions comprise a fluorescent emission from at least some of the biological samples in response to at least one of the excitation beams.

In alternative embodiment 11, the instrument of any of embodiments 1, [00125], [00127], [00127], [00128], [00129], or [00130] is provided, wherein at least some of the emissions comprise a fluorescent emission from at least some of the biological samples in response to at least one of the excitation beams.

In alternative embodiment 12, the instrument of any of embodiments 1, [00125], [00127], [00127], [00128], [00129], or [00130] is provided, wherein at least some of the emissions comprise radiation from at least one of the excitation beams that is reflected, refracted, diffracted, scattered, or polarized by at least some of the biological samples.

In alternative embodiment 13, the instrument of any of embodiments 1, [00125], [00127], [00127], [00128], [00129], or [00130], further comprising a temperature controlled cover disposed along the excitation optical path between the base and the beamsplitter.

In alternative embodiment 14, the instrument of embodiment [00135] is provided, further comprising a mirror disposed along the excitation optical path between the base and the beamsplitter.

In alternative embodiment 15, the instrument of any of embodiments 1, [00125], [00127], [00127], [00128], [00129], or [00130] is provided, further comprising a mirror disposed along the excitation optical path between the base and the beamsplitter.

In alternative embodiment 16, the instrument of any of embodiments 1, [00125], [00127], [00127], [00128], [00129], or [00130] is provided, wherein the base comprises a sample block assembly configured to control the temperature of the sample holder or biological samples.

In alternative embodiment 17, the instrument of embodiment [00138] is provided, wherein sample block assembly comprises one or more of a sample block, a Peltier device, or a heat sink.

In alternative embodiment 18, the instrument of any of embodiments [00125], [00127], [00127], [00128], [00129], or [00130] is provided, wherein base comprises a thermal cycler configured to perform a PCR assay.

In alternative embodiment 19, the instrument of any of embodiments 1, [00125], [00127], [00127], [00128], [00129], or [00130] is provided, wherein the instrument includes the sample holder.

In alternative embodiment 20, the instrument of embodiment [00141] is provided, wherein the sample holder comprises one or more of a microtiter plate, substrate comprising a plurality of wells or through-holes, a substrate comprising a one or more channels, or a chamber comprising plurality of beads or spheres containing the one or more biological samples.

In alternative embodiment 21, the instrument of embodiment [00141] is provided, wherein the plurality of spatially separated reaction regions comprise one or more of a plurality of wells, a plurality of through-holes in substrate, a plurality of distinct locations on a substrate or within a channel, or a plurality of beads or sphere within a reaction volume.

In alternative embodiment 22, the instrument of embodiment [00141] is provided, wherein at least some of the spatially separated reaction regions comprise the one or more biological samples.

In alternative embodiment 23, the instrument of embodiment [00144] is provided, wherein the one or more biological samples comprise one or more of at least one target nucleic acid sequence, at least one primer, at least one buffer, at least one nucleotide, at least one enzyme, at least one detergent, at least one blocking agent, or at least one dye, marker, and/or probe suitable for detecting a target or reference nucleic acid sequence.

In alternative embodiment 24, the instrument of embodiment [00141] is provided, wherein the sample holder comprises a microtiter plate and the reaction regions comprise at least 96 well, at least 384, or at least 1536 wells.

In alternative embodiment 25, the instrument of embodiment [00141] is provided, wherein the sample holder comprises a substrate including a first surface, an opposing second surface, and a plurality of through-holes disposed between the surfaces, the plurality of through-holes configured to contain the one or more biological samples.

In alternative embodiment 26, the instrument of embodiment [00147] is provided, wherein the substrate comprises at least 3096 through-holes or at least 20,000 through-holes.

In alternative embodiment 27, the instrument of embodiment [00141] is provided, wherein the sample holder comprises an array of capillaries configured to pass one or more target molecules or sequence of molecules.

In alternative embodiment 28, the instrument of embodiment [00141] is provided, wherein the sample holder is configured to perform at least one of a polymerase chain reaction, a sequencing assay, or a capillary electrophoresis assay, a blot assay.

In alternative embodiment 29, the instrument of any of embodiments [00125], [00127], [00127], [00128], [00129], or [00130] is provided, wherein the excitation optical system comprising a sample lens configured to direct the excitation beams toward the base.

In alternative embodiment 30, the instrument of any of embodiments 1 or [00151] is provided, wherein the sample lens comprises a field lens extending over the plurality of spatially separated regions during use.

In alternative embodiment 31, the instrument of any of embodiments 1 or [00151] is provided, wherein the sample lens comprises at least one of a field lens extending over the plurality of spatially separated regions during use or a plurality of lenses, each of the plurality of lenses disposed over a respective one of the plurality of reaction regions during use.

In alternative embodiment 32, the instrument of any of embodiments 1 or [00151] is provided, wherein the sample lens comprises at least one of a compound lens, a curved mirror, a diffractive optical element, or a holographic optical element.

In alternative embodiment 33, the instrument of embodiment 1 is provided, wherein, during use, the sample lens provides a telecentric optical system for a least one of the sample holder, the spatially separated reaction regions, or the optical sensor.

In alternative embodiment 34, the instrument of embodiment [00151] is provided, wherein, during use, the sample lens provides a telecentric optical system for a least one of the sample holder, the spatially separated reaction regions, or the optical sensor.

In alternative embodiment 35, the instrument of any of embodiments 1 or [00151] is provided, wherein the sample lens comprises a Fresnel lens.

In alternative embodiment 36, the instrument of any of embodiments 1 or [00151] is provided, wherein the sample lens comprises a plurality of lenses corresponding to the plurality of reaction regions.

In alternative embodiment 37, the instrument of any of embodiments 1, [00125], [00127], [00127], [00128], [00129], or [00130] is provided, wherein the beamsplitter is configured during use to transmit the excitation beams or is configured during use to reflect the excitation beams.

In alternative embodiment 38, the instrument of any of embodiments 1, [00125], [00127], [00127], [00128], [00129], or [00130] is provided, wherein the beamsplitter is comprises a broadband beamsplitter characterized by a reflectance that is constant over a wavelength band of at least 100 nanometers.

In alternative embodiment 39, the instrument of any of embodiments 1, [00125], [00127], [00127], [00128], [00129], or [00130] is provided, wherein the beamsplitter is characterized by a reflectance that is constant over a wavelength band from 450 nanometers to 680 nanometers.

In alternative embodiment 40, the instrument of any of embodiments 1, [00125], [00127], [00127], [00128], [00129], or [00130] is provided, wherein the beamsplitter is characterized by a reflectance that is constant over the visible wavelength band of the electromagnetic spectrum.

In alternative embodiment 41, the instrument of any of embodiments 1, [00125], [00127], [00127], [00128], [00129], or [00130] is provided, wherein the beamsplitter comprises a neutral density filter.

In alternative embodiment 42, the instrument of any of embodiments 1, [00125], [00127], [00127], [00128], [00129], or [00130] is provided, wherein the beamsplitter comprises a 50/50 beamsplitter.

In alternative embodiment 43, the instrument of any of embodiments 1, [00125], [00127], [00127], [00128], [00129], or [00130] is provided, wherein the beamsplitter comprises a dichroic beamsplitter.

In alternative embodiment 44, the instrument of any of embodiments 1, [00125], [00127], [00127], [00128], [00129], or [00130] is provided, wherein the beamsplitter comprises a multi-wavelength band beamsplitter.

In alternative embodiment 45, the instrument of any of embodiments 1, [00125], [00127], [00127], [00128], [00129], or [00130] is provided, wherein the first excitation beam and the second excitation beam are temporally separated and/or spatially separated.

In alternative embodiment 46, the instrument of any of embodiments 1, [00125], [00127], [00127], [00128], [00129], or [00130] is provided, wherein the first excitation beam and the second excitation beam are produced simultaneously.

In alternative embodiment 47, the instrument of any of embodiments 1, [00125], [00127], [00127], [00128], [00129], or [00130] is provided, wherein the excitation light source comprises one or more of at least one an incandescent lamp, at least one gas discharge lamp, at least one light emitting diode, at least one organic light emitting diode, or at least one laser.

In alternative embodiment 48, the instrument of embodiment [00169] is provided, wherein the at least one gas discharge lamp comprises one or more of a Halogen lamp, a Xenon lamp, an Argon lamp, or a Krypton lamp.

In alternative embodiment 49, the instrument of embodiment [00169] is provided, wherein the at least one laser comprises one or more of a diode laser, an Argon laser, a Xenon laser, an excimer laser, a solid-state laser, a Helium-Neon laser, or a dye laser.

In alternative embodiment 50, the instrument of any of embodiments 1, [00125], [00127], [00127], [00128], [00129], or [00130] is provided, wherein the first excitation beam comprises a first wavelength range over which an intensity, power, or energy of the first excitation beam is above a first predetermined value, the second excitation beam comprises a second wavelength range over which an intensity, power, or energy of the second excitation beam is above a second predetermined value, the first wavelength is at least one of (1) a central wavelength of the first wavelength range or (2) a wavelength of maximum electromagnetic intensity, power, or energy over the first wavelength range, and the second wavelength is at least one of (1) a central wavelength of the second wavelength range or (2) a wavelength of maximum electromagnetic intensity, power, or energy over the second wavelength range.

In alternative embodiment 51, the instrument of embodiment [00172] is provided, wherein at least one of the central wavelengths is an average wavelength over a corresponding wavelength range.

In alternative embodiment 52, the instrument of embodiment [00172] is provided, wherein at least one of the predetermined values is less than 20% of a corresponding maximum intensity, power, or energy over a corresponding wavelength range.

In alternative embodiment 53, the instrument of embodiment [00172] is provided, wherein the second predetermined value is equal to the first predetermined value.

In alternative embodiment 54, the instrument of embodiment [00172] is provided, wherein the first wavelength range does not overlap the second wavelength range or the first wavelength range only partially overlaps the second wavelength range.

In alternative embodiment 55, the instrument of embodiment [00172] is provided, wherein the second wavelength differs from the first wavelength by at least 20 nanometers.

In alternative embodiment 56, the instrument of embodiment [00172] is provided, wherein at least one of the first and second wavelength ranges has a value of at least 20 nanometers.

In alternative embodiment 57, the instrument of any of embodiments 1, [00125], [00127], [00127], [00128], [00129], or [00130] is provided, wherein the second wavelength differs from the first wavelength by at least 20 nanometers.

In alternative embodiment 58, the instrument of any of embodiments 1, [00125], [00127], [00127], [00128], [00129], or [00130] is provided, wherein excitation source comprises a light source, and the first wavelength and the second wavelength being in the visible band of the electromagnetic spectrum.

In alternative embodiment 59, the instrument of any of embodiments 1, [00125], [00127], [00127], [00128], [00129], or [00130] is provided, wherein the excitation source comprises a light source having a bandwidth of at least 100 nanometers.

In alternative embodiment 60, the instrument of any of embodiments 1, [00125], [00127], [00127], [00128], [00129], or [00130] is provided, wherein the excitation source comprises a plurality of excitation filters moveable into and out of the excitation optical path.

In alternative embodiment 61, the instrument of embodiment [00182] is provided, wherein at least one of the excitation filters has a wavelength band of at least ±10 nanometers.

In alternative embodiment 62, the instrument of embodiment [00182] is provided, wherein the excitation filters comprise at least 5 excitation filters.

In alternative embodiment 63, the instrument of embodiment [00182] is provided, wherein the excitation filters comprise a plurality of filters together providing a plurality of band passes suitable for fluorescing one or more of a SYBR® dye or probe, a FAMTM dye or probe, a VIC® dye or probe, a ROXTM dye or probe, or a TAMRATM dye or probe.

In alternative embodiment 64, the instrument of embodiment [00182], wherein the excitation filters are mounted onto a rotatable filter wheel configure to move each of the filters into and out of the excitation beam path.

In alternative embodiment 65, the instrument of any of embodiments 1, [00125], [00127], [00127], [00128], [00129], or [00130] is provided, wherein the excitation source comprises a plurality of individual excitation sources.

In alternative embodiment 66, the instrument of embodiment [00187] is provided, wherein the plurality of individual excitation sources form a two dimensional array of individual excitation sources.

In alternative embodiment 67, the instrument of any of embodiments 1, [00125], [00127], [00127], [00128], [00129], or [00130] is provided, wherein the optical sensor comprises an array sensor.

In alternative embodiment 68, the instrument of embodiment [00189] is provided, wherein the array sensor comprises at least one of a complementary metal-oxide-semiconductor sensor or a charge-coupled device sensor.

In alternative embodiment 69, the instrument of any of embodiments 1, [00125], [00127], [00127], [00128], [00129], or [00130] is provided, wherein the optical sensor comprises at least two array sensors.

In alternative embodiment 70, the instrument of any of embodiments [00127], [00127], [00128], or [00130] is provided, further comprising a sensor lens configured to direct emissions from at least some of the biological sample onto the optical sensor.

In alternative embodiment 71, the instrument of any of embodiments [00125], [00127], [00127], [00128], [00129], or [00130] is provided, wherein the optical sensor is a complementary metal-oxide-semiconductor sensor.

In alternative embodiment 72, the instrument of embodiment [00127] is provided, wherein the blocking structure is disposed to cooperate with the sensor aperture during use such that none of the reflected radiation impinges on the sensor lens that does not also reflect off another surface of the instrument.

CALIBRATION WORKFLOW

Advances in the calibration of biological analysis instruments advantageously allow for reduced operator error, reduced operator input, and reduced time necessary to calibrate a biological analysis instrument, and its various components, for proper and efficient installation.

As such, according to various embodiments of the present teachings can incorporate expert knowledge into an automated calibration and validation system providing pass/fail status and troubleshooting feedback when a failure is identified. If an instrument should fail the calibration process, then a service engineer can be called. The present teachings can minimize the cost of, and time required for, the installation and calibration procedures.

Overall Calibration Workflow

Biological instruments are often relied on to produce accurate and reliable data for experiments. Regular calibration and maintenance of biological instruments ensures proper and optimal operation of the instrument, which can maximize user productivity, minimize costly repairs by addressing potential problems before they manifest, and increase quality of results.

According to various embodiments of the present teachings, the calibration methods described in this document may be performed separately or in any combination together. Further, the calibration methods described herein may be performed after manufacture for initial calibration or any time after initial installation and use. The calibration methods described herein may be performed weekly, monthly, semi-annually, yearly, or as needed, for example.

According to various embodiments described in the present teachings, calibration methods such as Region-Of-Interest (ROI) calibration, background calibration, uniformity calibration, pure dye calibration, instrument normalization are used to determine the location and intensity of the fluorescent signals in each read, the dye associated with each fluorescent signal, and the significance of the signal. Further, according to various embodiments, auto-dye correction, auto-background calibration, and plate detection may be performed to further refine detection and dye readings, and determine errors. Instrument validation of proper performance may also be automatically performed by the system using RNase P validation.

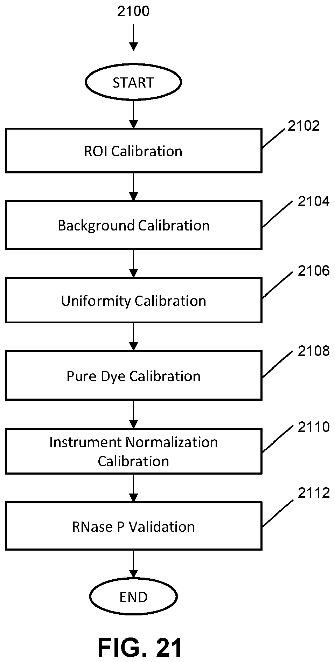

FIG. 21 illustrates an exemplary calibration workflow 2100 that may be performed on an instrument according to various embodiments described herein. It should be recognized that calibration workflow 2100 is an example and that the calibration methods described herein may be performed separately, or as a subset, in any combination and order.

In step 2102, an ROI calibration is performed. Generally ROI calibration will produce information defining the positions of wells in the detector's field of view. The present teachings can automate the ROI calibration through minimization or elimination of user interaction. Various embodiments can automate the process by providing methods and systems that determine the optimal exposure time per filter using histogram analysis and a binary search pattern. The ROI calibration, according to various embodiments described herein, identify wells in an image more accurately and with fewer errors than previous methods. ROI calibration methods and systems, according to various embodiments, are further described below.

In step 2104, a background calibration is performed. Often, a detector will read some amount of signal even in the absence of a sample emitting detectable signal. Accounting for this background signal can be important as the background signal can be subtracted from a sample signal reading in order to get a more accurate measurement of sample signal. Background calibration can be performed using a water plate to determine the instrument background signal for every filter/well combination. The step may be automated to minimize or eliminate user interaction. Automation can be provided that will test if the correct plate has been used for background calibration. For example, step 2104 can look at the signal level and eliminate the possibility of using an incorrect test plate such as the strong signal emitting test plate used in the ROI calibration. If the signal level far exceeds the expected level of the background, the user can be alerted to insert the proper test plate. Also this stage can test for contamination of one or more wells in the test plate by checking for wide divergence of signal levels and if so found, trigger a warning indicating the possible existence of dirty or contaminated wells. Contaminated wells can lead to an improper background signal level being subtracted from the sample signal level.

In step 2106, a uniformity calibration is performed. In some cases, variations in plate geometry (warping, thickness) can cause intensity readings to vary across a plate despite the presence of equal amounts of fluorescent dye in each well. Uniformity calibrations can calibrate the instrument using a multi-dye plate so that intensity variations due to plate variations can be corrected for. Step 2106 may be automated and reduce or eliminate user interaction. Parts of this automation can include detection of the use of the wrong calibration plate and detection and adjustments for empty or contaminated wells in the calibration plate.

In step 2108, a pure dye calibration is performed. Calibrating fluorescent dyes used in a qPCR instrument allows the instrument software to use the calibration data collected from dye standards to characterize and distinguish the individual contribution of each dye in the total fluorescence collected by the instrument. According to various embodiments of the present teachings, after a sample run, the instrument software receives data in the form of a raw spectra signal for each reading. The software determines the contribution of each of the fluorescent dyes used in each reaction site by comparing the raw spectra, contributed by each dye, to the pure spectra calibration data. When a user saves an experiment after analysis, the instrument software stores the pure spectra along with the collected fluorescence data for that experiment, as well as the contribution of each fluorescence dye per well. The method is further described below. Using the pure dye calibration, according to various embodiments of the present teachings, fewer pure calibration plates may be used, saving a user cost, and eliminating sources of errors in the calibration.

In step 2110, an instrument normalization calibration is performed. One difficulty commonly faced is the inability of researchers to easily compare results of experiments run on multiple instruments. Physical variations in the parameters of components such as light sources, optical elements and fluorescence detectors, for example, can result in variation in the results of analyses on what may be identical biological samples. There is, therefore, a continuing need for methods and apparatus to aid in minimizing the variations in the components.

In qPCR, amplification curves are often determined by normalizing the signal of a reporter dye to a passive reference dye in the same solution. This normalization can be reported as normalized fluorescence values labeled or "Rn". Passive reference normalization enables consistent Rn values even if the overall signal level is affected by liquid volume, or overall illumination intensity. Passive reference normalization, however, cannot work properly if the ratio in signal between the reporter dye and reference dye varies, such as from instrument-to-instrument differences in the spectrum of the illumination. According to various embodiments described herein, instrument normalization calibration includes reading fluorescence from the dye mixture to get a "normalization factor" to adjust Rn values requires additional expense.

In step 2112, an RNase P validation is performed. Performing a validation test checks to see if an instrument is functioning properly. For example, RNase P validation determines if an instrument can accurately distinguish between two different quantities of sample. Previously, an RNase P validation was manually performed using a standard curve, with the user doing the statistical calculations to validate the instrument. According to various embodiments described in the present teachings, the RNase P validation may be performed automatically by the system without using a standard curve. Various embodiments of an RNase P validation are further described below.

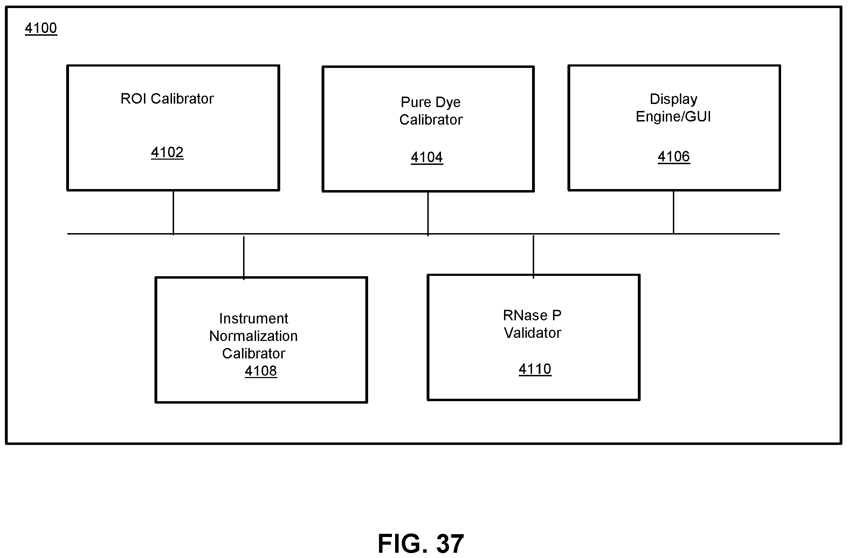

FIG. 37 illustrates a system 4100 for calibration of an instrument according to various embodiments described herein. System 4100 includes ROI calibrator 4102, pure dye calibrator 4104, instrument normalization calibrator 4108, RNase P validator 4110, and display engine/GUI 4106. ROI calibrator 4102 is configured to determine reaction site positions in an image. Pure dye calibrator 4104 is configured to determine the contribution of a fluorescent dye used in each reaction site by comparing a raw spectrum of the fluorescent dye to a pure spectrum calibration data of the fluorescent dye. Instrument normalization calibrator 4108 is configured to determine a filter normalization factor. RNase P validator 4110 is configured to validate the instrument is capable of distinguishing between two different quantities of sample. Display engine 4106 is configured to display calibration results.

The present teachings are described with reference to Real-Time Polymerase Chain Reaction (RT-PCR) instruments. In particular, an embodiment of the present teachings is implemented for RT-PCR instruments employing optical imaging of well plates. Such instruments can be capable of simultaneously measuring signals from a plurality of samples or spots for analytical purposes and often require calibration, including but not limited to processes involving: identifying ROI (Regions of Interest), determining background signal, uniformity and pure dye spectral calibration for multicomponent analysis. Calibration may also involve a RT-PCR validation reaction using a known sample plate with an expected outcome. One skilled in the art will appreciate that while the present teachings have been described with examples pertaining to RT-PCR instruments, their principles are widely applicable to other forms of laboratory instrumentation that may require calibration and verification in order to ensure accuracy and/or optimality of results.

Region of Interest (ROI) Calibration

As presented above, the present teachings are described with reference to Real-Time Polymerase Chain Reaction (RT-PCR) instruments. In particular, an embodiment of the present teachings is implemented for RT-PCR instruments employing optical imaging of well plates. Such instruments can be capable of simultaneously measuring signals from a plurality of samples or spots for analytical purposes and often require calibration. An example of a process that can require calibration is the identification of ROIs or Regions of Interest.

Generally ROI calibration can be performed using a plate with strong emissions in each cell corresponding to all filters. This can be useful since the ROIs may not be identical for each filter. Differences in the ROIs between filters can be caused by slight angular differences in the filters and other filter spectral characteristics. Thus, various embodiments perform per filter/per well (PFPR)-ROI calibration. These PFPR-ROI calibrations are useful to determine locations of the wells in the 96 well-plate for each filter. ROI calibration can be performed using a method such as the Adaptive Mask Making teachings as described in U.S. Pat. No. 6,518,068 BI.

The present teachings can automate the ROI calibration through minimization or elimination of user interaction. Various embodiments can automate the process by providing for software that determine the optimal exposure time per filter using histogram analysis and a binary search pattern. The exposure time is the amount of time required to capture an image of the plate. Again, this value can vary according to a filter's spectral characteristics. Generally ROI calibration will produce information defining the positions of wells in the detector's field of view. This information can be stored as mask files at 2304 with either a global mask or multiple masks corresponding to different filters.

Calibration processes such as what is described above frequently use row and column projections and intensity profiles. This can result in ROI determinations being susceptible to artifacts and saturation inside the wells, grid rotation, variation of magnification factors and optical radial distortion. It can therefore be advantageous to have a more robust determination of ROIs to minimize such susceptibilities and remove distortions and other unwanted background noise in the detected emission data.

Background noise may refer to inherent system noise as well as other undesired signals. For example, some background noise in the data may be due to physical sources on the substrate, such as dust particles or scratches, for example. Another example of a physical source that may provide background noise is a holder or case holding or enclosing the sample. Other background noise in the data may be due to natural radiation from the surfaces in the instrument, such as reflection and natural fluorescence. Other background noise may also be a result from the optical system detecting the emission data or the light source, for example.

The biological instrument may be detecting several hundred to several thousand samples, all of which may be a very small volume, such as less than one nanoliter. As such, other background noise removal methods may be used alone or in combination with the calibration methods described in this document according to various embodiments to be able to determine and analyze the emission data from the sample volumes. In some embodiments, the location of samples volumes may be more accurately determined within the substrate to perform a more accurate analysis. For example, in digital PCR analyses, being able to more accurately distinguish reactions in sample volumes versus non-reactions may produce more accurate results. Even further, according to various embodiments described herein, empty wells or through-holes may be distinguished from sample volumes in wells or throughholes that did not react, which may also be distinguished from sample volumes in wells or throughholes that did react.

According to various embodiments described herein, background noise removal may include image data analysis and processing. The method may include analyzing intensity values of the image data to interpolate the background noise that may be removed from the image of the substrate. In this way, locations of the regions-of-interest within the image may also be determined. The background noise removal may also include interpolating data from areas of the image known to include regions-of-interest. After determining the background noise over the image, the background noise may be subtracted from the image data.

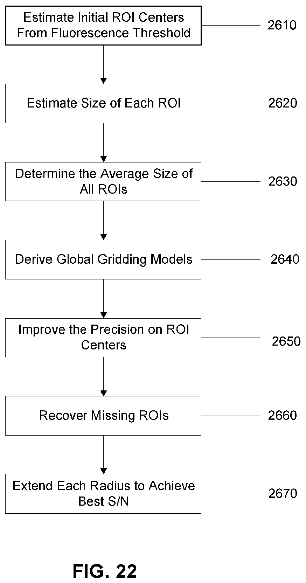

FIG. 22 depicts an exemplary in silico method 2600 according to one embodiment of the present invention. In silico method 2600 includes a plurality of set workflow subroutines in a computer readable format that can include subroutines for a biotechnology process. FIG. 22 is merely an exemplary method and the skilled artisan, in light of this disclosure, will realize that the actual number of subroutines can vary from at least about 2 subroutines to many (e.g. 2-10, 2-20, 2-30, 2-n (where n may be any number of subroutines from 3-100, 3-1000 and so on)). Each set subroutine 310 - 370 can include a single step or task, or optionally can include more than 1 step or task, also in a computer readable format, and each step can further include additional optional customizable steps or tasks. Each of the optional/customizable steps or tasks can have one or more optional parameters (options) that can be viewed, reviewed, set or customized by a user. In some embodiments, an in silico method of the invention includes selection by a user of at least one parameter each for each optional/customizable step of the biotechnological process using a graphical user interface (GUI) to select the at least one parameter for each optional/customizable step. In certain embodiments, every step and every parameter of the subroutines of a workflow are available to a user to view, and optionally edit. Bioinformatics programs typically hide some of these parameters and/or steps from users, which causes user frustration and inefficiency especially when the result of an in silico designed experiment is not the expected result for a user.

An exemplary in silico method of the disclosure illustrated generally in FIG. 22 can be carried out (performed) by generating at least one method file in a computer system (such as shown in FIG. 2), the method file comprising computer readable instructions for a plurality subroutines (10, 20, 30..) of customizable steps (A, B, C) each of which may have one or more parameters that may be viewed, selected, changed or inputted; and performing the biotechnological process in silico comprising executing the at least one method file comprising computer readable instructions by the computer system to obtain at least one biotechnology product.

In some embodiments, at least one customizable/optional parameter is selected from a default parameter, wherein the default parameter is stored in a component of the computer system (such as storage, database etc.).

Referring again to FIG. 22, the first step in calculating ROI locations is to estimate the initial ROI centers from the fluorescence threshold in step 2610. A sample plate configured to contain a plurality of biological samples is provided and inserted into an analytical instrument capable of analyzing biological samples through the process of PCR. Each biological sample is contained in a sample well and can be excited by a light source and in response to the excitation can fluoresce at a predetermined wavelength which can be detected by a fluorescence detector. As presented above with regards to FIG. 7, light source 1402 can be a laser, LED or other type of excitation source capable of emitting a spectrum that interacts with spectral species to be detected by computer system 200. Additionally, biological samples can include spectrally distinguishable dyes such as one or more of FAM, SYBR Green, VIC, JOE, TAMRA, NED, CY-3, Texas Red, CY-5, ROX (passive reference) or any other fluorochromes that emit a signal capable of being detected.

Prior to exciting the biological samples input parameters and algorithm parameters are set to provide a starting point for the ROI determination. Input parameters can include well size, well center-to-center distance, optical pixels per millimeter and plate layout. The plate layout can include the total number of wells and the configuration of the sample wells. A frequently used configuration can be a rectangular array comprising a plurality of rows and a plurality of columns. However one skilled in the art will understand that the configuration can be any geometry suitable for the instrument being used. Further, the total number of wells can vary. One skilled in the art will be familiar with configurations totaling from 1 well to thousands of wells in a single sample plate or sample containment structure. The ROI finding algorithm parameters can set acceptable ranges for well size, well center-to-center distance and minimum circularity. Circularity is a calculated value and can be a ratio of the perimeter to the area.

Once the input parameters and the algorithm parameters have been determined, the plurality of samples are excited with energy from an appropriate light source, and images are collected of the fluorescence emitted from each sample well in the sample plate. The fluorescence images of the sample plate are further analyzed to select ROI candidates based on the input parameters and the algorithm parameters. The ROI candidates that satisfy the parameters are saved for further analysis and the size and circularity of each well is determined in step 2620. ROI candidates that do not satisfy the parameters can be discarded along with any locations that did not fluoresce. The retained ROI candidates are further evaluated to determine the distance between ROIs based on the well-to-well spacing parameter and the allowed range parameter for the well-to-well spacing. ROIs that have centers that are in close proximity to each other based on the well-to-well parameters can be considered to be the same sample well, and the one with the best circularity is selected as the ROI for that well. Once all the ROI candidates have been determined, the average well size is calculated, the average is assigned to each sample well ROI in step 2630 and the initial estimated ROIs are saved.

The expected well locations are arranged in a grid pattern determined based on the plate layout parameter. This parameter can include the number of wells, number of columns and number of rows where each well has an expected set of XY grid co-ordinates based on the plate layout parameter. Further analysis can now be initiated on the initial estimated ROIs to better define the locations of each initial ROI and can be referred to as global gridding. The first step in global gridding is to analyze the centers of the initial estimated ROIs to find adjacent ROIs. This can be determined by comparing the center-to-center distance between ROIs to the grid co-ordinates based on the plate layout. The XY grid co-ordinates can then be determined for each of the initial estimated ROIs based on the spatial relationship between ROIs.

In order to improve the precision of the ROI locations it would be advantageous to relate the center-to-center ROI co-ordinates to the grid co-ordinates of the plate layout. This can be accomplished by determining and applying mapping functions. Mapping functions are a pair of 2-dimensional quadratic polynomial functions. These functions are calculated to map X (or Y) grid locations to the ROI center locations in the X (or Y) direction. Once the mapping functions have been determined, they can be applied to the expected grid co-ordinates to provide two benefits. First the precision of the ROI center locations can be improved, and second it can be possible to recover ROIs that were missing during the initial ROI finding.

Further adjustment of ROIs can provide additional benefits to optical performance. The inventors discovered that there was a relationship between ROI size and the signal-to-noise ratio (SNR) of the optical system. One skilled in the art would know that there are several equations to calculate SNR of electrical and optical systems. SNR can be characterized with Equation 1 below, for example:

$$SNR = \frac{S_{dye\ plate} - S_{BG}}{\sqrt{\dfrac{S_{dye} + S_{BG} - 2N \times offset}{G} + 2N\sigma_{R,y}^2}}$$

where:

SNR = Signal to Noise Ratio
Sdye plate = the sum of all pixel intensities within ROIs from the dye images
$S_{BG}$ = the sum of all pixel intensities within ROIs from background images
Sdye = the sum of all pixel intensities within ROIs from the dye images
N = the number of pixels within an ROI
offset = the camera offset
G = the camera gain
$\delta 2R,y$ = the read noise

An experiment was conducted using an optical system that included six pairs of filters. Each pair of filters included an excitation filter (Xn) and an emission filter (Mn). Each filter was sensitive to a narrow band of wavelengths that correspond to the excitation frequency and emission frequency of dye configured to be compatible with the PCR process. In addition ROIs were optimized according to the teachings presented in this document. In order to study the effect of ROI size on signal-to-noise, fluorescence was detected from a 96 well sample plate using 6 pairs of filters. The radius of each ROI was extended incrementally by 1 pixel. Equation 1 was used to calculate the SNR for each of 6 filter pairs and each pixel increment. The results of the experiment are shown below in Table 1:

TABLE 1

| SNR | X1M1 | X2M2 | X3M3 | X4M4 | X5M5 | X6M6 |
|---|---|---|---|---|---|---|
| ΔR=0 | 1709.5 | 2502.7 | 1840.3 | 1613.8 | 1632.4 | 475.5 |
| ΔR=1 | 1808.2 | 2642.0 | 1942.7 | 1706.3 | 1709.2 | **496.8** |
| ΔR=2 | **1826.6** | 2677.8 | **1964.2** | **1722.7** | **1718.8** | 491.2 |

(continued)

| SNR | X1M1 | X2M2 | X3M3 | X4M4 | X5M5 | X6M6 |
|---|---|---|---|---|---|---|
| ∆R=3 | 1818.7 | **2678.7** | 1958.4 | 1714.4 | 1708.2 | 479.0 |
| ∆R=4 | 1802.5 | 2667.3 | 1943.1 | 1697.6 | 1690.8 | 464.7 |

The bold entries identify the highest SNR for each of the 6 filter pairs, and a 2 pixel radius extension provides an overall improvement in SNR of approximately 6% across the 6 filter pairs.

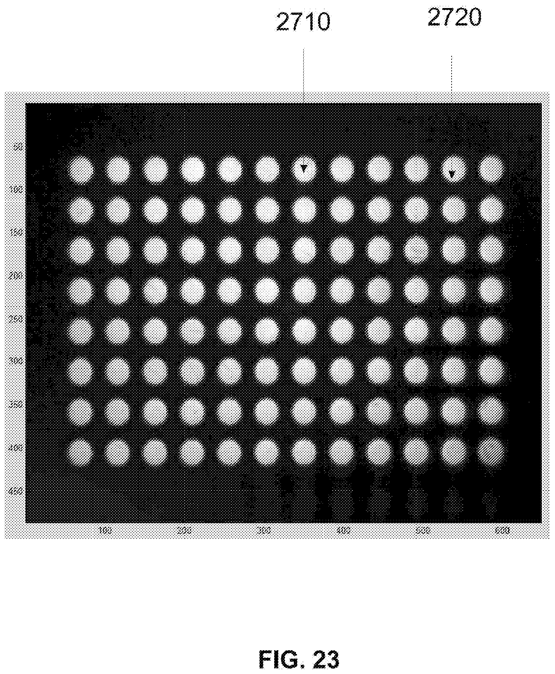

FIG. 23 shows an image of a sample plate with 96 wells 2710. Each of the wells 2710 produced a fluorescent image. After applying the teachings of this document ROIs were optimized and the blue circles identify the ROI for each well position.

Pure Dye Calibration

As described above, there is an increasing need to simplify the installation and setup of biological analysis systems so that operators can more quickly and efficiently use biological analysis systems for their intended purpose. This need is evident in, for example, calibrating a biological analysis instrument and associated components. One exemplary calibration is the calibrating of fluorescent dyes used for fluorescence detection in biological analysis systems such as, for example, qPCR systems.

Calibrating fluorescent dyes used in a qPCR instrument allows the instrument software to use the calibration data collected from dye standards to characterize and distinguish the individual contribution of each dye in the total fluorescence collected by the instrument. After a sample run, the instrument software receives data in the form of a raw spectra signal for each reading. The software determines the contribution of each of the fluorescent dyes used in each reaction site by comparing the raw spectra, contributed by each dye, to the pure spectra calibration data. When a user saves an experiment after analysis, the instrument software stores the pure spectra along with the collected fluorescence data for that experiment, as well as the contribution of each fluorescence dye per well.

The product of a dye calibration in a qPCR instrument, for example, is a collection of spectral profiles that represent the fluorescence signature of each dye standard for each reaction site. Each profile consists of a set of spectra that correspond to the fluorescence collected from reaction sites, such as wells, of a sample holder such as, for example, a calibration plate or array card. Following the calibration of each dye, the instrument software "extracts" a spectral profile for each dye at each reaction site. The software plots the resulting data for each profile in a graph of fluorescence versus filter. When the software extracts the dye calibration data, it evaluates the fluorescence signal generated by each well in terms of the collective spectra for the entire calibration plate or array card. Dye spectra are generally acceptable if they peak within the same filter as their group, but diverge slightly at other wavelengths.

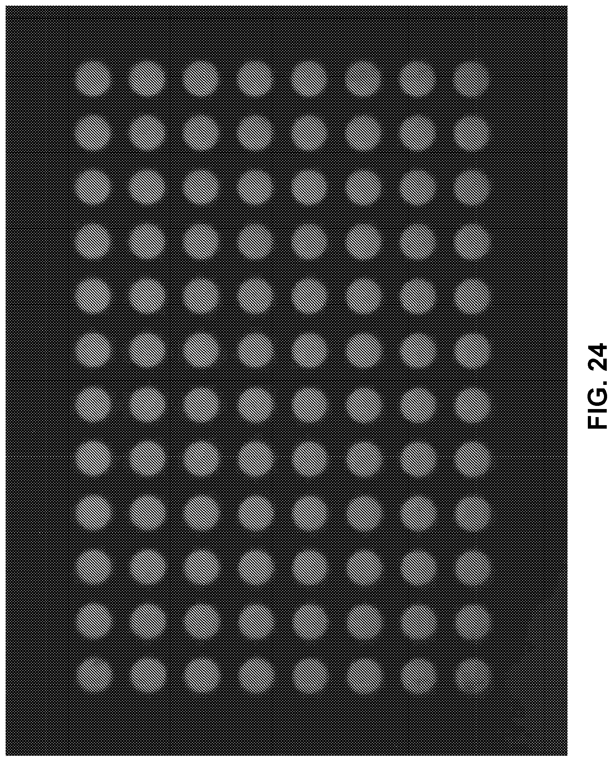

When running dye calibration on a sample holder, such as a calibration plate, the reaction sites (e.g., wells) generally contain identical concentrations of dye to allow generation of a pure spectra value at each well of the plate. FIG. 24 displays an image of a calibration plate with a single dye (in this case, FAM dye), occupying each well of a 96-well calibration plate. This allows for the comparison of fluorescence signal generated by each well in a run to a pure spectra read for that well. By using a single dye for each well of a calibration plate, the resulting signals for the wells should be similar. Variations in spectral position and peak position can be caused, for example, by minor differences in the optical properties and excitation energy between the individual wells. Taking these variations into account in dye calibration theoretically leads to a more accurate dye calibration.

However, the use of a single dye per calibration plate could be time intensive and complicated, particularly when calibrating numerous dyes. Non-limiting examples of fluorescent dyes include FAM, VIC, ROX, SYBR, MP, ABY, JUN, NED, TAMRA and CY5. Therefore, a need exists to simplify the dye calibration process and reduce the time required for calibration while maintaining the same quality of results of the dye calibration.

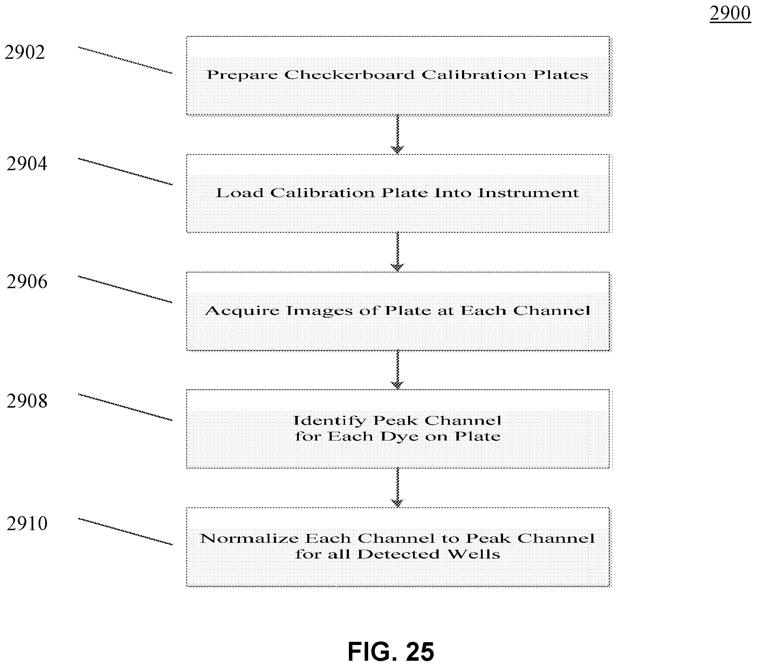

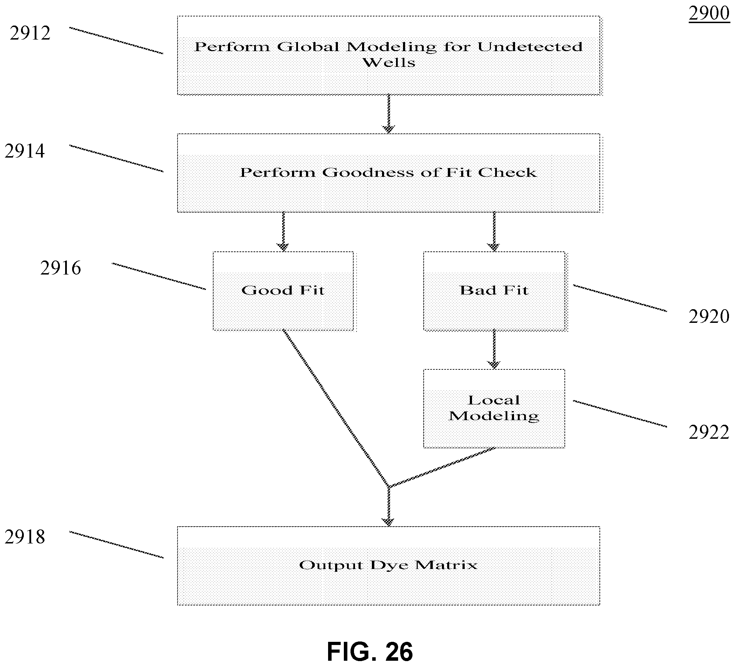

FIGS. 25 and 26 illustrate a flowchart depicting an exemplary method 900 of calibrating fluorescent dye(s) according to embodiments described herein. The steps of method 2900 may be implemented by a processor 204, as shown in FIG. 2. Furthermore, instructions for executing the method by processor 204 may be stored in memory 206.

With reference to FIG. 25, in step 2902, calibration plates are prepared by loading dyes into reaction sites of a substrate for processing. The substrate in this case is a 96- well plate, though different substrates may be used including, for example, a 384-well plate. In various embodiments, the substrate may be a glass or plastic slide with a plurality of sample regions. Some examples of a substrate may include, but are not limited to, a multi-well plate, such as a standard microtiter 96-well plate, a 384-well plate, or a microcard, a substantially planar support, such as a glass or plastic slide, or any other type of array or microarray. The reaction sites in various embodiments of a substrate may include wells, depressions, indentations, ridges, and combinations thereof, patterned in regular or irregular arrays formed on the surface of the substrate. Heretofore, reference to wells or plates are just for exemplary purposes only and not in any way to limit the type of reaction site or sample holder useable herein.

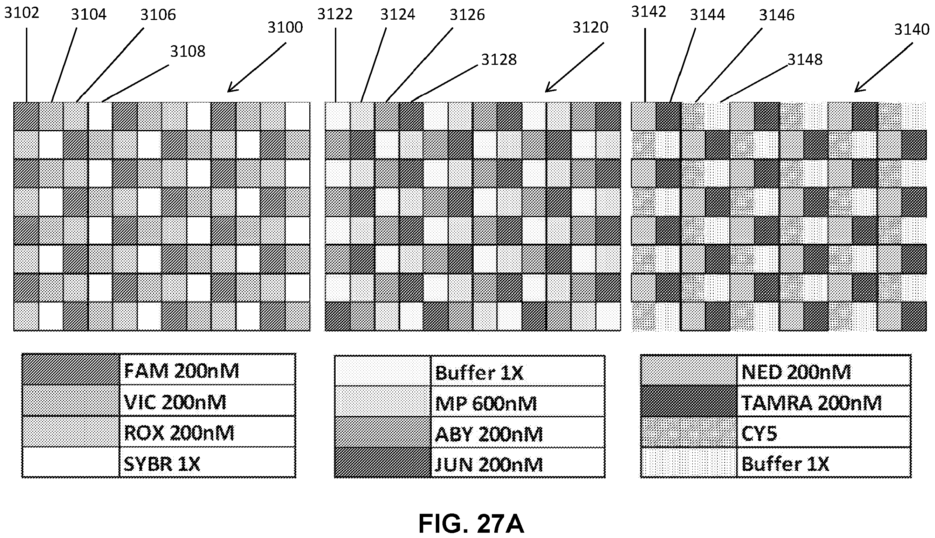

The calibration plates may be prepared in a checkerboard pattern as illustrated in FIG. 27A. As illustrated in calibration plates 3100, 3120 and 3140, the plates themselves may be of a 96-well format, though the number of wells on the calibration plate can be varied as needed depending on, for example, the number of dyes requiring calibration, the sample block 114 (see FIG. 1) format accepting the calibration plate, or the capabilities of the instrument (PCR instrument 100 for example) to image plates of different well densities.

The checkerboard pattern of dye distribution allows multiple dyes to be calibrated per calibration plate. As opposed to calibrating one dye per calibration plate, the checkerboard pattern advantageously allows a user to use fewer plates to calibrate a dye set, thus decreasing time and process steps needed for dye calibration.

In the embodiment illustrated in FIG. 27A, three plates are used to calibrate ten separate dyes. Each calibration plate 3100/3120/3140 is configured to accommodate four different dyes in a repeating pattern of alternating dyes along wells in each row of the plate such that each well presents a specific dye in the repeating pattern (dye presented well). For example, plate 3100 accommodates FAM, VIC, ROX and SYBR dyes in alternating wells exemplified by wells 3102 (FAM), 3104 (VIC), 3106 (ROX) and 3108 (SYBR); plate 3120 accommodates a buffer, MP dye, ABY dye and JUN dye in alternating wells exemplified by wells 3122 (buffer), 3124 (MP), 3126 (ABY) and 3128 (JUN); and plate 3140 accommodates NED dye, TAMRA dye, CY5 dye and a buffer in alternating wells exemplified by wells 3142 (NED), 3144 (TAMRA), 3146 (CY5) and 3148 (buffer). In this embodiment, since only ten dyes are being calibrated, buffers are used in plates 3120 and 3140 as filler for wells not accommodating a dye to be calibrated.

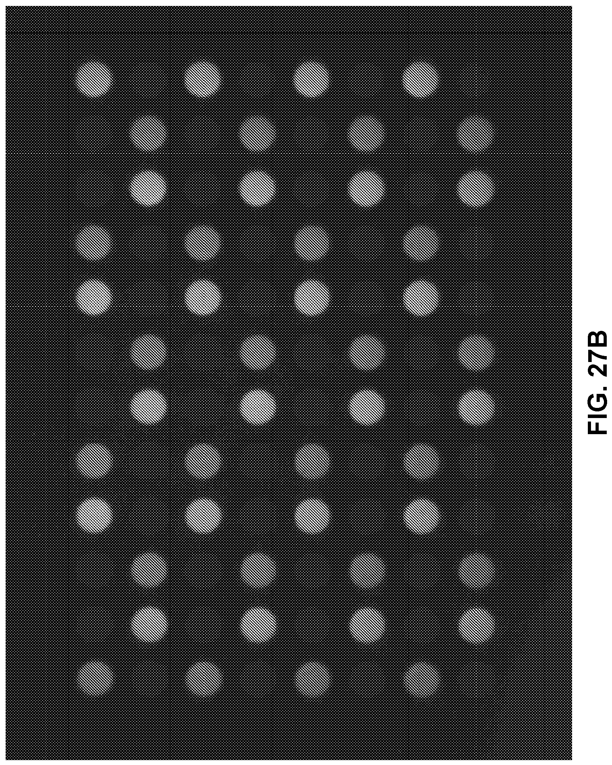

It should be appreciated that the embodiment in FIGS. 27A and 27B is an example only, and that the number of total dyes calibrated, the number of dyes per plate, and the number of plates, can all vary as needed based, for example, on a user's calibration needs, the number of wells on the plate, and capacity of the instrument handling the calibration. For example, if 12 dyes were being calibrated in the embodiment illustrated in FIG. 27A, a buffer would not be needed in plates 3120 and 3140, as four dyes could be calibrated in each of the three calibration plates 3100/3120/3140 for a total of 12 dyes.

Moreover, the number of dyes per plate can be two or more, with the maximum number of dyes per plate based on, for example, the number of wells on the calibration plate, the capability of the instrument used to properly model a full plate (see below for further explanation), and the capability of the imaging system to obtain usable fluorescence data from the plate chosen. For example, rather than using a 96-well plate as illustrated in FIG. 27A, one may have a sufficiently robust instrument and associated imaging system to be able to use a 384-well calibration plate. With the additional well density provided, one could calibrate more dyes per plate, for example 16 dyes per plate, and still get the same number of data points (i.e., dye presented wells) per dye (e.g., 24) needed to get a sufficient global model (discussed in more detail below). For example, with a 384-well plate, 10 dyes can be calibrated using two plates and five dyes per plate.

Even the type of sample holder and type of reaction site may affect the number of dyes possible. As stated above, other types of sample holders and reaction sites may be used for calibration.

Returning to FIG. 25, in step 2904, prepared checkerboard calibration plates can be loaded into the instrument. The number of plates loadable into an instrument at one time depends on the capabilities and capacity of the instrument used. For example, a standard qPCR thermal cycler with a 96-well block will only accept on calibration plate at a time. However, multi-block thermal cyclers may offer multiple blocks that can each accept a calibration plate. Moreover, if a calibration plate is not used, depending on the format of the sample holder used (e.g., a microarray or microchip array), multiple sample holders may be received in a single instrument using, for example, a loading assembly that fits into the instrument.

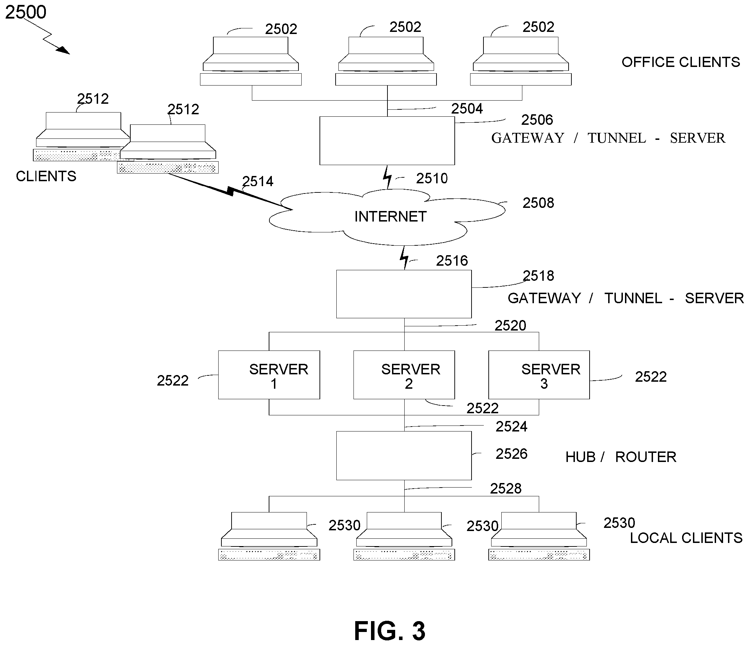

In step 2906 of FIG. 25, the instrument, using its associated optical imaging system (see, for example, FIG. 3), acquires images of the loaded calibration plate, or plates, in series or parallel. The acquired images and associated data can be stored, for example, on memory 206 or storage device 210 of computing system 200 in FIG. 2. The optical imaging system can acquire images of each plate at each optical channel. The number of channels depends on the number of excitation and emission filters provided in the imaging system. For example, for an optical imaging system having 6 excitation filters (X filters) and 6 emission filters (M filters), the total number of channels is 21, represented by the following filter combinations: X1M1, X1M2, X1M3, X1M4, X1M5, X1M6, X2M2, X2M3, X2M4, X2M5, X2M6, X3M3, X3M4, X3M5, X3M6, X4M4, X4M5, X4M6, X5M5, X5M6, and X6M6. The number of images or exposures acquired at each channel can vary. For example, the imaging system can acquire two images or exposures per channel. The number of images or exposures taken depends on user needs, as taking fewer images or exposures per channel may decrease the time needed to acquire images or exposures, while taking more images or exposures per channel provides greater likelihood of quality data.

In step 2908 of FIG. 25, the instrument, using the data gathered from the images or exposures acquired by the optical imaging system (see, for example, FIG. 7), identifies the peak channel for each dye on the calibration plate. This peak channel for each reaction site is the channel where the specific dye analyzed shows the greatest fluorescence for that reaction site. The peak channel identification can occur when, for example, 95% or more reaction sites are dye occupied, in this case allowing no more than 5% outlier reaction sites during calibration. The percentage of allowable outliers can vary. The outlier reaction sites can then be discarded from future calculation and analysis. Outliers can occur, for example, when the wrong dyes are loaded, the dyes are loaded in the incorrect configuration, there is improper loading of dyes, or optical components become dirty (e.g., dust particles). The peak channel for each dye on the calibration plate can be identified, for example, by processor 204, of computing system 200, utilizing data stored on memory 206. The identification results can be stored, for example, on memory 206 or storage device 210 of computing system 200.

Alternatively, the collected fluorescence data gathered from the images or exposures acquired by the optical imaging system for each filter combination on each reaction site can be corrected by background and uniformity correction before peak channel identification, using background component and uniformity factors determined using background and uniformity calibrations methods known in the art.

In step 2910 of FIG. 25, the instrument, using the data gathered from the images or exposures acquired by the optical imaging system (see, for example, FIG. 7), normalizes each channel to the identified peak channel of step 2908 for all the dye presented wells. Each channel can be normalized to the identified peak channel, for example, by processor 204, of computing system 200, utilizing data stored on memory 206. The results of the normalization can be stored, for example, on memory 206 or storage device 210 of computing system 200.

All dye presented wells are given a baseline quant value from which to normalize from. Generally, the greater the quant value, the greater the detected fluorescence. Therefore, the identified peak channel for a given dye would have the largest quant value for that dye in the dye presented wells, excluding peak channel outliers. Regardless of the quant value in that peak channel, to normalize, that quant value at that channel is reset to a value of one. The remaining quant values for that same dye at the other channels are then adjusted according to the reset value of one for the peak channel. For example, if for dye X, the peak channel A had a quant value of 100 in the wells, and other channel B had a quant value of 40 in the wells, upon normalization, peak channel A gets set to 1.0 and channel B gets set to 0.40. This normalized value can also be referred to as a calibration factor, with the calibration factor for the peak channel being set to 1.0 as discussed above.

In the embodiment illustrated in FIGS. 27A and B, where four dyes are equally dispersed among the wells of a 96-well plate, the number of dye presented wells per dye would be 24. The number of dye presented wells can vary for reasons discussed previously such as, for example, the number of reaction sites (e.g., wells) on the sample holder (e.g., calibration plate), the number of dyes per dispersed on the sample holder. For example, on a 96-well plate, if three dyes are dispersed, the number of dye presented wells would be 32 per dye. If there are six dyes dispersed on the 96-well plate, there would be 16 dye presented wells per dye.

With reference now to FIG. 26, in step 2912, the instrument performs global modeling for all wells per dye. In order to calibrate a dye for all wells of a sample holder format, the instrument can use the data from the dye presented wells for a specific dye to model for all wells, including the ones without a specific dye. The global modeling can be performed, for example, by processor 2404, of computing system 2400, by using the data from the dye presented wells for a specific dye to model for all wells. The resulting model can be stored, for example, on memory 2406 or storage device 2410. Referring to FIG. 27A, for the FAM dye present in 24 wells 3102 of plate 3100, the other 312 wells on that plate would be FAM dye unpresented. The same 24 presented /72 presented distribution would apply to each dye in FIG. 27A. The number of dye unpresented wells depends on the number of dye presented wells, which, as discussed above, can depend for various reasons. Regardless, the sum of dye presented and dye unpresented wells for a given plate equals the number of wells on that plate. FIG. 27B is an image of a 4 dye checkerboard 96-well calibration plate with FAM, VIC, ROX and SYBR dyes in the same configuration as illustrated by plate 3100 in FIG. 27A.

In an alternative embodiment, the instrument performs global modeling for all channels or those channels that have a normalized value, for example, greater than 0.01, or 1% of the identified peak channel. For those channels below this threshold, the instrument would perform a local modeling (see step 2922 of FIG. 26) instead of performing global modeling. Global modeling may become unnecessary at such low levels at certain channels such that detected fluorescence is primarily a result of, for example, noise or other disturbance, rather than contribution of the actual dye being calibrated.

A global modeling algorithm can function in a dye calibration to derive a model of dye calibration factors for each filter channel for each dye based on the measured dye calibration factors of the specific dye presented wells. For example, if 24 wells are presented on the 96-well checkerboard plate for a specific dye, global modeling utilizes the dye calibration factors of those 24 wells to derive calibration factors for all the wells including the other dye unpresented detected 72 wells, and thus produce a model for the whole plate per channel, per dye.

The two-dimensional (2D) quadratic polynomial function is an example of a function that can be applied as a global model for dye calibration factors. Other global modeling functions are known and can be used herein. A non-linear least square solver can be used to derive the 2D quadratic polynomial function from the measured dye calibration factors on the specific dye presented wells by minimizing the modeling residuals (the difference between the values calculated from the model and the measured dye calibration factors). Levenberg-Marquardt Trust region algorithm can be used as the optimization algorithm in this solver. While many other optimization algorithms are useable herein, one other example is the Dogleg method, whose key idea is to use both Gauss-Newton and Cauthy methods to calculate the optimization step to optimize the non-linear objective. This approach approximates the objective function using a model function (often a quadratic) over a subset of the search space known as the trust region. If the model function succeeds in minimizing the true objective function, the trust region is expanded. Conversely, if the approximation is poor, then the region is contracted and the model function is applied again. A loss function, for example, may also be used to reduce the influence of the high residuals (greatest difference between calculated and measured calibration factors). These high residuals usually constitute outliers on the optimization.

In step 2914 of FIG. 26, after all wells are modeled for a given dye or dyes, the instrument performs a goodness of fit (GOF) check. This can ensure that the global modeling step is sufficiently reliable. A GOF check can be performed, for example, by processor 204 of computing system 200, with the results stored, for example, on memory 206 or storage device 210. Measures of goodness of fit typically summarize the discrepancy between observed values and the values expected under the model in question. GOF can be determined in many ways including, for example, coefficient of determination R-squared and root-mean-square error (RMSE) values. R-squared, for example, is a statistic that will give some information about the goodness of fit of a model. In regression, the R-squared coefficient of determination is a statistical measure of how well the regression line approximates the real data points. An R-squared of 1 indicates that the regression line perfectly fits the data. RMSE is the square root of the mean square of the differences or residuals between observed values and the values expected under the model in question. RMSE is a good measure of the predication accuracy of the model. A RMSE of 0 indicates the values expected under the model are exactly matched to the observed values.

In step 2916 of FIG. 26, if there is a good fit, then the instrument outputs a dye matrix at step 2918 of FIG. 26. A statistical good fit may occur, in R-squared analysis for example, when R-squared values are, for example, greater than or equal to 0.85, or RMSE values that are, for example, less than or equal to 0.01, such as that illustrated in FIG. 9. The dye matrix can be prepared, for example, by processor 204 of computing system 200, and outputted to display 212.

In step 2920 of FIG. 26, if there is a bad fit, then the instrument performs a local modeling at step 2922 of FIG. 26. This can become necessary, for example, if the calculated $R^2$ value for a GOF check is less than 0.85, for example, and RMSE values are greater than 0.01, for example. The local modeling can be performed, for example, by processor 204, of computing system 200, by using the data from the dye presented wells for a specific dye to model for the remaining dye unpresented wells. The resulting model can be stored, for example, on memory 206 or storage device 210.

A local modeling method can include, for example, using the calibration factors from the surrounding dye presented wells for the same dye on the plate. For example, to determine the calibration factor value in a dye unpresented well for a specific dye, the local model can take the median value of all specific dye presented wells of the same dye that are within a 5x5 local window of surrounding wells or from the whole plate. That median value is determined until a full modeling of the plate is completed. The local modeling output can then replace the global modeling output.

At the conclusion of the local modeling, the dye matrix is sufficient such that the instrument outputs the dye matrix at step 2918 of FIG. 26. This dye matrix serves as a profile of the fluorescence signature of each calibrated dye. After each run, the instrument receives data in the form of a raw spectra signal for each reading. The instrument determines the contribution of the fluorescent dyes used in each reaction by comparing the raw spectra to the pure spectra calibration data of the dye matrix. The instrument uses the calibration data collected from the dye standards (i.e., the dye matrix) to characterize and distinguish the individual contribution of each dye in the total fluorescence collected by the instrument.

Instrument Normalization Calibration

Currently, genomic analysis, including that of the estimated 30,000 human genes is a major focus of basic and applied biochemical and pharmaceutical research. Such analysis may aid in developing diagnostics, medicines, and therapies for a wide variety of disorders. However, the complexity of the human genome and the interrelated functions of genes often make this task difficult. One difficulty commonly faced is the inability of researchers to easily compare results of experiments run on multiple instruments. Physical variations in the parameters of components such as light sources, optical elements and fluorescence detectors, for example, can result in variation in the results of analyses on what may be identical biological samples. There is, therefore, a continuing need for methods and apparatus to aid in minimizing the variations in the components.

In qPCR, amplification curves are often determined by normalizing the signal of a reporter dye to a passive reference dye in the same solution. Examples of reporter dyes can include, but not be limited to FAM, SYBR Green, VIC, JOE, TAMRA, NED CY-3, Texas Red, CY-5. An example of a passive reference can be, but not limited to ROX. This normalization can be reported as normalized fluorescence values labeled or "Rn". Passive reference normalization enables consistent Rn values even if the overall signal level is affected by liquid volume, or overall illumination intensity. Passive reference normalization, however, cannot work properly if the ratio in signal between the reporter dye and reference dye varies, such as from instrument-to-instrument differences in the spectrum of the illumination. In order to adjust for this, normalization solutions can be manufactured to normalize the ratio of reporter to passive reference. An example of such a normalization solution can be a 50:50 mixture of FAM and ROX, which can be referred to as a "FAM/ROX" normalization solution.

This current method of instrument normalization, including reading fluorescence from the dye mixture to get a "normalization factor" to adjust Rn values requires additional expense. Typically, it can require the manufacture of normalization solutions and normalization plates, and the time to run the additional calibrations. Further, this method only works for the dye mixtures you are calibrating with a standard paired filter set. A paired filter set can be a combination of an excitation filter and an emission filter. One skilled in the art will understand that the addition of an additional dye would require a different normalization solution and calibration.

Manufacturing processes for producing the normalization solutions also contribute to variations in the response of the dyes. It has been found that it can be difficult to control dye concentrations due to the lack of an absolute fluorescence standard. In order to minimize these errors and variations it can be advantageous to target the dye ratio of the solution to within +/-15% of the desired mix, or within +/-10% of the desired mix from the manufacturing process. The manufacturing process is typically not controlled well enough to simply mix a 50:50 mixture of the dyes and meet those specifications, so an additional step in the process is necessary to adjust the dye mixture with a fluorimeter.

Acceptable percent variations disclosed above have been determined by studying the relationship between variation in dye mixture and $C_t$s. A $C_t$ is a common abbreviation for a "threshold cycle". Quantitative PCR (qPCR) can provide a method for determining the amount of a target sequence or a gene that is present in a sample. During PCR a biological sample is subjected to a series of 35 or 40 temperature cycles. A cycle can have multiple temperatures. For each temperature cycle the amount of target sequence can theoretically double and is dependent on a number of factors not presented here. Since the target sequence contains a fluorescent dye, as the amount of target sequence increases i.e., amplified over the 35 or 40 temperature cycles the sample solution fluoresces brighter and brighter with each thermal cycle. The amount of fluorescence required to be measured by a fluorescence detector is frequently referred to as a "threshold", and the cycle number at which the fluorescence is detected is referred to as the "threshold cycle" or $C_t$. Therefore by knowing how efficient the amplification is and the $C_t$, the amount of target sequence in the original sample can be determined.

The tolerated percent variation described above can also be related to the standard deviation of $C_t$ shifts in the instrument. It has been determined that a +/- 15% variation in dye mixture can result in a standard deviation of 0.2 $C_t$s which can be 2 standard deviations.

As presented above the ability to reliably compare experimental results from multiple instruments is desirable and instrument-to-instrument variability is frequently an issue. This variability can result from two sources; variability of components within the instruments such as, for example, lamps and filters and variability over time such as, for example lamp and filter aging. It would be advantageous to implement a process through which experimental results from multiple instruments can be reliably, easily and inexpensively compared. The teachings found herein disclose such a process.

The amount of fluorescent signal of a sample in an optical system can be dependent on several factors. Some of the factors can include, but not be limited to, the wavelength of the fluorescence light, the detector efficiency at that wavelength of fluorescence light, the efficiency of the emission filter, the efficiency of the excitation filter and the efficiency of the dye. The present teachings suggest that instrument-to-instrument variability can be minimized if the physical optical elements of the instruments could be normalized.

In one embodiment the normalization factors can be derived from pure dye spectra rather than from dye mixtures. Pure dyes can be easier to manufacture than dye mixtures, because the concentrations do not have to be exact, and there is only one fluorescent component. This concept was tested by normalizing 2 filter sets in an instrument using 10 pure dyes and comparing the results to the normalization obtained from using dye mixtures. The normalization was implemented by determining a correction factor for each excitation filter and emission filter. The resulting correction factors can be used to normalize any combination of dyes, even from different instruments.

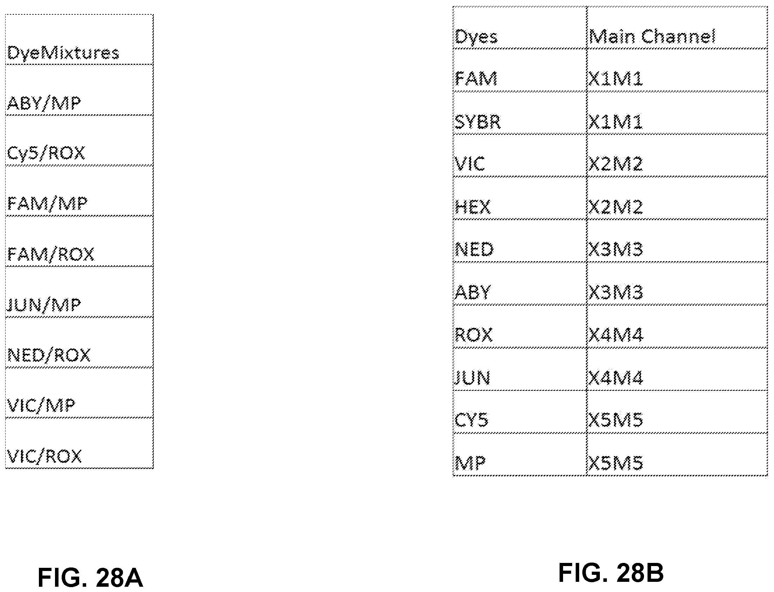

In another embodiment, the normalization taught above was applied to multiple instruments of various types. Eight dye mixture solutions and 10 pure dye solutions were created. Each solution was pipetted into 8 wells of three 96 well plates. Potential spatial crosstalk was minimized by pipetting into every other well. The dye mixtures used are shown in FIG. 28A and the pure dyes used are shown in FIG. 28B. In addition, the instruments used included 6 sets of filters. FIG. 28B further identifies the filter pairs for the main optical channel for each pure dye. The excitation filter is depicted with an "X" and the emission filter is depicted with an "M".

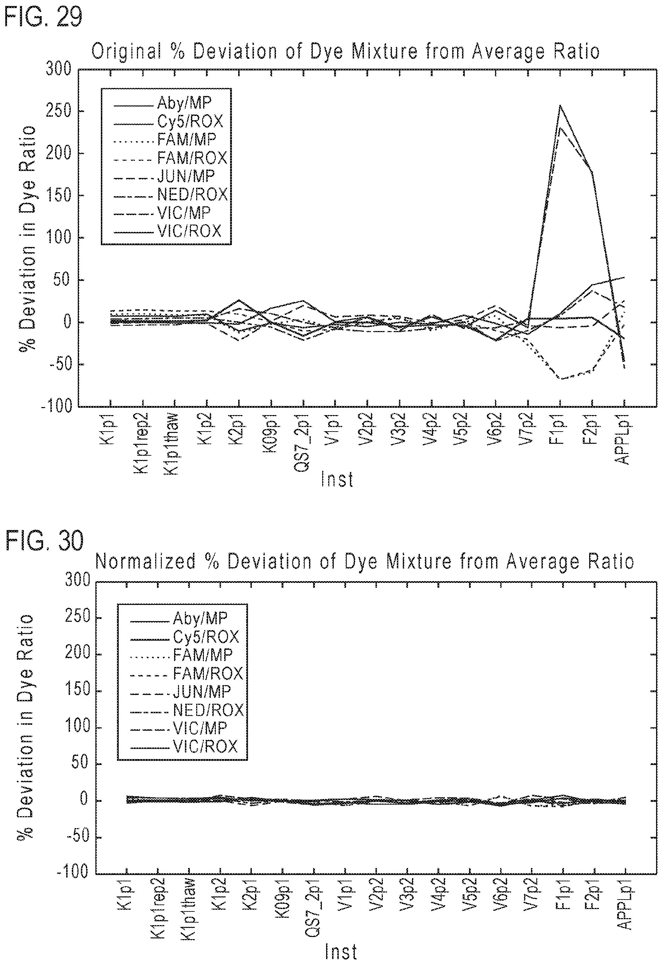

In an effort to quantify the effectiveness of the normalization process, the dye ratios were measured before and after normalization. FIG. 29 shows the percent deviation of dye mixtures from the average ratio for 17 tested instruments. The instruments are labeled on the X-axis and the percent deviation is on the Y-axis. One skilled in the art will notice that the deviations across the instruments are frequently greater than the desired +/-15% previously discussed. This data, therefore, shows a need for an improved normalization process such as the current teachings.

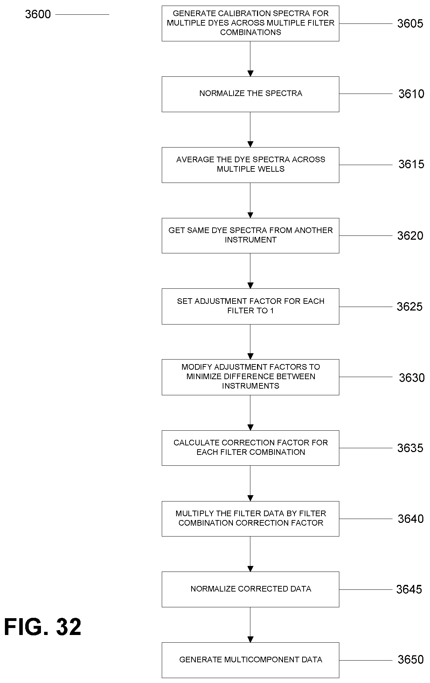

The current teachings were applied to all 17 instruments. The normalization method determines a correction factor for each individual filter rather than for each dye ratio. Because the instruments provided 6 excitation and 6 emission filters, 12 factors were determined. The process is shown in FIG. 32 and flowchart 3600. In step 3605, calibration spectra were generated for multiple dyes across multiple filter combinations. For the instruments being normalized, there were 10 pure dyes and 21 filter combinations. In step 3610, the spectra were normalized so the maximum signal is 1. In step 3615 the dye spectra are averaged across multiple wells. This averaging will result in producing one spectrum per dye. Collectively, the dye spectra can be referred to as a dye matrix "M" containing dye and filter combinations. At this point, a reference instrument is identified. The reference instrument would an instrument or group of instruments that the test instruments will be normalized to. The same set of dye spectra used in the test instrument can be obtained from the reference instrument(s). In some embodiments the reference can be a group of instruments. In such an embodiment the spectra for each dye can be averaged across the group. This step is represented in flowchart 3600 at step 3620. As an example, the reference spectra can be referred to as matrix "Mref".

In step 3625 each of the 12 filters has an adjustment factor initially set to 1. What is desired, is to multiply the adjustment factors by matrix "M" while iteratively modifying the adjustment factors between 0 and 1 and preferably between 0.04 and 1 until the difference between matrix "M" and matrix Mref' is minimized as shown in step 3630. In step 3635, correction factors each filter pair are calculated. The correction factor for each filter pair is the product of the emission filter factor times the excitation filter factor. The main channel filter pairs are shown in FIG. 28B. Once the correction factors for each filter pair has been determined, each filter pair factor can then be multiplied by the fluorescence data for the test instrument as well as for the pure dye spectra. The corrected pure dye spectra can then be renormalized to a maximum value of 1 as shown in step 3645. The final step in the process at step 3650 is to generate multicomponent data. One skilled in the art will understand the multicomponenting procedure to be the product of the fluorescence data and the pseudo-inverse of the dye matrix. The multicomponent values are already normalized so it would not be necessary to make dye specific corrections since the data has been normalized at the filter level.

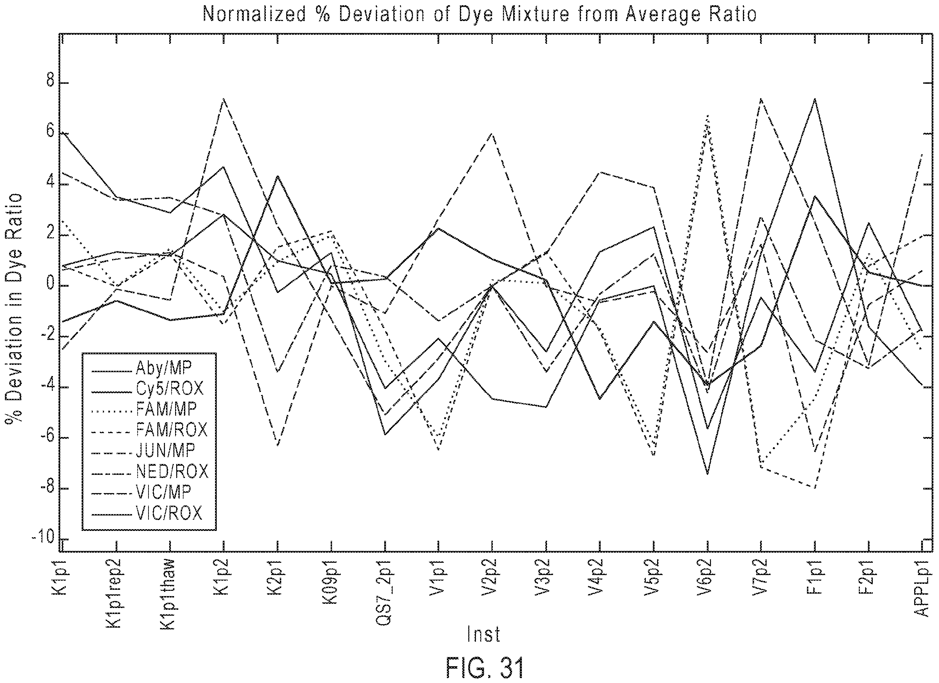

At the completion of normalization the % deviation of dye mixtures from the average ratio were calculated across all 17 instruments. The results are shown in FIG. 30. These results are significantly improved as compared to the data in FIG. 29 before normalization. A closer view of the normalized data is shown in FIG. 31, where the deviations after normalization have been reduced to +/- 8% which is well below the target of +/-15% as presented previously.

RNase P Validation

As mentioned above, it is important to validate an instrument to be sure it is working properly especially after a new installation or after several uses. In this way, a user may be sure experimental results and analyses are accurate and reliable. Previously, a validation assay was run on the instrument by a user and the user manually performed data analysis on the amplification data from the verification assay to validate the instrument. Because the data analysis was performed manually by the user, the validation process was more prone to error and took time.

According to various embodiments of the present teachings, automated validation methods and systems are provided. An example of a validation assay is an RNase P assay. However, as used herein, validation assay may be any assay that has known and reliable properties and can be used to validate an instrument.

After installation and after several uses, it is important to validate that the instrument is working properly. Often, a user will manually run a known assay to validate an instrument, such as an RNase P assay. The RNase P gene is a single-copy gene encoding the RNA moiety of the RNase P enzyme. It is often used as a validation assay because of its known properties and characteristics.

A validation plate is preloaded with the reagents necessary for the detection and quantitation of genomic copies of the sample. For example, in an RNase P validation plate, each well contains PCR master mix, RNase P primers, FAM™ dye-labeled probe, and a known concentration of human genomic DNA template.

In a traditional RNase P assay example, a standard curve is generated from the $C_t$ (cycle threshold) values obtained from a set of replicate standards (1,250, 2,500, 5,000, 10,000 and 20,000 copies). The standard curve is then used to determine the copy number for two sets of unknown templates (5,000 and 10,000 replicate populations). The instrument is validated if it can demonstrate the ability to distinguish between 5,000 and 10,000 genomic equivalents with a 99.7% confidence level for a subsequent sample run in a single well.

To pass installation, the instruments must demonstrate the ability to distinguish between 5,000 and 10,000 genomic equivalents with a 99.7% confidence level for a subsequent sample run in a single well.

According to various embodiments, the present teachings can incorporate expert knowledge into an automated calibration and validation system providing pass/fail status and troubleshooting feedback when a failure is identified. If an instrument should fail the validation process, then the user knows that a service engineer can be called, for example. The present teachings can minimize the cost of, and time required for, the installation and calibration procedures.

As stated above, according to various embodiments described herein, the goal of a validation analysis is to confirm that two quantities of the same sample are sufficiently distinguishable by the instrument. This way, the instrument performance may be validated.

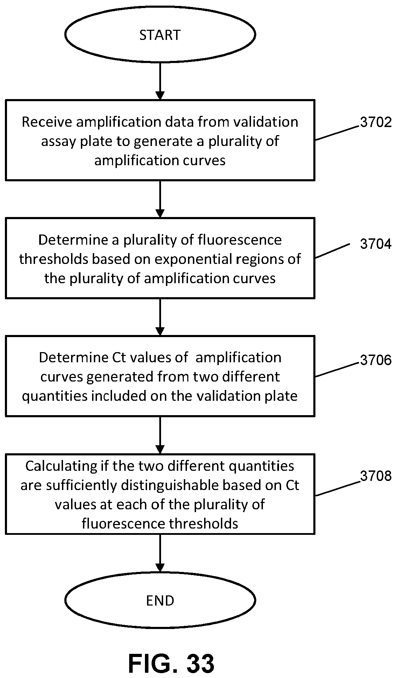

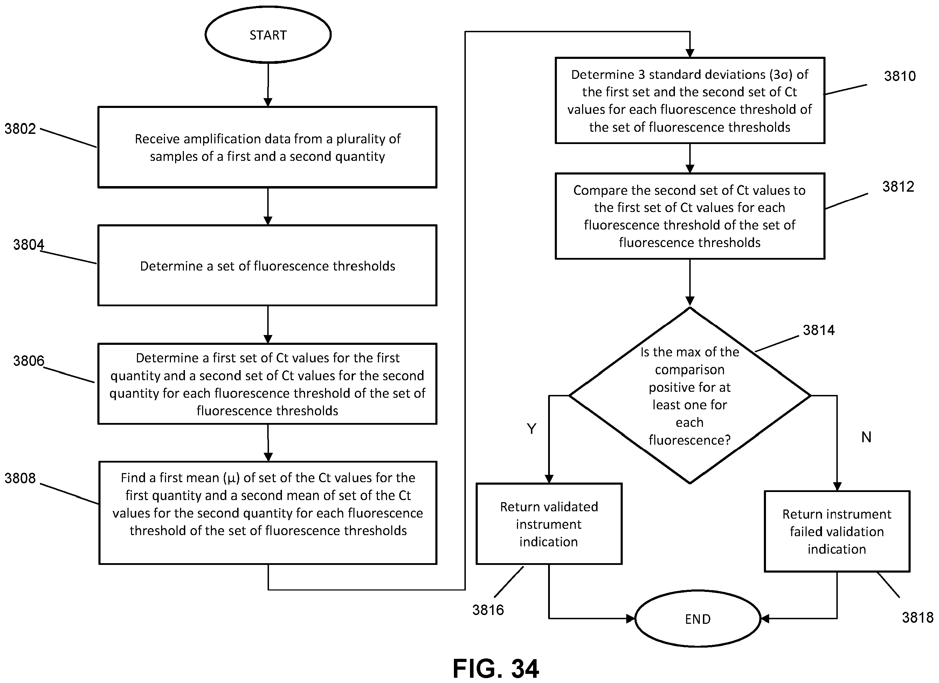

According to various embodiments of the present teachings, an automated validation method and system is provided. Cycle threshold values ($C_t$s) of a validation assay are analyzed and compared by a system to determine if an instrument can sufficiently distinguish two quantities of a sample. An example of a validation assay is the RNase P assay. In this example, a system determines $C_t$ values generated for RNase P samples of 5000 and 10000 genomic copies to determine if the data from the 5000 and 10000 genomic copies are sufficiently distinguishable. Sufficiently distinguishable, according to the embodiments described herein, means at least 3 standard deviations ($3\sigma$) (-99.7%) separate the amplification data from two quantities. In this example, the two quantities are 5000 and 10000 genomic copies. The method according to various embodiments is described below with reference to FIGS. 33 and 34.

FIG. 33 illustrates an exemplary method for validating an instrument according to various embodiments described herein. In general, the begins in step 3702 by receiving amplification data from a validation assay plate to generate a plurality of amplification curves, each corresponding to a well on the plate.

Plates contain a plurality of wells. In some examples, a plate contains 96 wells. In other examples, a plate contains 384 wells. A portion of the wells in the plate may contain a sample of a first quantity and another portion of the wells in the plate may contain a sample of a second quantity. The first quantity and the second quantity are different. The second quantity is greater than the first quantity in various embodiments described herein. The second quantity may be a 1.5 fold difference than the first quantity in some embodiments. In other embodiments, the second quantity may be a 2 fold difference than the first quantity. According to various embodiments described herein, the second quantity may be any fold difference than the first quantity. In some embodiments, the first quantity may be 5000 genomic copies per well and the second quantity may be 10000 genomic copies per well.

With reference back to FIG. 33, in step 3704, a plurality of fluorescence thresholds are determined based on the plurality of generated amplification curves. Exponential regions of the plurality of amplification curves are compared to determine a range of fluorescence values where the exponential regions fall. For example, the range of fluorescence values from the lowest fluorescence value of a bottom of an exponential region to the highest fluorescence value of a top of an exponential region of the plurality of amplification curves is determined. The fluorescence value range is used in the automated analysis of the plurality of amplification curves to validate the instrument according to embodiments of the present teachings.

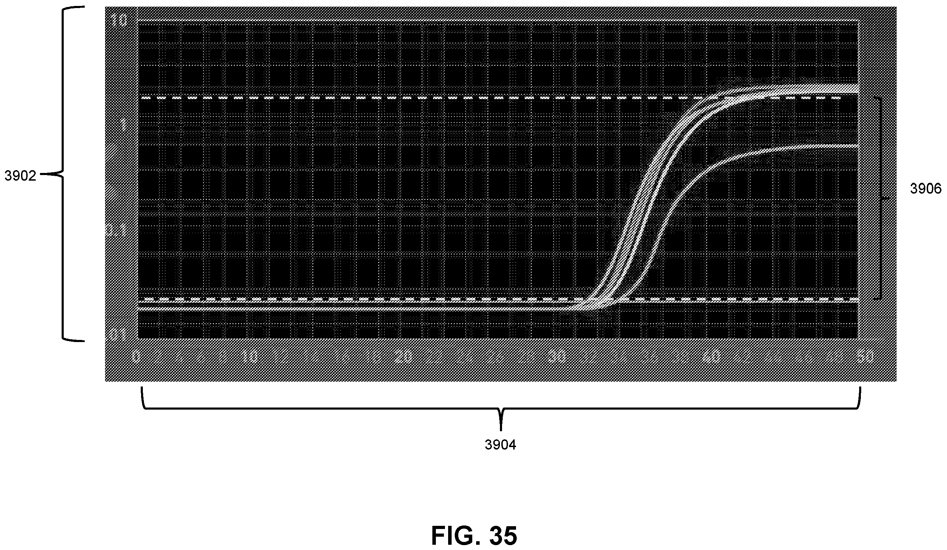

With reference to FIG. 35, a plurality of amplification curves and determination of a range of fluorescence values and corresponding cycle threshold is illustrated. Each of the plurality of amplification curves includes an exponential region of the curve. Axis 3902 indicates fluorescence values. Axis 3904 illustrates cycle numbers. Fluorescence range 3906 shows the range of fluorescence values from the lowest fluorescent value of a determined bottom of an exponential region of the plurality of exponential regions and highest fluorescent value of a determined top of an exponential region of the plurality of exponential regions. According to various embodiments, the range of fluorescence values is divided evenly by a predetermined number to generate a set of fluorescence values for automated analysis by the system. In one example, the range of fluorescence values 3906 is divided by 100 to determine 100 fluorescence values for a set of fluorescence thresholds. In some embodiments, the top 5 fluorescence values and the bottom 5 fluorescence values are discarded so that analysis proceeds with a set of 90 fluorescence thresholds.

With reference back to FIG. 33, in step 3706, for each fluorescence value of the set of fluorescence values, the cycle threshold ($C_t$) is determined for each of the plurality of amplification curves generated from wells containing the first quantity of the sample. Similarly, for each fluorescence value of the set of fluorescence values, the cycle threshold ($C_t$) is determined for each of the plurality of amplification curves generated from wells containing the second quantity of the sample.

In step 3708, using the $C_t$ values for the first and second quantities for each of the fluorescence values of the set, it is determined if the first and second quantities are sufficiently distinguishable. Sufficiently distinguishable, according to various embodiments, means that, using equation (1), yields a positive result for at least one of the fluorescence values of the set:

$$\left(\left(\mu C_{tquant1} - 3\sigma C_{tquant1}\right) - \left(\mu C_{tquant2} + 3\sigma C_{tquant2}\right)\right) \tag{1}$$

Equation 1 determines if a first and second quantity are sufficiently distinguishable, where quant2 is greater than quant1, according to the embodiments described herein. Sufficiently distinguishable means at least 3 standard deviations ($3\sigma$) (-99.7%) separate the $C_t$ values of the first and second quantities. If it is found that the quantities are sufficiently distinguishable, an indication is provided to the user that the instrument is validated. The indication may be provided to the user on a display screen.

FIG. 34 illustrates another exemplary method for validation an instrument according to various embodiments described herein. In step 3802, amplification data is received from a plurality of samples included in wells of a validation plate. A portion of the wells in the validation plate contain a sample in a first quantity. Another portion of the wells of the validation plate contain the sample in a second quantity. The first quantity and the second quantity are different. The second quantity is greater than the first quantity in various embodiments described herein. The second quantity may be a 1.5 fold difference than the first quantity in some embodiments. In other embodiments, the second quantity may be a 2 fold difference than the first quantity. According to various embodiments described herein, the second quantity may be any fold difference than the first quantity. In some embodiments, the first quantity may be 5000 genomic copies per well and the second quantity may be 10000 genomic copies per well.

In step 3804, a first set of fluorescence thresholds are determined based on the plurality of generated amplification curves. Exponential regions of the plurality of amplification curves are compared to determine a range of fluorescence values where the exponential regions fall. For example, the range of fluorescence values from the lowest fluorescence value of a bottom of an exponential region to the highest fluorescence value of a top of an exponential region of the plurality of amplification curves is determined. The fluorescence value range is used in the automated analysis of the plurality of amplification curves to validate the instrument according to embodiments of the present teachings.

According to various embodiments, the range of fluorescence values is divided evenly by a predetermined number to generate a set of fluorescence values for automated analysis by the system. In one example, the range of fluorescence values 3906 is divided by 100 to determine 100 fluorescence values for a set of fluorescence thresholds. In some embodiments, the top 5 fluorescence values and the bottom 5 fluorescence values are discarded so that analysis proceeds with a set of 90 fluorescence thresholds.

In step 3806, for each fluorescence threshold of the set, a first set of $C_t$ values for the amplification curves corresponding to the first quantity is determined. Similarly, for each fluorescence threshold of the set, a second set of $C_t$ values for the amplification curves corresponding to the first quantity is determined. This is repeated for every fluorescence threshold in the set.

In some embodiments, a predetermined number of outlier $C_t$ values are removed from each set of $C_t$ values before further calculations are performed. For example, in some embodiments, if a 96 well plate is used, 6 outliers are removed from each set of $C_t$ values. An outlier is the $C_t$ values furthest away from the mean value of the set of $C_t$ values. In another example, if a 364 well plate is used, 10 outliers are removed from each set of $C_t$ values. After the outliers are removed, the remaining $C_t$ values of each set are used in the remaining steps of the method.

In step 3808, for each set of $C_t$ values, a mean is calculated. In other words, a first $C_t$ mean is calculated for the first quantity amplification curves and a second $C_t$ mean is calculated for the second quantity amplification curves for each fluorescence threshold of the set determined in step 3804.

Similar to step 3808, in step 3810, 3 standard deviations of each set of $C_t$ values is calculated. In other words, a first 3 standard deviations is calculated for the first quantity amplification curves and a second 3 standard deviations is calculated for the second quantity amplification curves for each fluorescence threshold of the set determined in step 3804.

To determine if the $C_t$ values of the first quantity and the second quantity or sufficiently distinguishable, the $C_t$ values at a fluorescence value, according to various embodiments, the $C_t$ values are compared. According to various embodiments, equation (1) is used for the comparison.

$$\left(\left(\mu C_{tquant1} - 3\sigma C_{tquant1}\right) - \left(\mu C_{tquant2} + 3\sigma C_{tquant2}\right)\right) \qquad (1)$$

Equation 2 determines if a first and second quantity are sufficiently distinguishable, where quant2 is greater than quant1, according to the embodiments described herein. Sufficiently distinguishable means at least 3 standard deviations ($3\sigma$) (-99.7%) separate the $C_t$ values of the first and second quantities.

In step 3814, the results of equation (2) for all fluorescence thresholds of the set are compared to determine a maximum value. If the maximum value is a positive number, the instrument can sufficiently distinguish between the first and second quantity and an indication that the instrument is validated is provided to the user in step 3816. If the maximum value is a negative number, the instrument cannot sufficiently distinguish between the first and second quantity and an indication the instrument failed validation is provided to the user in step 3818.

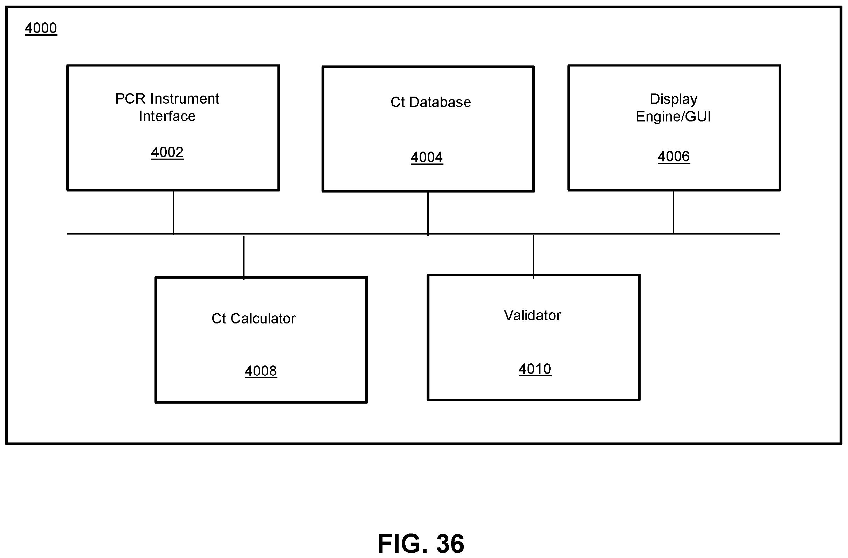

FIG. 36 illustrates system 4000 for validation of an instrument according to various embodiments described herein. System 4000 includes PCR instrument interface 4002, $C_t$ database 4004, display engine/GUI 4006, $C_t$ calculator 4008, and validator 4010.

PCR instrument interface 4002 receives the amplification data from the PCR instrument to generate amplification curves. As described above, the PCR instrument amplifies the samples contained in the validation plate. The validation plate includes a portion of wells containing a sample of a first quantity and another portion of wells containing a sample of a second quantity. Fluorescence data generated from amplification of the samples is received by PCR instrument interface 4002.

After a set of fluorescence thresholds are determined as in steps 1704 and 1804, with reference to FIGS. 33 and 34, respectively, $C_t$ calculator 4006 calculates a first and second set of $C_t$ values corresponding to the amplification curves generated from the samples of the first quantity and the second quantity, respectively. A first and second set of

$C_t$ values is calculated for each fluorescence threshold in the set of fluorescence thresholds. The plurality of sets of $C_t$ values are stored in $C_t$ database 4004.

Validator 4010 determines whether the first and second quantities are sufficiently distinguishable as described in step 3708 in FIG. 33 and steps 3810 and 3812 in FIG. 34.

Display engine/GUI displays the plurality of amplification curves to the user. Further, after validator 4010 determines whether the first and second quantities are sufficiently distinguishable, display engine/GUI 4006 displays an indication of validation or failed validation to the user.

Auto-Dye Correction

According to various embodiments of the present teachings, auto-dye correction methods may be used to perform a real-time spectral calibration of the multi-component data. Auto-dye correction may be performed in real-time or after amplification data is collected and secondary analysis is performed. In the auto-dye correction algorithm, a multicomponent correlation matrix is generated. According to various embodiments, an auto-dye correction algorithm adjusts the elements of the dye matrix so that the off diagonal terms in the multicomponent correlation matrix are minimized. In this way, errors in $C_t$ determinations are minimized.

Auto-Background Correction

According to various embodiments of the present teachings, an auto-background calibration may be performed to reduce the need for a background calibration plate and improve the overall efficacy of background correction.

Physical contaminants in the block (particulate or chemical) that occur over use of the instrument can negatively-impact the analysis results of the system by artificially inflating certain spectral components of the analyzed wells that are impacted by contamination. A recalibration can address this problem. However, to prolong periods between required calibrations, a method of automatically-calculating/compensating for background changes after background calibration is described. To accomplish auto-background calibration, a method is performed using the empty/unoccupied block. The effective signal bleed-through for consumables is known (empirically determined), and effective background calibration slopes and offsets can be approximated using scaling factors that address the effective signal bleed-through.

Plate Detection

According to various embodiments described herein, plate detection methods may be performed to identify errors in plate placement in the instrument.

During instrument use, the optics of the system are positioned at either the upper limit (during idle periods) or at the lower limit (during operation) of travel. The ability to readout the optics position at an intermediate location between the travel limits was not designed into the hardware; as such, one cannot rely on the motor position value to determine if a plate or tube is present or absent (where the difference in optics position would be caused by the added material thickness from the tube or plate present). Without needing an added component for plate or tube detection (such as a depression switch or positional sensor), the detection camera in the system is used for sample detection. However, since only a small portion of the block region is captured through the use of a discrete and segregated well lens array (each lens in the array focuses and collects light from one and only one well), a traditional 'photo' of the consumable plane capturing the entire block region cannot be acquired for image processing. Since only focused light from each well is collected and manifests as a circulate spot of brightness on the detector, there is no spatial or dynamic range in the detected image. However, if the optics are moved to an intermediate position that allows for focusing on the seal or lid of a container, this focus spot can be captured as a reflected image (contrasted with fluorescence, which is the normal signal collected by the system), and used for plate/tube detection. The spot of focus would be smaller than a well, and this would manifest in the captured image as a small bright region relative to the size of a well (known as the region of investigation, ROI). Understanding that the focus spot would yield bright pixels and all other regions would yield darker pixels, a numerical analysis of the pixel-level information can yield a presence/absence determination, according to various embodiments described herein.

Instrument Normalization Using a Reflective Material

According to various embodiments of the present teachings, instrument normalization using a reflective material, such as a photodiode, may be used to auto-calibrate the instrument after any initial calibrations done after manufacturing or installation.

According to various embodiments, a stable reflective material is measured during manufacturing as a control. The reflective material may be placed above the heated cover. Subsequently, the stable reflective material can be measured in all channels to detect any changes or variability. Any changes or variability may be used to adjust color balance factors, as described above in the instrument normalization calibration method to re-normalize for the changes in the excitation light.

THERMAL BLOCK TRAY

As summarized above and illustrated in FIG. 1, thermal cycler system 100 can include sample cover 114, heat/cool elements 116, and heat exchanger 118.

Instruments for analyzing biological samples frequently provide a researcher with the ability to manually or automatically place biological samples into a sample loading region of an instrument for analysis. In some embodiments a cover can be raised and a container capable of containing a biological sample can be placed into the sample loading region of an instrument. In other embodiments, a door can be opened to insert a container capable of containing a biological sample into the sample loading region of an instrument.

In another embodiment, a drawer or tray can be slid out of the instrument to allow a container capable of containing a biological sample to be inserted into the sample loading region of an instrument wherein the container is inserted into the instrument upon closing the drawer. In another embodiment the container capable of containing a biological sample can be inserted into the instrument through the use of, for example, springs, latches, handles and levers. In still other embodiments access to the sample loading area of the instrument can be automated. This can frequently be done for instruments where robotics are used in high throughput environments. Covers, doors and drawers can be automated through the use of motors. Automated embodiments can also be found in instruments that are user friendly to assist a researcher in loading biological samples into an instrument for analysis. Automation can be controlled by interfacing the instrument with a computer system programmed to provide motion to assist with loading biological samples.

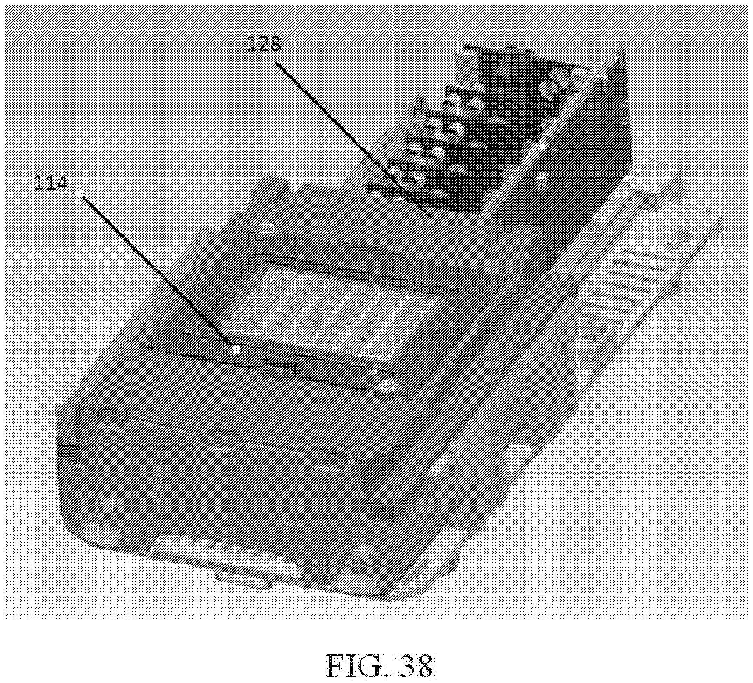

FIG. 5, discussed above, illustrated an embodiment of thermal cycler system 100 with a movable drawer or tray 160 in an open, exposed position. FIG. 38 illustrates an embodiment for an automated drawer or tray with sample block assembly provided therein. As will be discussed below, a sample block assembly can include those components that contribute to controlling temperature of the sample block. Sample block 114 can be used to hold a container of biological samples. Sample block 114 can also provide heating and cooling to affect a change in temperature of the biological samples. Changing the temperature of biological samples during analysis is known in the art when performing Polymerase Chain Reaction, also known as PCR. Electronics 128 can be interfaced to the sample block through a system of wires and connectors, for example, to control the temperature of the sample block. The assembly of FIG. 38 can be one of several assemblies making up an instrument for analysis. Additional assemblies in an instrument can include, but not be limited to, power supplies, optical excitation, optical emission, data communications and user interface. All of the mentioned assemblies can also be interfaced to a computer to control data collection and timing of the instrument.

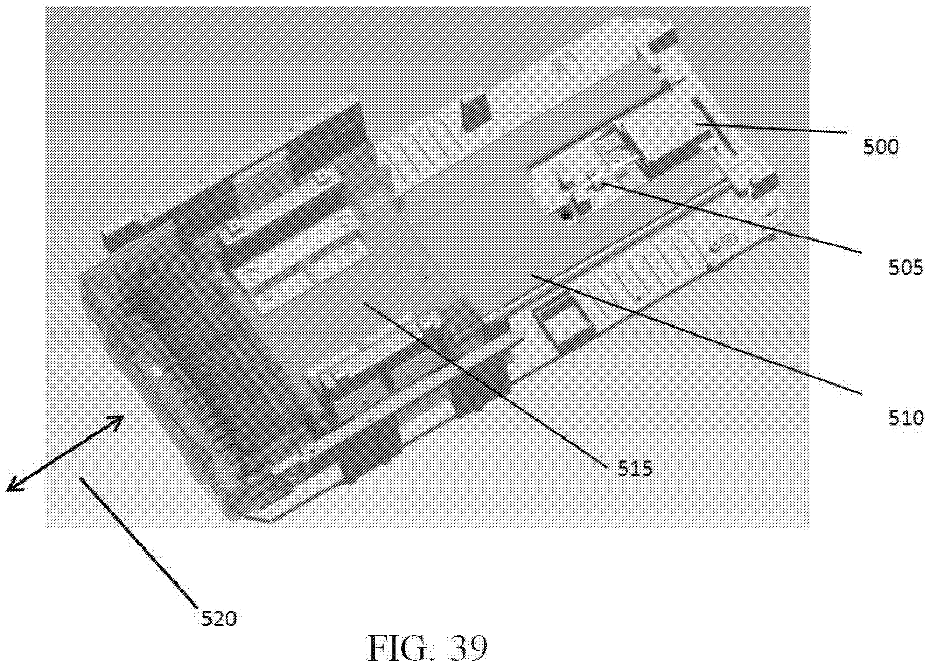

FIG. 39 illustrates the assembly of FIG. 38 with block assembly 114 and electronics 128 removed. FIG. 39 illustrates an example of an automated system, which can provide a user with access to the sample loading region, for example block assembly 114, of the instrument. FIG. 39 illustrates motor 500, coupling 505, slide 510 and block support 515. In such a configuration block support 515 can be mechanically connected to slide 510 by any number of fasteners known in the art. Some examples of fasters can be, but not be limited to, screw, bolts, rivets, and any other faster suitable to securely fasten block support 515 to slide 510. By securely fastening block support 515 to slide 510, block support can be moved in the direction indicated by arrow 520. The ease with which the block support can be moved can be facilitated through the use of bearings or slippery surfaces such as, for example, Teflon (not shown). FIG. 39 further shows motor 500. Motor 500 can be any suitable motor known in the art, such as, a DC motor, an AC motor or a stepper motor to name a few. In any case motor 500 can be interfaced to electronics presented above to provide rotational motion of the motor's shaft. Motor 500 can also be interfaced to a computer system programmed to control the direction and speed of the motor precisely if necessary. As previously presented motor 500 can provide rotational motion that can be converted to a linear motion as depicted by arrow 520. The conversion from rotational motion to linear motion can be accomplished through coupling 505 and one or more lead screws (not shown). Such a configuration, therefore, can provide automated motion of the block support in a controlled manner. Coupling 505 can additionally provide compensation for any misalignment between motor 500 and lead screw (not shown).

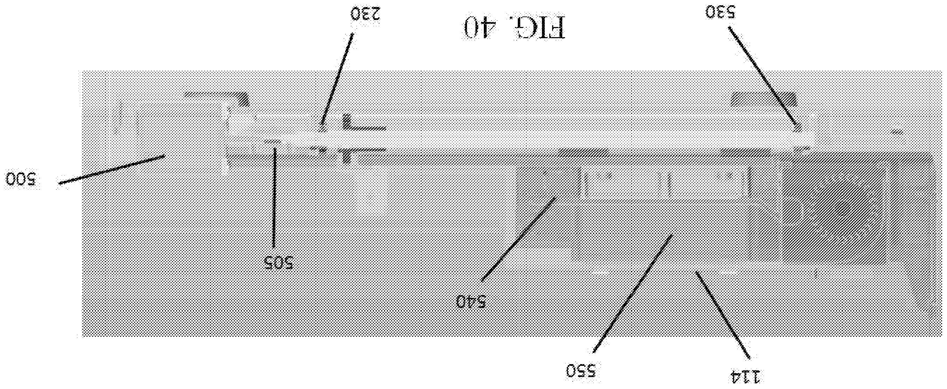

Moving to FIG. 40, a side view of the assembly is illustrated. Sample block 114, motor 500 and coupling 505 can be seen. Additionally bearings 530 are also depicted. Bearings 530, as presented previously, can assist in providing smooth translational motion of the block assembly. The size and type of bearings 530 are dependent on the load the bearings have to support. Further details of bearings 530 will be presented later.

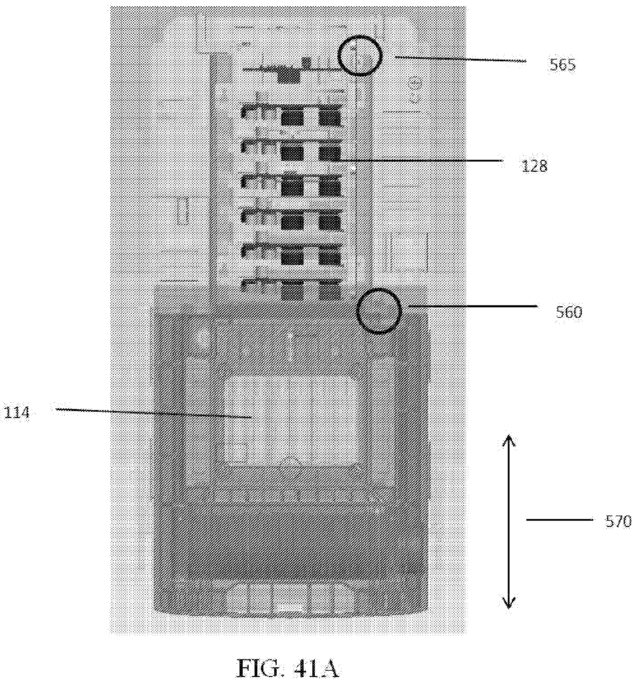

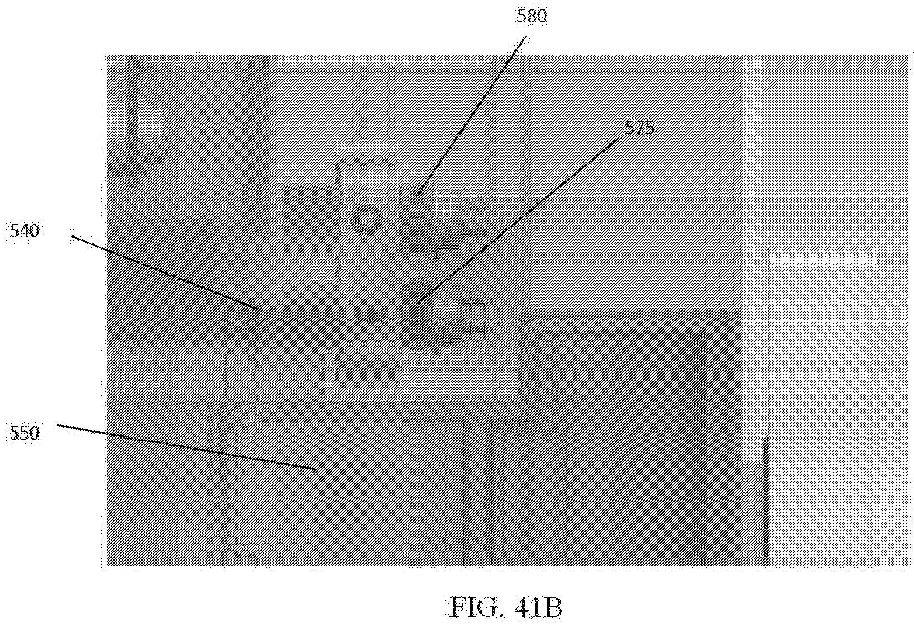

One skilled in the art will understand that automated systems frequently include some type of positional feedback to a motion controller. Feedback may be fine or course or a combination of the two depending on the system being controlled. For example, stepper motors can provide accurate positioning based on the size and number of steps the motor moves. For stepper systems a computer can be programmed to count steps for determining the location of the device being moved either rotationally or linearly. FIG. 41A illustrates a top view of block assembly 114 and electronics 128 as previously presented. As previously discussed, block assembly 114 and electronics 128 are secured to rail 510 of FIG. 39 and coupled to motor 500 of FIG. 39 to provide motion according to arrow 570 of FIG. 41A. Arrow 570 is used to represent movement of block assembly 114 and electronics 128 "in" and "out" of the instrument. As depicted in FIG. 41A the assembly is said to be in and is evidenced by area 560, a close up of which is illustrated in FIG. 41B as is described below.

FIG. 41B depicts 2 optical sensors 575 and 580. Optical sensors continuously emit and detect infrared light across a gap. One side of the gap is an emitter and the other side is a receiver. As long as power is applied to the device the emitter constantly emits infrared light and the receiver continuously detects that light. If an opaque object is inserted in the gap, the emitter continues to emit the infrared light but the light is blocked from reaching the receiver. The difference between receiving light and not receiving light can be detected and identified by a computer programmed to detect the difference. As a result the computer can detect if the light is blocked or not blocked. Automated systems can use this effect by mounting or providing an opaque object connected to a moving object to detect whether the moving object is at the location of the optical switch or not and motor 500 would be turned off.

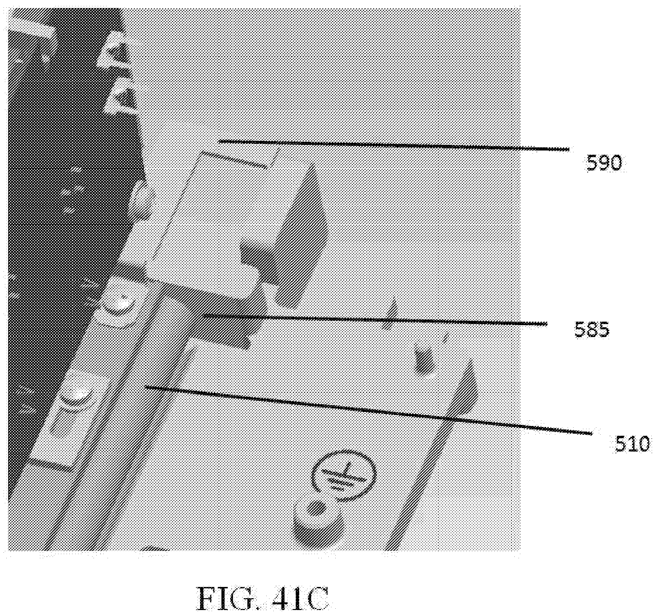

Referring back to FIG. 41B, tab 540 can be seen in outline. Tab 540 can also be seen in FIG. 40 and is an opaque tab of air duct 550. As shown, tab 540 is in the gap of optical switch 575 and as such represents the "in" position of the block assembly and electronics. One skilled in the art will understand that electrical components can fail in their operation. A failed "in" switch would not be able to detect the "in" condition and motor 500 would not be turned off. In order to prevent damage to the assembly, hard stop 585 is provided at the rear of slide 510. This is depicted is FIG. 41C.

FIG. 41B also depicts optical switch 580, and can be configured to detect when the assembly is all the way "out". Optical switch 580 works as described above for optical switch 575 in that it emits and detects infrared light across a gap. If an opaque object is inserted in the gap a programmed computer can detect if the light path or blocked or not blocked. For the "out" condition tab 590 is provided at the back of rail 510 as shown in FIG. 41C.

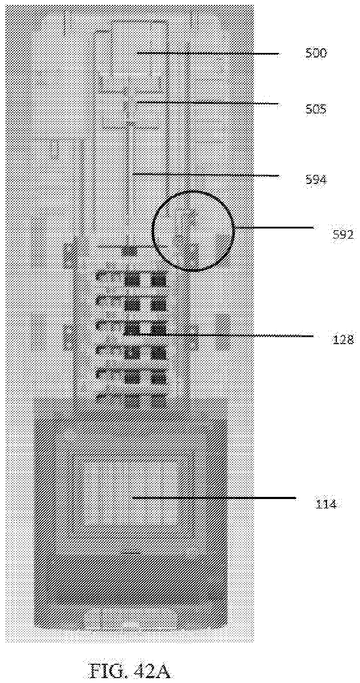

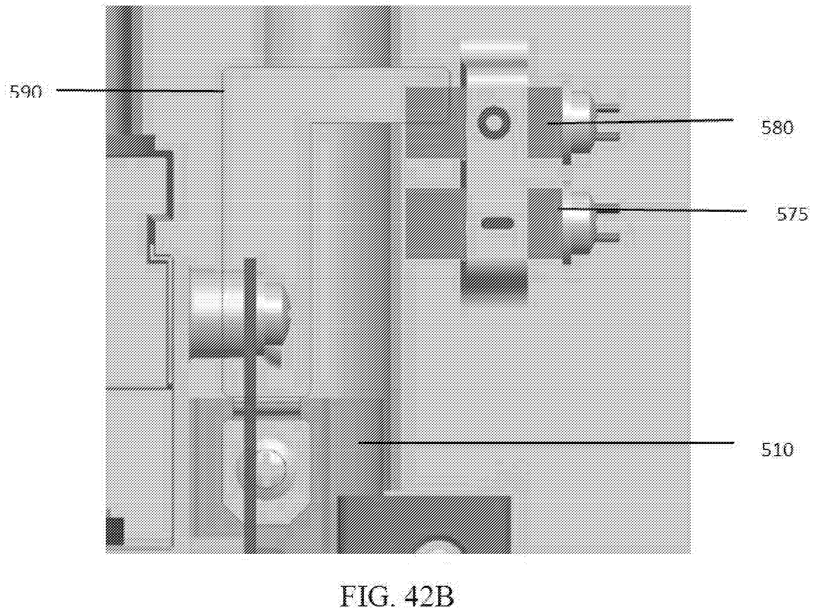

FIG. 42A illustrates an assembly that is in the "out" position. This is evident because motor 500, coupling 505 and a lead screw 594 are visible. In the "in" position of FIG. 41A, these components are not visible. The detection of the "out" position is shown in an area 592 and a close up is illustrated in FIG. 42B. FIG. 42B shows the 'in' optical switch 575 previously discussed. In addition, opaque tab 590 is shown in outline, attached to rail 510 and located in the gap of optical switch 580. A programmed computer, as discussed above, can detect whether tab 590 is in the gap or not and therefore "know" when the assembly is all the way "out".

THERMAL BLOCK

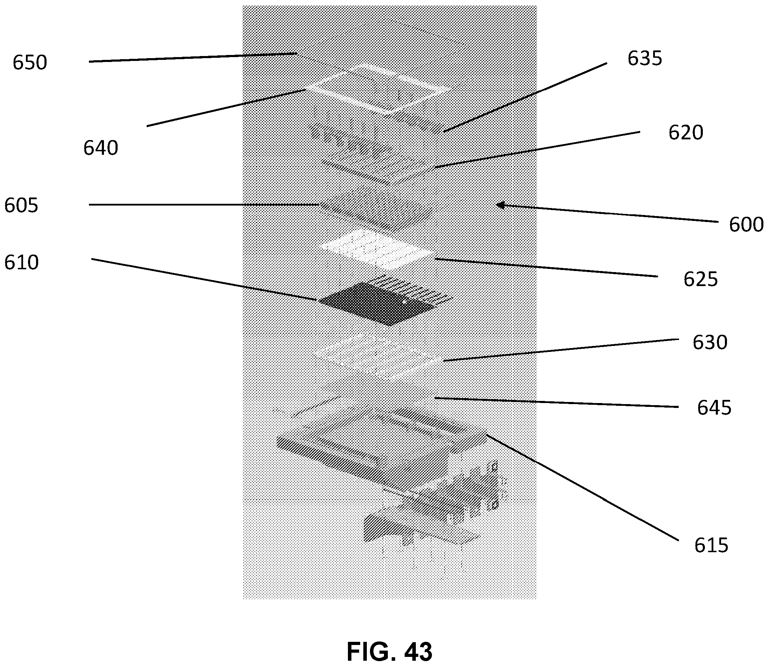

As summarized above and illustrated in FIG. 1, thermal cycler system 100 can include sample block 114, heat/cool elements 116 and heat exchanger 118. Together, these elements can be referred to as the sample block assembly. FIG. 43 illustrates an embodiment of a sample block assembly 600 according to various embodiments described herein. Assembly 600 can include a sample block (or multiple blocks) 605, a thermoelectric cooler (TEC) (or multiple TECs) 610 and associated frame 630, and a heat sink 615. Frame 630 is designed to receive and align TECs 610 with block 605 and heat sink 615.

While sample block 605 can be a single, unitary block, FIG. 43 illustrates multiple blocks that together make up sample block 605, with a seal 620 fitting in gaps between the multiple blocks and an interface foil 625 below block 605. Again, depending on block 605 design (single vs. multiple blocks), foil 625 can either be a single foil having substantially the same dimensions as block 605, or can be multiple foils each having substantially the same dimensions as the individual blocks of the multiple block design. Similarly, foil 625 can mimic the number and dimensions of TECs 610 used. Foil 625 can be made, for example, from aluminum.

Block 605 can be fixed, or clamped, to other components of the block assembly such as, for example, heat sink 615. Alternatively, block 605 can be floating. Floating block 605 may not be constrained, or fully constrained, by screws and/or other attachments. Floating block 605 may sit on a provided flat surface or surfaces to keep block 605 substantially aligned with the other components of the block assembly. However, floating block 605 can move laterally at all sides. Generally, such movement will be limited to prevent block 605 from getting misaligned with, for example, the heated cover, heat sink and/or TECs. The assembly may provide, for example, an abutment that constrains the lateral movement. Movement can be restrained, for example, to 1mm at all sides. By allowing such constrained lateral movement, the floating block can adjust to any stacked up tolerances and misalignment that the block may have to the heated cover due to the automated in and out movement of the slide rail as discussed above.

Assembly 600 of FIG. 43 also includes clamps 635, which rest along the major lengths of block 605 and clamps TECs 610 to block 605. Floating heater 640 is also provided in assembly 600 and can be located along an exterior perimeter ledge of block 605, the ledge being at the bottom of the block nearest to the base of the wells on the block. Heater 640 can be, for example, a kapton heater with one side coated with aluminum foil, and can be used to offset cold temperatures around the perimeter wells as compared to the more centrally located wells.

A second interface foil 645 can be provided between TECs 610 and heat sink 615. Dimensions of foil 645 can, like foil 625, mimic the number and dimensions of TECs 610 used. It may also be a single piece of foil along the total surface area of TECs 610. Foil 645 can be made, for example, from aluminum.

Seal 650 can be provided on a top surface of heat sink 615. This seal can interface with, for example, a drip pan surrounding the perimeter of sample block 605. The seal between sample block and heat sink helps prevent moisture from entering that sealed chamber and damaging TEC functionality.

HEATED COVER

As summarized above and illustrated in FIG. 1, thermal cycler system 100 includes a heated cover 110.

In a large number of PCR instruments, sample tubes or microtiter plates are inserted into sample wells on a thermal block assembly. To perform the PCR process, the temperature of the thermal block assembly is cycled according to prescribed temperatures and times specified by the user in a PCR protocol file. The cycling can be controlled by a computing system and associated electronics. As the thermal block assembly changes temperature, ideally the samples in the various tubes or plate experience similar changes in temperature. However, there can be various factors that can affect the efficiency of the thermal transfer from the thermal block to the samples and also the efficiency of the sample reaction. Examples of various factors can include how well the sample tube is contacting the sample well on the thermal block assembly and how much condensation or evaporation takes place within the sample tube or plate as the sample is heated and cooled. For at least these reasons instruments frequently also include a heated cover that can be located above and in contact with the sample tube or plate. A heated cover as presented can provide a downward force to the tubes or plate to improve the thermal contact between the thermal block assembly and the sample and also provide heat to the top of the tubes or plate to minimize condensation and evaporation.

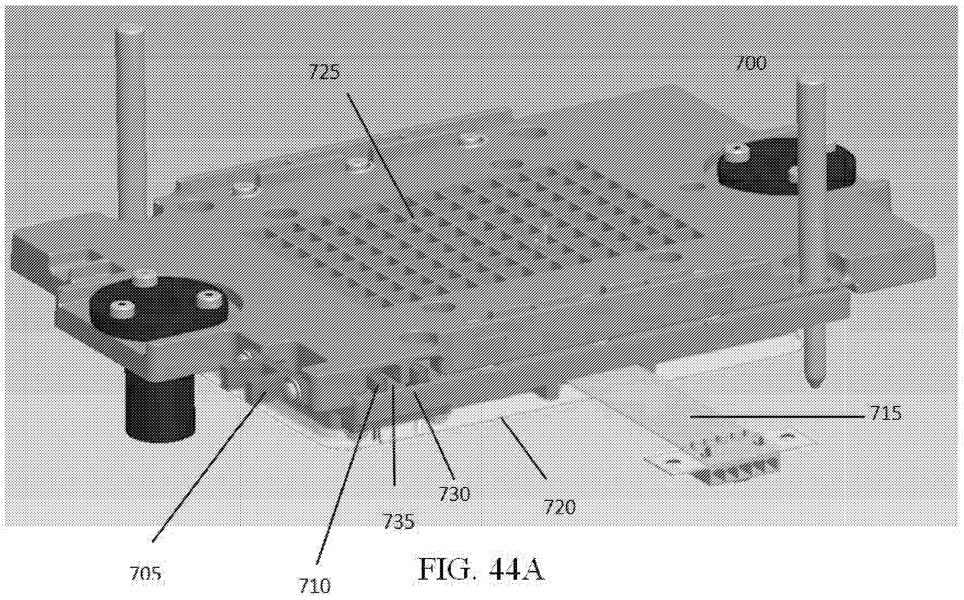

An example of such a heated cover is illustrated in FIG. 44A. Heated cover assembly 700 is depicted according to the present teachings. Lower plate 720 provides a surface that can mate with the upper surfaces of sample tubes or plates. In one embodiment, lower plate 720 can include a flat uninterrupted mating surface. In another embodiment, lower plate 720 can include depressions. In another embodiment the depressions can be through-hole apertures. Through-hole apertures can be beneficial in some instruments to allow optical detection of samples contained in the sample vessels. In another embodiment the number of through-hole apertures can be 96 apertures. In another embodiment the number of apertures can be 384. In another embodiment the number of through-hole apertures can equal the number of sample vessels. As presented above, one benefit of the heated cover is to provide sufficient heat to the tops of the sample vessels to minimize condensation and evaporation of the sample. As such lower plate 720 can be heated. In FIG. 44A heating of lower plate 720 can be provided by heater 715. Heater 715 can be any number of types known in the art. Heater 715 can further be attached to lower plate 715 by any number of techniques known in the art like, for example, vulcanization, pressure sensitive adhesive, epoxy and adhesive tape. Heater 715 can be controlled by a programmed computer to provide an amount of heat necessary to prevent evaporation and evaporation. In some embodiments heater 715 can be heated to 95°C. In another embodiment heater 715 can be heated to between 95°C and 105°C. In another embodiment heater 715 can be heated to greater than 105°C.

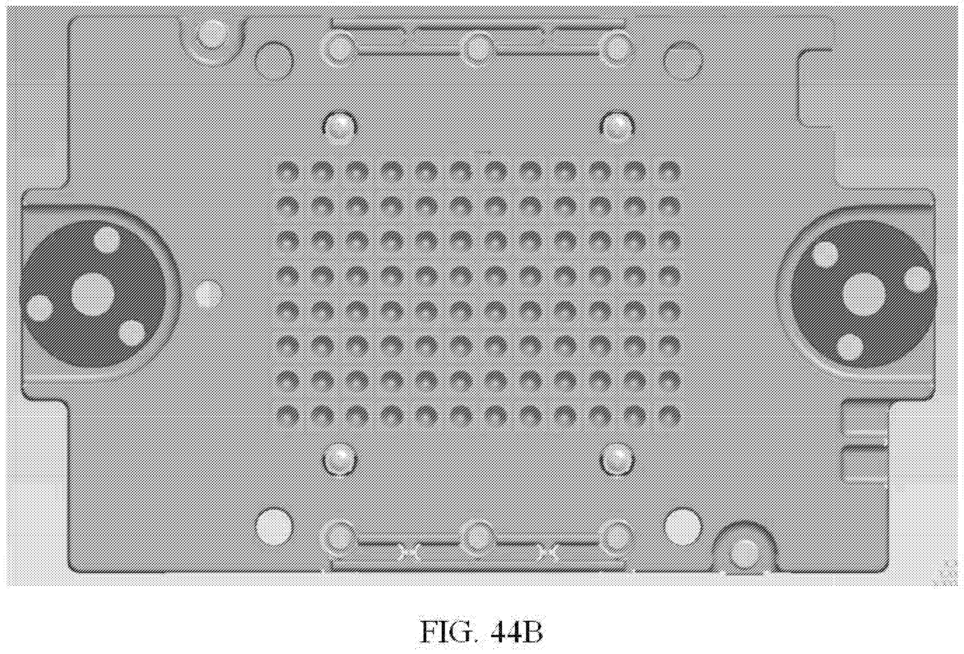

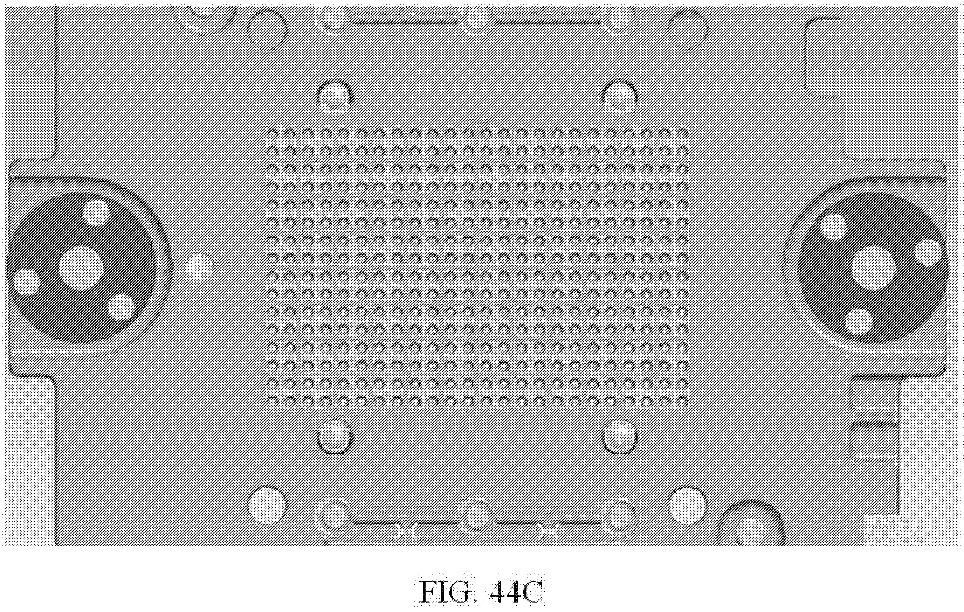

Heated cover assembly 700 further includes top plate 725. Top plate 725 is depicted with depressions in the upper surface. The depressions can align with the depressions of lower plate 720 as described previously. In one embodiment the depressions in upper plate 725 can be through-hole apertures. In another embodiment the through-hole apertures can allow optical detection of samples contained in the sample wells. In another embodiment the number of through-hole apertures can be 96 apertures. In another embodiment the number of apertures can be 384. In another embodiment the number of through-hole apertures can equal the number of sample vessels. In another embodiment the through-hole apertures can be circular. In another embodiment the through-hole apertures can be rectangular. In another embodiment the through-hole apertures can be square. In yet another embodiment the square apertures can provide optical access to samples for both 96 well and 384 well formats. FIG. 44B illustrates how square apertures can align with 96 sample wells in the thermal block assembly. Each square aperture is depicted to align with 1 sample well. FIG. 44C illustrates how square apertures can align with 384 sample wells in the thermal block assembly. Each square aperture is depicted to align with 4 sample wells. Such a configuration can increase the usability of the heated cover components over multiple instruments and can reduce the cost of each component.

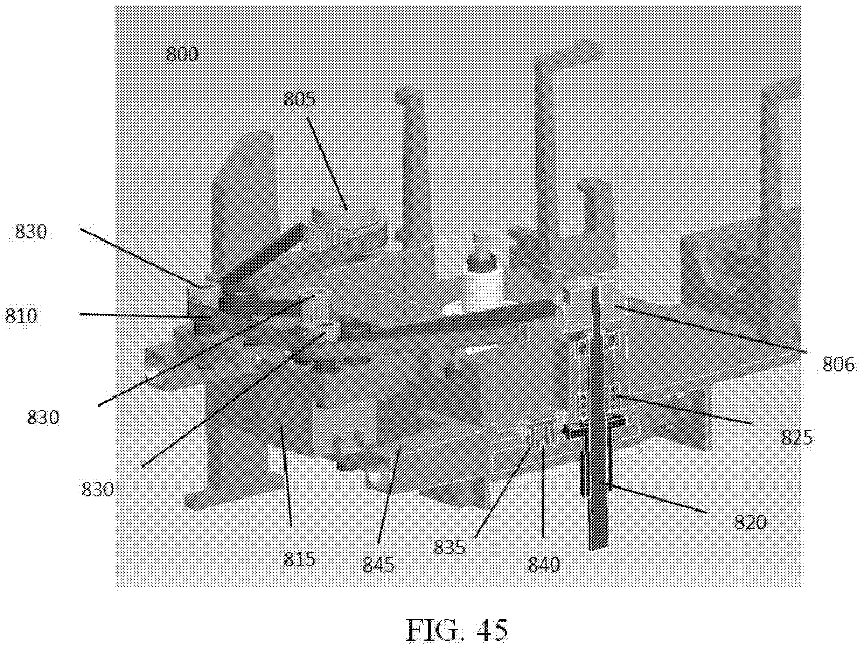

The second function of heated cover 700 is to provide a downward force on the top of the sample vessels to firmly engage the sample vessels with the sample wells to minimize the thermal resistance between the sample wells and the samples. The amount of force necessary can be dependent on the type of material or materials used to form the sample vessels. In one embodiment the amount of force can be 90 pounds. In another embodiment the force can be between 90 pounds and 150 pounds. In yet another embodiment the force can be greater than 150 pounds. Cover 700 can be vertically moved to engage the sample vessels to provide the necessary force. Cover 700 can be moved through the use of cams, levers, pistons, solenoids or motors. One skilled in the art will recognize that these elements are not the only mechanisms capable of providing the necessary movement and that any mechanism that can provide movement can be utilized. One such mechanism is illustrated in FIG. 45.

The illustration in FIG. 45 depicts automated system 800 for translating heated cover 700 between a position that does not engage the sample vessels to a position to engage the sample vessels. System 800 can include motor 815, belt 810, pulley 806, pulleys 830 and lead screw 820. A second lead screw (not shown) is also included with pulley 805 is also located in a similar location as lead screw 820. Each lead screw and associated pulley is located on each side of cover heated 700. System 800 can be modified to provide any speed and force required by the application. One skilled in the art will know that the size of the motor, the pulley ratios and the specifications for the lead screws all contribute to determining the potential force that can be supplied. A system as described in FIG. 45 can therefore be design to provide 150 pounds of force on the tops of the sample vessels. Such a force needs to be distributed throughout the system and concentrated on the sample vessels. The distribution of the force can be aided by including bearings 825 located around the 2 lead screws. The force applied to the sample block assembly can also be distributed through support brackets (not shown) that aid in transferring the force to rail 510 shown in FIG. 39. Rail 510, therefore, would have to support that force without jeopardizing the smooth motion of block 114 and electronics 128 in FIG. 38.

Referring back to FIG. 44A, optical sensor 710 is depicted. Optical sensors continuously emit and detect infrared light across a gap. One side of the gap is an emitter and the other side is a receiver. As long as power is applied to the device the emitter constantly emits infrared light and the receiver continuously detects that light. If an opaque object is inserted in the gap, the emitter continues to emit the infrared light but the light is blocked from reaching the receiver. The difference between receiving light and not receiving light can be detected and identified by a computer programmed to detect the difference. As a result the computer can detect if the light is blocked or not blocked. Automated systems can use this effect by mounting or providing an opaque object connected to a moving object to detect whether the moving object is at the location of the optical switch or not and motor 500 would be turned off.

As presented above, heated cover 700 and system 800 together can provide at least 150 pounds of force to the tops of the sample vessels. It would also be advantageous if that force could be delivered consistently, regardless of the dimensions of the sample vessels. Spring assembly 730 and optical switch 710 are included to provide the desired force for all sample vessels. Spring assembly can be designed to respond to the desired force for the system. Spring assembly 730 also includes opaque tab 735 located in the gap of optical switch 710. When cover 700 is lowered and engages the sample vessels the applied force increases as cover 700 lowers. As the force increases, spring assembly 730 responds and moves in an upward direction. When the desired force is applied, spring assembly 730 will have moved upward enough that tab 735 will block the light path of optical switch 710. The blocked light path can be detected by a programmed computer as described above and motor 815 can be turned off.

In an instrument with automated features such as disclosed in the present teachings, it would be advantageous to prevent the sample block from opening before the heated cover was raised. Protection against this scenario is provided by optical switch 835 and opaque tab 840 as depicted in FIG. 45. Optical switch 835 can be mounted to cover support 845 in a fixed position. Opaque tab 840 can be located on cover 700 and aligned with the gap of optical switch 835. At the completion of PCR the heated cover and opaque tab 840 can be raised until opaque tab 840 enters the gap of optical switch 835. When opaque tab 840 is raised enough to block the light path of optical switch 835 a programmed computer can detect the position of the cover to be completely raised and motor 815 can be turned off.

**Claims**

1. A biological analysis system comprising:

   a sample block assembly comprising a sample block (114) configured to accommodate a sample holder, the sample holder configured to receive a plurality of samples (112);
   a control system (120) configured to cycle the plurality of samples through a series of temperatures; and
   an automated tray (160) comprising a slide assembly, the automated tray configured to reversibly slide the sample block assembly from a closed to an open position to allow user access to the plurality of samples (112), and
   a heated cover,
   wherein the automated tray further comprises a positional sensor (575, 580) configured to determine when the automated tray has achieved a defined closed position and defined open position.
   wherein the automated tray (160) or sample block assembly further comprises an opaque tab (540, 580) configured to block emitted light from the positional sensor, and
   wherein the positional sensor is configured to determine when the automated tray has achieved the defined closed position such that the sample block is aligned with the heated cover, wherein the positional sensor is an infrared light optical sensor or infrared light optical switch.

**Patentansprüche**

1. System für eine biologische Analyse, umfassend:

eine Probenblockanordnung, umfassend einen Probenblock (114), der konfiguriert ist, um einen Probenhalter aufzunehmen, wobei der Probenhalter konfiguriert ist, um eine Vielzahl von Proben (112) aufzunehmen;
ein Steuersystem (120), das konfiguriert ist, um die Vielzahl von Proben durch eine Temperaturserie zu fahren; und
eine automatisierte Schublade (160), umfassend eine Gleitanordnung, wobei die automatisierte Schublade konfiguriert ist, um die Probenblockanordnung von einer geschlossenen in eine offene Position reversibel zu schieben, um den Benutzerzugriff auf die Vielzahl von Proben (112) zu ermöglichen, und
eine beheizte Abdeckung,
wobei die automatisierte Schublade ferner einen Positionssensor (575, 580) umfasst, der konfiguriert ist, um zu bestimmen, wann die automatisierte Schublade eine definierte geschlossene Position und definierte offene Position erreicht hat.
wobei die automatisierte Schublade (160) oder die Probenblockanordnung ferner eine lichtundurchlässige Lasche (540, 580) umfasst, die konfiguriert ist, um emittiertes Licht von dem Positionssensor zu blockieren, und
wobei der Positionssensor konfiguriert ist, um zu bestimmen, wann die automatisierte Schublade die definierte geschlossene Position derart erreicht hat, dass der Probenblock an der beheizten Abdeckung ausgerichtet ist,
wobei der Positionssensor ein optischer Infrarotlichtsensor oder ein optischer Infrarotlichtschalter ist.

**Revendications**

1. Système d'analyse biologique comprenant :

un ensemble bloc d'échantillon comprenant un bloc d'échantillon (114) configuré pour recevoir un porte-échantillon, le porte-échantillon étant conçu pour recevoir une pluralité d'échantillons (112) ;
un système de commande (120) configuré pour faire passer la pluralité d'échantillons par une série de températures ; et
un plateau automatisé (160) comprenant un ensemble coulissant, le plateau automatisé étant conçu pour faire coulisser de manière réversible l'ensemble bloc d'échantillon d'une position fermée à une position ouverte pour permettre à un utilisateur d'accéder à la pluralité d'échantillons (112), et
un couvercle chauffé,
dans lequel le plateau automatisé comprend en outre un capteur de position (575, 580) configuré pour déterminer quand le plateau automatisé a atteint une position fermée définie et une position ouverte définie.
dans lequel le plateau automatisé (160) ou l'ensemble bloc d'échantillon comprend en outre une languette opaque (540, 580) conçue pour bloquer la lumière émise à partir du capteur de position, et
dans lequel le capteur de position est configuré pour déterminer quand le plateau automatisé a atteint la position fermée définie de telle sorte que le bloc échantillon est aligné avec le couvercle chauffé, dans lequel le capteur de position est un capteur optique à lumière infrarouge ou un commutateur optique à lumière infrarouge.

100

124

OPTICAL SYSTEM

110

HEATED COVER

112

SAMPLES

114

SAMPLE BLOCK

116

HEAT/COOL ELEMENTS

118

HEAT EXCHANGER

CONTROL SYSTEM

120

USER INTERFACE

122

FIG. 1

# FIG. 2

DISPLAY
212

INPUT
DEVICE
214

CURSOR
CONTROL
216

MEMORY
206

ROM
208

STORAGE
DEVICE
210

BUS
202

PROCESSOR
204

COMM
INTERFACE
218

200

**FIG. 3**

FIG. 4

FIG. 5

100

405

410

425

415

420

FIG. 6

**FIG. 7**

## Normalized Spectrum

FIG. 8

## Energy over different channels (normalized to X2)

FIG. 9

**FIG. 10**

FIG. 11

FIG. 12

FIG. 13

1452 1435 1445 1450 1455 1470

FIG. 14

1452 1435 1450 1445 1449

FIG. 15

FIG. 16

**FIG. 17**

**FIG. 18**

1455

1455

1470

1470

1455

**FIG. 19**

1472

1470

FIG. 20

2100

START

ROI Calibration 2102

Background Calibration 2104

Uniformity Calibration 2106

Pure Dye Calibration 2108

Instrument Normalization
Calibration 2110

RNase P Validation 2112

END

**FIG. 21**

Estimate Initial ROI Centers
From Fluorescence Threshold ——— 2610

↓

Estimate Size of Each ROI ——— 2620

↓

Determine the Average Size of
All ROIs ——— 2630

↓

Derive Global Gridding Models ——— 2640

↓

Improve the Precision on ROI
Centers ——— 2650

↓

Recover Missing ROIs ——— 2660

↓

Extend Each Radius to Achieve
Best S/N ——— 2670

**FIG. 22**

2720

2710

**FIG. 23**

**FIG. 24**

EP 3 253 492 B1

2902 — Prepare Checkerboard Calibration Plates

2904 — Load Calibration Plate Into Instrument

2906 — Acquire Images of Plate at Each Channel

2908 — Identify Peak Channel for Each Dye on Plate

2910 — Normalize Each Channel to Peak Channel for all Detected Wells

2900

**FIG. 25**

2900

2912 — Perform Global Modeling for Undetected Wells

2914 — Perform Goodness of Fit Check

2916 — Good Fit

Bad Fit — 2920

Local Modeling — 2922

2918 — Output Dye Matrix

**FIG. 26**

**FIG. 27A**

| | FAM 200nM |
| | VIC 200nM |
| | ROX 200nM |
| | SYBR 1X |

| | Buffer 1X |
| | MP 600nM |
| | ABY 200nM |
| | JUN 200nM |

| | NED 200nM |
| | TAMRA 200nM |
| | CY5 |
| | Buffer 1X |

FIG. 27B

| DyeMixtures |
| --- |
| ABY/MP |
| Cy5/ROX |
| FAM/MP |
| FAM/ROX |
| JUN/MP |
| NED/ROX |
| VIC/MP |
| VIC/ROX |

**FIG. 28A**

| Dyes | Main Channel |
| --- | --- |
| FAM | X1M1 |
| SYBR | X1M1 |
| VIC | X2M2 |
| HEX | X2M2 |
| NED | X3M3 |
| ABY | X3M3 |
| ROX | X4M4 |
| JUN | X4M4 |
| CY5 | X5M5 |
| MP | X5M5 |

**FIG. 28B**

FIG. 29

Original % Deviation of Dye Mixture from Average Ratio

FIG. 30

Normalized % Deviation of Dye Mixture from Average Ratio

FIG. 31

3600 ———

| | |
|---|---|
| GENERATE CALIBRATION SPECTRA FOR MULTIPLE DYES ACROSS MULTIPLE FILTER COMBINATIONS | 3605 |
| NORMALIZE THE SPECTRA | 3610 |
| AVERAGE THE DYE SPECTRA ACROSS MULTIPLE WELLS | 3615 |
| GET SAME DYE SPECTRA FROM ANOTHER INSTRUMENT | 3620 |
| SET ADJUSTMENT FACTOR FOR EACH FILTER TO 1 | 3625 |
| MODIFY ADJUSTMENT FACTORS TO MINIMIZE DIFFERENCE BETWEEN INSTRUMENTS | 3630 |
| CALCULATE CORRECTION FACTOR FOR EACH FILTER COMBINATION | 3635 |
| MULTIPLY THE FILTER DATA BY FILTER COMBINATION CORRECTION FACTOR | 3640 |
| NORMALIZE CORRECTED DATA | 3645 |
| GENERATE MULTICOMPONENT DATA | 3650 |

FIG. 32

START

Receive amplification data from validation
assay plate to generate a plurality of
amplification curves — 3702

Determine a plurality of fluorescence
thresholds based on exponential regions of
the plurality of amplification curves — 3704

Determine Ct values of amplification
curves generated from two different
quantities included on the validation plate — 3706

Calculating if the two different quantities
are sufficiently distinguishable based on Ct
values at each of the plurality of
fluorescence thresholds — 3708

END

**FIG. 33**

**FIG. 34**

EP 3 253 492 B1

EP 3 253 492 B1

**FIG. 35**

FIG. 36

**FIG. 37**

EP 3 253 492 B1

FIG. 38

FIG. 39

FIG. 40

FIG. 41A

580

575

540

550

FIG. 41B

FIG. 41C

590

585

510

FIG. 42A

580

575

510

590

FIG. 42B

FIG. 43

FIG. 44A

FIG. 44B

FIG. 44C

FIG. 45

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2615462 A1
- US 20140242596 A
- WO 2013138706 A
- US 6518068 B